(19) Europäisches Patentamt
European Patent Office
Office européen des brevets

(11) EP 2 455 362 A1

(12) EUROPEAN PATENT APPLICATION

(43) Date of publication:
**23.05.2012 Bulletin 2012/21**

(21) Application number: **12150818.8**

(22) Date of filing: **28.11.2006**

(51) Int Cl.:
***C07C 233/65*** *(2006.01)*
***C07D 221/12*** *(2006.01)*
***A61K 31/445*** *(2006.01)*
***A61K 31/138*** *(2006.01)*
***A61K 31/165*** *(2006.01)*
***A61K 31/55*** *(2006.01)*
***A61K 31/40*** *(2006.01)*

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **28.11.2005 US 73989805 P**

(62) Document number(s) of the earlier application(s) in accordance with Art. 76 EPC:
**06838432.0 / 1 954 670**

(71) Applicant: **GTX, Inc.**
**Memphis, TN 38163 (US)**

(72) Inventors:
- **Dalton, James, T.**
**Upper Arlington, Ohio 43220 (US)**
- **Barrett, Christina**
**Oakland, Tennessee 38060 (US)**
- **He, Yali**
**Germantown, Tennessee 38139 (US)**
- **Hong, Seoung-Soo**
**Collierville, Tennessee 38017 (US)**
- **Miller, Duane, D.**
**Germantown, Tennessee 38139 (US)**
- **Mohler, Michael, L.**
**Memphis, Tennessee 38134 (US)**
- **Narayanan, Ramesh**
**Cordova, Tennessee 38018 (US)**
- **Wu, Zhongzhi**
**Memphis, Tennessee 38107 (US)**

(74) Representative: **Lord, Hilton David**
**Marks & Clerk LLP**
**90 Long Acre**
**London**
**WC2E 9RA (GB)**

Remarks:
This application was filed on 11-01-2012 as a divisional application to the application mentioned under INID code 62.

# (54) Nuclear receptor binding agents

(57) The present invention relates to a novel class of selective estrogen receptor modulators (SERMs). The SERM compounds are applicable for use in the prevention and/or treatment of a variety of diseases and conditions including prevention and treatment of cancers such as prostrate and breast cancer, osteoporosis, hormone-related diseases, hot flashes or vasomotor symptoms, neurological disorders, cardiovascular disease and obesity.

EP 2 455 362 A1

## Description

### FIELD OF THE INVENTION

The present invention relates to a novel class of nuclear receptor binding agents (NRBAs). The NRBA compounds are applicable for use in the prevention and/or treatment of a variety of diseases and conditions including, *inter alia,* prevention and treatment of hormone-related diseases, cancers, inflammation, osteoporosis, peripheral vascular disease, neurological disorders, ocular disorders, cardiovascular disease and obesity.

### BACKGROUND OF THE INVENTION

The nuclear hormone receptor superfamily of ligand activated transcription factors is present in various tissues, and responsible for a multitude of effects in these tissues.

The nuclear receptor (NR) superfamily presently comprises approximately 48 different proteins, most of which are believed to function as ligand activated transcription factors, exerting widely different biological responses by regulating gene expression. Members of this family include receptors for endogenous small, lipophilic molecules, such as steroid hormones, retinoids, vitamin D and thyroid hormone.

The nuclear receptor (NR) superfamily includes the steroid nuclear receptor subfamily, such as the mineralocorticoid receptor (MR) (or aldosterone receptor), the estrogen receptors (ER), ER alpha and ER beta, the androgen receptor (AR), the progesterone receptors (PR), glucocorticoid receptors (GR) and others. Also closely related in structure are the estrogen related receptors (ERRs) ERR-alpha, ERR-beta and ERR-gamma. The steroid nuclear receptors perform important functions in the body some of which are related to the transcriptional homeostasis of electrolyte and water balance, growth, development and wound healing, fertility, stress responses, immunological function, and cognitive functioning. The effects may be mediated by cytosolic or nuclear events. The effects may be mediated by cytosolic or nuclear events. Accordingly, compounds that modulate (i.e. antagonize, agonize, partially antagonize, partially agonize) the activity of steroid nuclear receptors are important pharmaceutical agents that have specific utility in a number of methods, as well as for the treatment and prevention of a wide range of diseases and disorders modulated by the activity of steroid nuclear receptors.

Members of the steroid nuclear receptor sub-family exhibit significant homology to each other and possess closely related DNA and ligand binding domains.

Given the close similarity in ligand binding domains of the steroid nuclear receptors, it is not surprising that many naturally occurring and synthetic molecules possess the ability to modulate the activity of more than one steroid nuclear receptor.

### SUMMARY OF THE PRESENT INVENTION

In one embodiment, this invention provides a nuclear receptor binding agent (NRBA), which in one embodiment is a selective estrogen receptor modulator (SERM) compound or its prodrug, analog, isomer, metabolite, derivative, pharmaceutically acceptable salt, pharmaceutical product, polymorph, crystal, impurity, *N*-oxide, ester, hydrate or any combination thereof, represented by the structure of formula I:

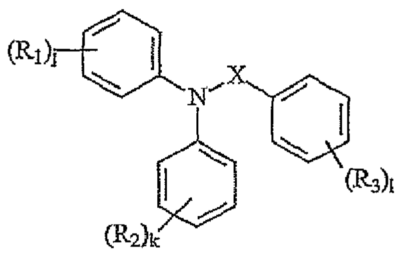

(I)

wherein

X is CO, CS, $(CH_2)q$, branched alkyl, branched alkyl with haloalkyl side chain, haloalkyl, $C(O)(CH_2)_q$, SO, or $SO_2$;

$R_1$, $R_2$ and $R_3$ are independently, hydrogen, halogen, aldehyde, COOH, CHNOH, $CH{=}CHCO_2H$, hydroxyalkyl, hydroxyl, alkoxy, cyano, nitro, $CF_3$, $NH_2$, NHR, NHCOR, $N(R)_2$, sulfonamide, $SO_2R$, alkyl, aryl, protected hydroxyl, $OCH_2CH_2NR_4R_5$, Z-Alk-Q, Z-Alk-NR4$R_5$, Z-Alk-heterorycle or $OCH_2CH_2$-heterocycle in which the heterocycle is a 3-7

membered substituted or unsubstituted heterocyclic ring, optionally aromatic,
or $R_1$ $R_2$ or $R_3$ together with the benzene ring to which the R-group is attached comprises a fused ring system represented by structure A

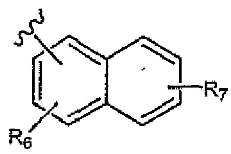

A

wherein;
$R_6$ and $R_7$ are independently $R_1$, $R_2$ or $R_3$;
R is alkyl, hydrogen, haloalkyl, dihaloalkyl, trihaloalkyl, $CH_2F$, $CHF_2$, $CF_3$, $CF_2CF_3$, aryl, phenyl, halogen, alkenyl, CN, $NO_2$, or OH;
$R_4$ and $R_5$ are independently hydrogen, phenyl, an alkyl group of 1 to 6 carbon atoms, a 3 to 7 membered cycloalkyl, a 3 to 7 membered heterocycloalkyl, or a 3 to 7 membered
heteroaryl group;
Z is O, NH, $CH_2$ or

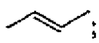

Q is $SO_3H$, $CO_2H$, $CO_2R$, $NO_2$, tetrazole, $SO_2NH_2$ or $SO_2NHR$;
j, k, l are independently 1-5;
q is 1-5;
Alk is linear alkyl of 1-7 carbons, branched alkyl of 1-7 carbons, or cyclic alkyl of 3-8 carbons; and
if X is $(CH_2)q$, CO or $C(O)(CH_2)q$, and $R_2$ is $OCH_2CH_2NR_4R_5$, or $OCH_2CH_2$-heterocycle when k is 1, then $R_1$ or $R_3$ is not hydrogen, lower alkyl (1-4 carbons), lower alkoxy (1-4 carbons), halogen, nitro or amino;
if X is $(CH_2)_q$, CO or $C(O)(CH_2)_q$, aud $R_3$ is $OCH_2CH_2NR_4R_5$, or $OCH_2CH_2$-heterocycle when 1 is one, then $R_1$ or $R_2$ is not hydrogen, lower alkyl (1-4 carbons), lower alkoxy (1-4 carbons), halogen, nitro or amino.

In one embodiment, this invention provides a NRBA, which in one embodiment is a SERM compound or its prodrug, analog, isomer, metabolite, derivative, pharmaceutically acceptable salt, pharmaceutical product, polymorph, crystal, impurity, *N*-oxide, ester, hydrate or any combination thereof, represented by the structure of formula I:

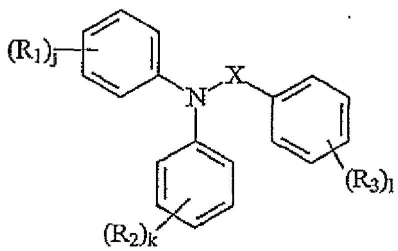

(I)

wherein
X is CS, branched alkyl, branched alkyl with haloalkyl side chain, haloalkyl, SO, or $SO_2$;
$R_1$, $R_2$ and $R_3$ are independently, hydrogen, halogen, aldehyde, COOH, CHNOH, $CH=CHCO_2H$, hydroxyalkyl, hydroxyl, alkoxy, cyano, nitro, $CF_3$, $NH_2$, NHR, NHCOR, $N(R)_2$, sulfonamide, $SO_2R$, alkyl, aryl, protected hydroxyl, $OCH_2CH_2NR_4R_5$, $Z(CH_2)qQ$, Z-Alk-$NR_4R_5$, Z-Alk-heterocycle or $OCH_2CH_2$-heterocycle in which the heterocycle is a 3-7 membered substituted or unsubstituted heterocyclic ring, optionally aromatic;
or $R_1$, $R_2$ or $R_3$ together with the benzene ring to which the R-group is attached comprises a fused ring system represented by structure A

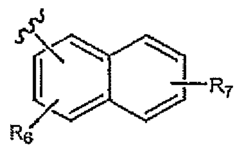

A

wherein $R_6$ or $R_7$ are independently $R_1$, $R_2$ or $R_3$;
R is alkyl, hydrogen, haloalkyl, dihaloalkyl, trihaloalkyl, $CH_2F$, $CHF_2$, $CF_3$, $CF_2CF_3$, aryl, phenyl, halogen, alkenyl, CN, $NO_2$, or OH;
$R_4$ and $R_5$ are independently hydrogen, phenyl, an alkyl group of 1 to 6 carbon atoms, a 3 to 7 membered cycloalkyl, a 3 to 7 membered heterocycloalkyl, or a 3 to 7 membered heteroaryl group;
Z is O, NH, $CH_2$, or

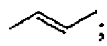;

Q is $SO_3H$, $CO_2H$, $CO_2R$, $NO_2$, tetrazole, $SO_2NH_2$, or $SO_2NHR$;
j, k, 1 are independently 1-5;
q is 1-5;
Alk is linear alkyl of 1-7 carbons, branched alkyl of 1-7 carbons or cyclic alkyl of 3-8 carbons.

In one embodiment this invention provides a NRBA, which in one embodiment is a SERM compound or its prodrug, analog, isomer, metabolite, derivative, Pharmaceutically acceptable salt, pharmaceutical product, polymorph, crystal, impurity, *N*-oxide, ester, hydrate or any combination thereof, represented by the structure of formula II:

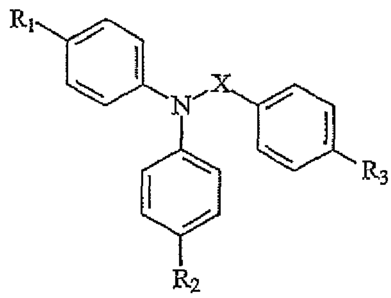

(II)

X is CO, CS, $(CH_2)q$, branched alkyl, branched alkyl with haloalkyl side chain, haloalkyl, $C(O)(CH_2)_q$, SO, or $SO_2$;
$R_1$, $R_2$ and $R_3$ are independently, hydrogen, halogen, aldehyde, COOH, CHNOH, $CH=CHCO_2H$, hydroxyalkyl, hydroxyl, alkoxy, cyano, nitro, $CF_3$, $NH_2$, NHR, NHCOR, $N(R)_2$, sulfonamide, $SO_2R$, alkyl, aryl, protected hydroxyl, $OCH_2CH_2NR_4R_5$, Z-Alk-Q, Z-Alk-$NR_4R_5$, Z-Alk-heterocycle or $OCH_2CH_2$-heterocycle in which the heterocycle is a 3-7 membered substituted or unsubstituted heterocyclic ring, optionally aromatic,
or $R_1$, $R_2$ or $R_3$ together with the benzene ring to which the R-group is attached comprises a fused ring system represented by structure A

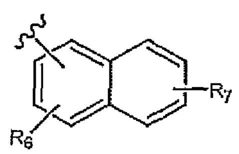

A

wherein
$R_6$ and $R_7$ are independently $R_1$, $R_2$ or $R_3$;
R is alkyl, hydrogen, haloalkyl, dihaloalkyl, trihaloalkyl, $CH_2F$, $CHF_2$, $CF_3$, $CF_2CF_3$, aryl, phenyl, halogen, alkenyl, CN, $NO_2$, or OH;

$R_4$ and $R_5$ are independently hydrogen, phenyl, an alkyl group of 1 to 6 carbon atoms, a 3 to 7 membered cycloalkyl, a 3 to 7 membered heterocycloalkyl, or a 3 to 7 membered
heteroaryl group;
Z is O, NH, $CH_2$, or

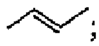 ;

Q is $SO_3H$, $CO_2H$, $CO_2R$, $NO_2$, tetrazole, $SO_2NH_2$, or $SO_2NHR$; q is 1-5;
Alk is linear alkyl of 1-7 carbons, branched alkyl of 1-7 carbons or cyclic alkyl of 3-8 carbons; and
if X is $(CH_2)_q$, CO or $C(O)(CH_2)_q$, and $R_2$ is $OCH_2CH_2NR_4R_5$, or $OCH_2CH_2$-heterocycle when k is 1, then $R_1$ or $R_3$ is not hydrogen, lower alkyl (1-4 carbons), lower alkoxy (1-4 carbons), halogen, nitro or amino;
if X is $(CH_2)$q, CO or $C(O)(CH_2)_q$, and $R_3$ is $OCH_2CH_2NR_4R_5$, or $OCH_2CH_2$-heterocycle when 1 is one, then $R_1$ or $R_2$ is not hydrogen, lower alkyl (1-4 carbons), lower alkoxy (1-4 carbons), halogen, nitro or amino.

In one embodiment this invention provides a NRBA, which in one embodiment is a SERM compound or its prodrug, analog, isomer, metabolite, derivative, pharmaceutically acceptable salt, pharmaceutical product, polymorph, crystal, impurity, *N*-oxide, ester, hydrate or any combination thereof, represented by the structure of formula II:

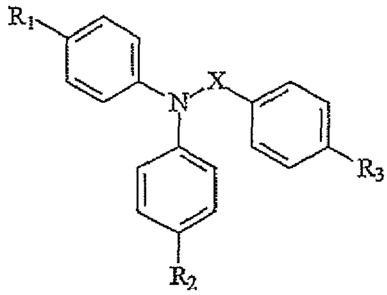

(II)

wherein
X is CS, branched alkyl, branched alkyl with haloalkyl side chain, haloalkyl, SO, or $SO_2$;
$R_1$, $R_2$ and $R_3$ are independently, hydrogen, halogen, aldehyde, COOH, CHNOH, $CH=CHCO_2H$, hydroxyalkyl, hydroxyl, alkoxy, cyano, nitro, $CF_3$, $NH_2$, NHR, NHCOR, $N(R)_2$, sulfonamide, $SO_2R$, alkyl, aryl, protected hydroxyl, $OCH_2CH_2NR_4R_5$, Z-Alk-Q, Z-Alk-$NR_4R_5$, Z-Alk-heterocycle or $OCH_2CH_2$-heterocycle in which the heterocycle is a 3-7 membered substituted or unsubstituted heterocyclic ring, optionally aromatic,
or $R_1$, $R_2$ or $R_3$ together with the benzene ring to which the R-group is attached comprises a fused ring system represented by structure A

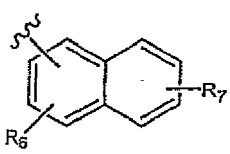 A

wherein $R_6$ or $R_7$ are independently $R_1$, $R_2$ or $R_3$;
R is alkyl, hydrogen, haloalkyl, dihaloalkyl, trihaloalkyl, $CH_2F$, $CHF_2$, $CF_3$, $CF_2CF_3$, aryl, phenyl, halogen, alkenyl, CN, $NO_2$, or OH;
$R_4$ and $R_5$ are independently hydrogen, phenyl, an alkyl group of 1 to 6 carbon atoms, a 3 to 7 membered cycloalkyl, a 3 to 7 membered heterocycloalkyl, or a 3 to 7 membered
heteroaryl group;
Z is O, NH, $CH_2$, or

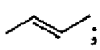 ;

Q is $SO_3H$, $CO_2H$, $CO_2R$, $NO_2$, tetrazole, $SO_2NH_2$, or $SO_2NHR$;
Alk is linear alkyl of 1-7 carbons, branched alkyl of 1-7 carbons or cyclic alkyl of 3-8 carbons.

In another embodiment this invention provides a NRBA, which in one embodiment is a SERM compound or its prodrug, analog, isomer, metabolite, derivative, pharmaceutically acceptable salt, pharmaceutical product, polymorph, crystal, impurity, *N*-oxide, ester, hydrate or any combination thereof, represented by the structure of formula III:

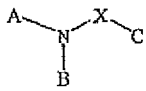

(III)

wherein
A is a ring selected from

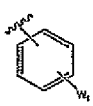
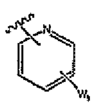
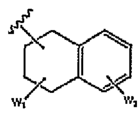
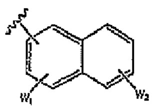
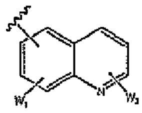
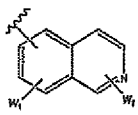

B is a ring selected from

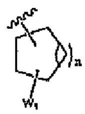
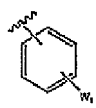
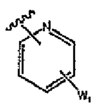
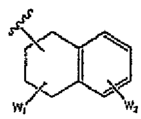
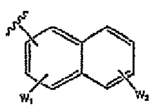
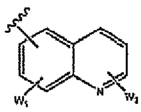
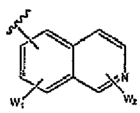

C is a ring selected from

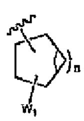
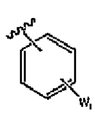
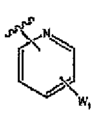
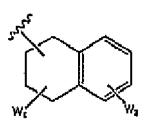
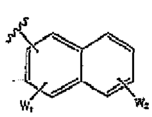
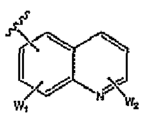
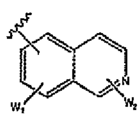

X is CO, CS, $(CH_2)_q$, branched alkyl, branched alkyl with haloalkyl side chain, haloalkyl, $C(O)(CH_2)_q$, SO, or $SO_2$;
$W_1$ and $W_2$ are independently, hydrogen, halogen, aldehyde, COOH, CHNOH, $CH=CHCO_2H$, hydroxyalkyl, hydroxyl, alkoxy, cyano, nitro, $CF_3$, $NH_2$, NHR, NHCOR, $N(R)_2$, sulfonamide, $SO_2R$, alkyl, aryl, protected hydroxyl, $OCH_2CH_2NR_4R_5$, Z-Alk-Q, Z-Alk-$NR_4R_5$, Z-Alk-heterocycle or $OCH_2CH_2$-heterocycle in which the heterocycle is a 3-7 membered substituted or unsubstituted heterocyclic ring, optionally aromatic;
R is alkyl, hydrogen, haloalkyl, dihaloalkyl, trihaloalkyl, $CH_2F$, $CHF_2$, $CF_3$, $CF_2CF_3$, aryl, phenyl, halogen, alkenyl, CN, $NO_2$, or OH;
$R_4$ and $R_5$ are independently hydrogen, phenyl, an alkyl group of I to 6 carbon atoms, a 3 to 7 membered cycloalkyl, a

3 to 7 membered heterocycloalkyl, or a 3 to 7 membered heteroaryl group;
Z is O, NH, $CH_2$, or

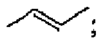;

Q is $SO_3H$, $CO_2H$, $CO_2R$, $NO_2$, tetrazole, $SO_2NH_2$, or $SO_2NHR$; q is 1-5;
n is 0-5;
Alk is linear alkyl of 1-7 carbons, branched alkyl of 1-7 carbons, or cyclic alkyl of 3-8 carbons;
A, B and C cannot simultaneously be a benzene ring; and
if X is $(CH_2)_q$, CO or $C(O)(CH_2)_q$, A is pyridyl ring and B and C are phenyl rings and C is substituted with $OCH_2CH_2NR_4R_5$, or $OCH_2CH_2$-heterocycle, then A or B is not substituted with hydrogen, lower alkyl (1-4 carbons), lower alkoxy (1-4 carbons), halogen, nitro or amino;
If X is $(CH_2)_q$, CO or $C(O)(CH_2)_q$, A is pyridyl ring and B and C are phenyl rings and B is substituted with $OCH_2CH_2NR_4R_5$, or $OCH_2CH_2$-heterocycle, then A or C is not substituted with hydrogen, lower alkyl (1-4 carbons), lower alkoxy (1-4 carbons), halogen, nitro or amino.

In another embodiment, this invention provides, a NRBA, which in one embodiment is a SERM compound or its prodrug, analog, isomer, metabolite, derivative, pharmaceutically acceptable salt, pharmaceutical product, polymorph, crystal, impurity, *N*-oxide, ester, hydrate or any combination thereof, represented by the structure of formula IV:

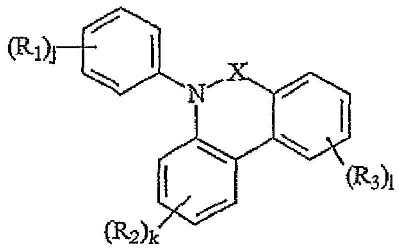

(IV)

wherein
X is CO, CS, $(CH_2)$q, branched alkyl, branched alkyl with haloalkyl side chain, haloalkyl, $C(O)(CH_2)_q$, SO, or $SO_2$;
$R_1$, $R_2$ and $R_3$ are independently, hydrogen, halogen, aldehyde, COOH, CHNOH, $CH=CHCO_2H$, hydroxyalkyl, hydroxyl, alkoxy, cyano, nitro, $CF_3$, $NH_2$, NHR, NHCOR, $N(R)_2$, sulfonamide, $SO_2R$, alkyl, aryl, protected hydroxyl, $OCH_2CH_2NR_4R_5$, Z-Alk-Q, Z-Alk-$NR_4R_5$, Z-Alk-heterocycle or $OCH_2CH_2$-heterocycle in which the heterocycle is a 3-7 membered substituted or unsubstituted heterocyclic ring, optionally aromatic,
or $R_1$, $R_2$ and $R_3$ together with the benzene ring to which the R-group is attached comprises a fused ring system represented by structure A

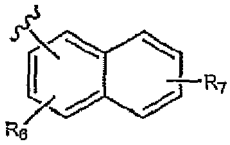

A

wherein
$R_6$ and $R_7$ independently are $R_1$, $R_2$ or $R_3$;
R is alkyl, hydrogen, haloalkyl, dihaloalkyl, trihaloalkyl, $CH_2F$, $CHF_2$, $CF_3$, $CF_2CF_3$, aryl, phenyl, halogen, alkenyl, CN, $NO_2$, or OH;
$R_4$ and $R_5$ are independently hydrogen, phenyl, an alkyl group of 1 to 6 carbon atoms, a 3 to 7 membered cycloalkyl, a 3 to 7 membered heterocycloalkyl, or a 3 to 7 membered heteroaryl group;
Z is O, NH, $CH_2$, or

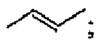 ;

Q is $SO_3H$, $CO_2H$, $CO_2R$, $NO_2$, tetrazole, $SO_2NH_2$, or $SO_2NHR$;
j, k, 1 are independently 1-5;
q is 1-5;
Alk is linear alkyl of 1-7 carbons, branched alkyl of 1-7 carbons or cyclic alkyl of 3-8 carbons.

In another embodiment, this invention provides a NRBA, which in one embodiment is a SERM compound or its prodrug, analog, isomer, metabolite, derivative, pharmaceutically acceptable salt, pharmaceutical product, polymorph, crystal, impurity, *N*-oxide, ester, hydrate or any combination thereof, represented by the structure of formula V:

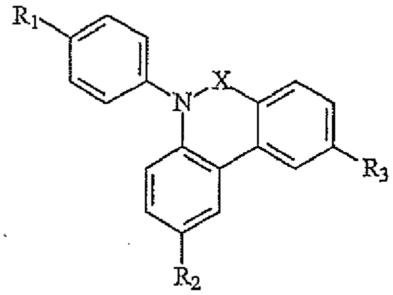

(V)

X is CO, CS, $(CH_2)_q$, branched alkyl, branched alkyl with haloalkyl side chain, haloalkyl, $C(O)(CH_2)_q$, SO, or $SO_2$;
$R_1$, $R_2$ and $R_3$ are independently, hydrogen, halogen, aldehyde, COOH, CHNOH, $CH=CHCO_2H$, hydroxyalkyl, hydroxyl, alkoxy, cyano, nitro, $CF_3$, $NH_2$, NHR, NHCOR, $N(R)_2$, sulfonamide, $SO_2R$, alkyl, aryl, protected hydroxyl group, $OCH_2CH_2NR_4R_5$, Z-Alk-Q, Z-Alk-$NR_4R_5$, Z-Alk-heterocycle or $OCH_2CH_2$-heterocycle in which the heterocycle is a 3-7 membered substituted or unsubstituted heterocyclic ring, optionally aromatic,
or $R_1$, $R_2$ and $R_3$ together with the benzene ring to which the R-group is attached comprises a fused ring system represented by structure A

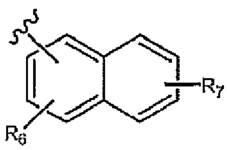 A

wherein
$R_6$ and $R_7$ independently are $R_1$, $R_2$ or $R_3$;
R is alkyl, hydrogen, haloalkyl, dihaloalkyl, trihaloalkyl, $CH_2F$, $CHF_2$, $CF_3$, $CF_2CF_3$, aryl, phenyl, halogen, CN, $NO_2$, alkenyl or OH;
$R_4$ and $R_5$ are independently hydrogen, phenyl, an alkyl group of 1 to 6 carbon atoms, a 3 to 7 membered cycloalkyl, a 3 to 7 membered heterocycloalkyl, or a 3 to 7 membered heteroaryl group; Z is O, NH, $CH_2$, or

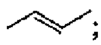 ;

Q is $SO_3H$, $CO_2H$, $CO_2R$, $NO_2$, tetrazole, $SO_2NH_2$, or $SO_2NHR$; q is 1-5;
Alk is linear alkyl of 1-7 carbons, branched alkyl of 1-7 carbons, or cyclic alkyl of 3-8 carbons.

In one embodiment this invention provides a NRBA, which in one embodiment is a SERM compound or its prodrug, analog, isomer, metabolite, derivative, pharmaceutically acceptable salt, pharmaceutical product, polymorph, crystal, impurity, *N*-oxide, ester, hydrate or any combination thereof, represented by the structure of formula VI:

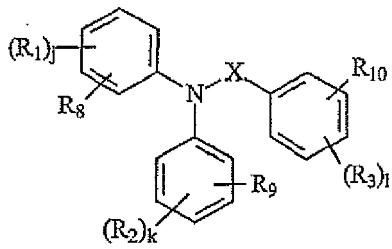

VI

wherein

X is CO, CS, $(CH_2)_q$, branched alkyl, branched alkyl with haloalkyl side chain, haloalkyl, $C(O)(CH_2)_q$, SO, or $SO_2$;

$R_1$, $R_2$, $R_3$, $R_8$, $R_9$, and $R_{10}$ are independently hydrogen, halogen, aldehyde, COOH, CHNOH, $CH{=}CHCO_2H$, hydroxyalkyl, hydroxyl, alkoxy, cyano, nitro, $CF_3$, $NH_2$, NHR, NHCOR, $N(R)_2$, sulfonamide, $SO_2R$, alkyl, aryl, protected hydroxyl, $OCH_2CH_2NR_4R_5$, Z-Alk-Q, Z-Alk-$NR_4R_5$, Z-Alk-heterocycle or $OCH_2CH_2$-heterocycle in which the heterocycle is a 3-7 membered substituted or unsubstituted heterocyclic ring, optionally aromatic,

or $R_1$, $R_2$ or $R_3$ together with the benzene ring to which the R-group is attached comprises a fused ring system represented by structure A

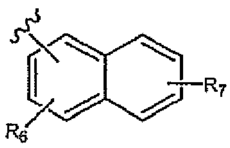

A

wherein

$R_6$ and $R_7$ are independently $R_1$, $R_2$ or $R_3$;

R is alkyl, hydrogen, haloalkyl, dihaloalkyl, trihaloalkyl, $CH_2F$, $CHF_2$, $CF_3$, $CF_2CF_3$, aryl, phenyl, halogen, CN, $NO_2$, alkenyl or OH;

$R_4$ and $R_5$ are independently hydrogen, phenyl, an alkyl group of 1 to 6 carbon atoms, a 3 to 7 membered cycloalkyl, a 3 to 7 membered heterocycloalkyl, or a 3 to 7 membered heteroaryl group;

Z is O, NH, $CH_2$, or

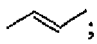;

Q is $SO_3H$, $CO_2H$, $CO_2R$, $NO_2$, tetrazole, $SO_2NH_2$, or $SO_2NHR$;

j, k, l are independently 1-4;

q is 1-5;

Alk is linear alkyl of 1-7 carbons, branched alkyl of 1-7 carbons, or cyclic alkyl of 3-8 carbons; and

if X is $(CH_2)_q$, CO or $C(O)(CH_2)_q$ , and $R_2$ is $OCR_2CH_2NR_4R_5$, or $OCH_2CH_2$-heterocycle when k is 1, and $R_9$ is hydrogen, lower alkyl (1-4 carbons), lower alkoxy (1-4 carbons), halogen, nitro or amino, then $R_1$ or $R_3$ is not hydrogen, lower alkyl (1-4 carbons), lower alkoxy (1-4 carbons), halogen, nitro or amino;

if X is $(CR_2)_q$, CO or $C(O)(CH_2)$q, and $R_3$ is $OCH_2CH_2NR_4R_5$, or $OCH_2CH_2$-heterocycle when 1 is one, and $R_{10}$ is hydrogen, lower alkyl (1-4 carbons), lower alkoxy (1-4 carbons), halogen, nitro or amino;

then $R_1$ or $R_2$ is not hydrogen, lower alkyl (1-4 carbons), lower alkoxy (1-4 carbons), halogen, nitro or amino.

In another embodiment, the 3-7 membered substituted or unsubstituted heterocyclic ring, optionally aromatic, is represented by the structure of formula B:

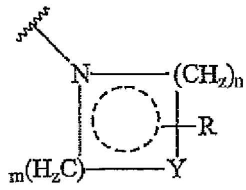

B

wherein Y is $CH_2$, CH, bond, O, S, NH, N or NR,
R is alkyl, hydrogen, haloalkyl, dihaloalkyl, trihaloalkyl, $CH_2F$, $CHF_2$, $CF_3$, $CF_2CF_3$, aryl, phenyl, halogen, alkenyl, CN, $NO_2$, or OH;
if B is aryl, then z is 1; and ifB is cycloalkyl, then z is 2;
m is 0-4;
n is 0-4;
wherein m and n cannot both be zero.

In one embodiment B is substituted or unsubstituted piperidine; in another embodiment B is substituted or unsubstituted pyrrolidine; in another embodiment B is substituted or unsubstituted morpholine; in another embodiment B is substituted or unsubstituted piperazine.

In one embodiment this invention provides a NRBA, which in one embodiment is a SERM compound or its prodrug, analog, isomer, metabolite, derivative, pharmaceutically acceptable salt, pharmaceutical product, polymorph, crystal, impurity, *N*-oxide, ester, hydrate or any combination thereof, represented by the structure of formula VII:

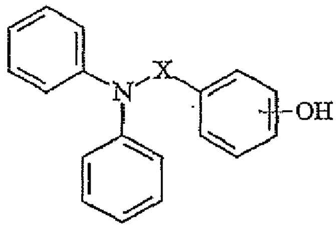

(VII)

wherein X is CS, $(CH_2)_q$, branched alkyl, branched alkyl with haloalkyl side chain, haloalkyl, $C(O)(CH_2)_q$, SO, or $SO_2$; or X is CO, and OH is meta or ortho.

In another embodiment this invention provides a NRBA, which in one embodiment is a SERM compound or its prodrug, analog, isomer, metabolite, derivative, pharmaceutically acceptable salt, pharmaceutical product, polymorph, crystal, impurity, *N*-oxide, ester, hydrate or any combination thereof, represented by the structure of formula VIII:

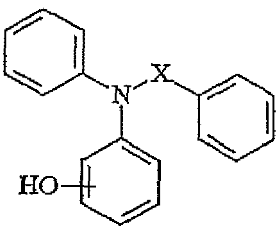

(VIII)

wherein X is CS, $(CH_2)_q$, branched alkyl, branched alkyl with haloalkyl side chain, haloalkyl, $C(O)(CH_2)_q$, SO, or $SO_2$; or X is CO, and OH is meta or ortho.

In another embodiment, this invention provides a NRBA, which in one embodiment is a SERM compound or its prodrug, analog, isomer, metabolite, derivative, pharmaceutically acceptable salt, pharmaceutical product, polymorph, crystal, impurity, *N*-oxide, ester, hydrate or any combination thereof, represented by the structure of formula IX:

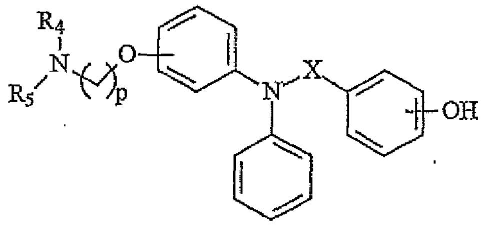

IX

wherein X is CO, CS, $(CH_2)_q$, branched alkyl, branched alkyl with haloalkyl side chain, haloalkyl, $C(O)(CH_2)_q$, SO, or $SO_2$; $R_4$ and $R_5$ are independently hydrogen, phenyl, an alkyl group of 1 to 6 carbon atoms, a 3 to 7 membered cycloalkyl, a 3 to 7 membered heterocycloalkyl, a 3 to 7 membered heteroaryl group, or $R_4$ and $R_5$ form together with the nitrogen atom a 3-7 heterocyclic ring, optionally aromatic, is represented by the structure of formula B:

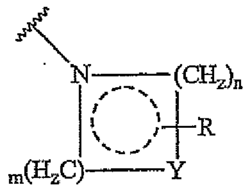

B

wherein Y is $CH_2$, CH, bond, O, S, NH, N or NR;
if B is aryl then z is 1; and ifB is cycloalkyl z is 2;
m is 0-4;
n is 0-4;
wherein m and n cannot both be zero;
q is 1-5;
p is 1-4;
R is alkyl, hydrogen, haloalkyl, dihaloalkyl, trihaloalkyl, $CH_2F$, $CHF_2$, $CF_3$, $CF_2CF_3$, aryl, phenyl, halogen, alkenyl, CN, $NO_2$ or OH.

In another embodiment, this invention provides a NRBA, which in one embodiment is a SERM compound or its prodrug, analog, isomer, metabolite, derivative, pharmaceutically acceptable salt, pharmaceutical product, polymorph, crystal, impurity, *N*-oxide, ester, hydrate or any combination thereof, represented by the structure of formula X:

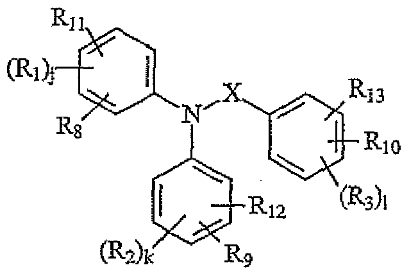

X

wherein
X is CO, CS, $(CH_2)_q$, branched alkyl, branched alkyl with haloalkyl side chain, haloalkyl, $C(O)(CH_2)_q$, SO, or $SO_2$;
$R_1$, $R_2$, $R_3$, $R_8$, $R_9$, $R_{10}$, $R_{11}$, $R_{12}$ and $R_{13}$ are independently hydrogen, halogen, aldehyde, COOH, CHNOH, $CH=CHCO_2H$, hydroxyalkyl, hydroxyl, alkoxy, cyano, nitro, $CF_3$, $NH_2$, NHR, NHCOR, $N(R)_2$, sulfonamide, $SO_2R$, alkyl, aryl, protected hydroxyl, $OCH_2CH_2NR_4R_5$, Z-Alk-Q, Z-Alk-$NR_4R_5$, Z-Alk-heterocycle or $OCH_2CH_2$-heterocycle in which the heterocycle is a 3-7 membered substituted or unsubstituted heterocyclic ring, optionally aromatic,
or $R_1$, $R_2$ or $R_3$ together with the benzene ring to which the R-group is attached comprises a fused ring system represented

by structure A

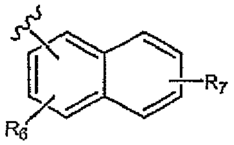

A

wherein
$R_6$ and $R_7$ are independently is $R_1$, $R_2$ or $R_3$;
R is alkyl, hydrogen, haloalkyl, dihaloalkyl, trihaloalkyl, $CH_2F$, $CHF_2$, $CF_3$, $CF_2CF_3$, aryl, phenyl, halogen, CN, $NO_2$, alkenyl or OH;
$R_4$ and $R_5$ are independently hydrogen, phenyl, an alkyl group of 1 to 6 carbon atoms, a 3 to 7 membered cycloalkyl, a 3 to 7 membered heterocycloalkyl, or a 3 to 7 membered heteroaryl group; or $R_4$ and $R_5$ form together with the nitrogen atom a 3-7 heterocyclic ring, optionally aromatic, is represented by the structure of formula B:

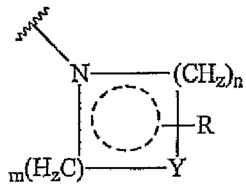

B.

wherein Y is $CH_2$, CH, bond, O, S, NH, N or NR;
if B is aryl then z is 1; and if B is cycloalkyl z is 2;
m is 0-4;
n is 0-4;
wherein m and n cannot both be zero;
q is 1-5;
p is 1-4;
R is alkyl, hydrogen, haloalkyl, dihaloalkyl, trihaloalkyl, $CH_2F$, $CHF_2$, $CF_3$, $CF_2CF_3$, aryl, phenyl, halogen, alkenyl, CN, $NO_2$ or OH;
Z is O, NH, $CH_2$, or

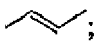

;

Q is $SO_3H$, $CO_2H$, $CO_2R$, $NO_2$, tetrazole, $SO_2NH_2$, or $SO_2NHR$;
j, k, l are independently 1-3;
q is 1-5;
Alk is linear alkyl of 1-7 carbons, branched alkyl of 1-7 carbons, or cyclic alkyl of 3-8 carbons; and
if X is $(CH_2)_q$, CO or $C(O)(CH_2)_q$, and $R_2$ is $OCH_2CH_2NR_4R_5$, or $OCH_2CH_2$-heterocycle when k is 1, and $R_9$ and $R_{12}$ are hydrogen, lower alkyl (1-4 carbons), lower alkoxy (1-4 carbons), halogen, nitro or amino, then
$R_1$ or $R_3$ is not hydrogen, lower alkyl (1-4 carbons), lower alkoxy (1-4 carbons),
halogen, nitro or amino;
if X is $(CH_2)_q$, CO or $C(O)(CH_2)_q$, and $R_3$ is $OCH_2CH_2NR_4R_5$, or $OCH_2CH_2$-heterocycle when l is one, and $R_{10}$ and $R_{13}$ are hydrogen, lower alkyl, (1-4 carbons), lower alkoxy (1-4 carbons), halogen, nitro or amino;
then $R_1$ or $R_2$ is not hydrogen, lower alkyl (1-4 carbons), lower alkoxy (1-4 carbons), halogen, nitro or amino.

In one embodiment this invention provides a method of reducing circulating lipid levels in a male subject with prostate cancer having undergone Androgen Deprivation Therapy (ADT), said method comprising administering to said subject a composition comprising a nuclear receptor binding agent (NRBA) compound or its pharmaceutically acceptable salt, hydrate, N-oxide, or any combination thereof.

In one embodiment, this invention provides a method of treating atherosclerosis and its associated diseases including cardiovascular disorders, cerebrovascular disorders, peripheral vascular disorders, and intestinal vascular disorders in a subject with prostate cancer having undergone Androgen Deprivation Therapy (ADT), comprising administering to said subject a composition comprising a nuclear receptor binding agent (NRBA) compound or its pharmaceutically acceptable salt, hydrate, N-oxide, or any combination thereof.

In one embodiment, this invention provides a method of treating ischemia in a tissue of a subject with prostate cancer having undergone Androgen Deprivation Therapy (ADT), comprising administering to said subject a composition comprising a a nuclear receptor binding agent (NRBA) compound or its pharmaceutically acceptable salt, hydrate, N-oxide, or any combination thereof.

In another embodiment this invention provides a method of (i) treating and preventing osteoporosis; (ii) treating, preventing or reducing the risk of mortality from cardiovascular disease in a subject; (iii) improving a lipid profile; (iv) reducing the incidence of, inhibiting, suppressing, or treating androgen-deprivation induced osteoporosis, bone fractures and/or loss of bone mineral density (BMD) in men having prostate cancer; (v) ameliorating symptoms and/or clinical complications associated with menopause in a female subject; (vi) treating, preventing or reducing the severity of Alzheimer's disease; (vii) treating, preventing, suppressing, inhibiting, or reducing the incidence of hot flashes, gynecomastia, and/or hair loss in a male; (viii) treating, suppressing, inhibiting or reducing the risk of developing prostate cancer in a subject with prostate cancer; (ix) treating, suppressing, inhibiting or reducing the amount of precancerous precursors of prostate adenocarcinoma lesions; (x) treating, suppressing, inhibiting or reducing the risk of developing breast cancer in a subject; (xi) treating, suppressing, inhibiting or reducing the risk of developing colon cancer in a subject; (xii) treating, suppressing, inhibiting or reducing the risk of developing leukemia in a subject; (xiii) treating, suppressing, inhibiting or reducing the risk of developing bladder cancer in a subject; (xiv) treating, suppressing, inhibiting or reducing the incidence of inflammation in a subject; (xv) treating, suppressing, inhibiting or reducing the incidence of neurological disorders in a subject; (xvi) treating, suppressing, inhibiting or reducing the incidence of ocular disorders,(xvii) reducing the lipid profile of a male subject with prostate cancer having undergone ADT, (xviii) treating, suppressing, inhibiting or reducing the risk of atherosclerosis of a male subject with prostate cancer having undergone ADT. (xix) treating, suppressing, inhibiting or reducing the risk of ischemia of a male subject with prostate cancer having undergone ADT. using the nuclear receptor binding agents (NRBAs) of the invention, which in one embodiment are SERUM compounds.

## BRIEF DESCRIPTION OF THE DRAWINGS

The subject matter regarded as the invention is particularly pointed out and distinctly claimed in the concluding portion of the specification. The invention, however, both as to organization and method of operation, together with objects, features, and advantages thereof, may best be understood by reference to the following detailed description when read with the accompanying drawings in which:

**Fig. 1:** Effects of the indicated compounds on ER-$\alpha$ transactivation. COS or 293 cells plated in DME without phenol red +10% charcoal stripped fetal bovine serum (csFBS) at 90,000 cells per well of 24 well plates were transfected with 0.25 $\mu$g ERE-LUC, 0.02 $\mu$g CMV-LUC (Renilla) and 25 ng of ER-$\alpha$ by lipofectamine. Twenty four hours after transfection, the cells were treated as indicated in the figure, harvested 48 hrs after transfection and were assayed for firefly and renilla luciferase.

**Fig. 2:** Effect of the indicated compounds on ER-$\beta$ transactivation. COS or 293 cells plated in DME without phenol red +10% csFBS at 90,000 cells per well of a 24 well plate were transfected with 0.25 $\mu$g ERE-LUC, 0.02 $\mu$g CMV-LUC (Renilla) and 50 ng of ER- $\beta$ by lipofectamine. Twenty four hours after transfection, the cells were treated as indicated in the figure, harvested 48 hrs after transfection and were assayed for firefly and renilla luciferase.

**Fig. 3:** Effect of the indicated compounds on AR transactivation. COS cells plated in DME without phenol red + 10% csFBS at 90,000 cells per well of a 24 well plate were transfected with 0.25 $\mu$g ARE-LUC, 0.02 $\mu$g CMV-LUC (Renilla) and 12.5 ng of AR by lipofectamine. Twenty four hours after transfection, the cells were treated as indicated in the figure, harvested 48 hrs after transfection and were assayed for firefly and renilla luciferase.

**Fig. 4:** Compound 4a functions as a partial agonist of ER action in MCF-7 cells. MCF-7 cells were plated at 500,000 cells per well of a 6 well plate. The cells were serum starved for 3 days and then were treated or not treated as indicated in the figure for 16 hrs. RNA was isolated and the message levels of pS2 (gene encoding the trefoil peptides) measured and normalized to 18S ribosomal RNA by realtime polymerase chain reaction (rtPCR).

**Fig. 5:** Effect of the indicated compounds on ER-$\alpha$ transactivation. COS or 293 cells plated in DME without phenol red + 10% csFBS at 90,000 cells per well of a 24 well plate were transfected with 0.25 $\mu$g ERE-LUC, 0.02 $\mu$g CMV-LUC (Renilla) and 5 ng of ER-$\alpha$ by lipofectamine. Twenty four hours after transfection, the cells were treated as indicated in the figure, harvested 48 hrs after transfection and were assayed for firefly and renilla luciferase.

**Fig. 6:** Effect of the indicated compounds on ER-$\beta$ transactivation. COS or 293 cells plated in DME without phenol red + 10% csFBS at 90,000 cells per well of a 24 well plate were transfected with 0.25 $\mu$g ERE-LUC, 0.02 $\mu$g CMV-LUC (Renilla) and 50 ng of ER-$\beta$ by lipofectamine. Twenty four hours after transfection, the cells were treated as indicated in the figure, harvested 48 hrs after transfection and assayed for firefly and renilla luciferase.

**Fig. 7:** Effect of the indicated compounds on TRAP positive multinucleated osteoclasts. Bone marrow cells from rat femur was cultured in Alpha MEM + 10% csFBS without phenol red in the presence or absence of 30 ng/ml

RANKL and 10 ng/ml GMCSF. The cells were treated for 12 days and were stained for tartarate resistant acid phosphatase activity (TRAP) and multinucleated osteoclasts were counted.

**Fig. 8:** Some embodiments of this invention include inhibition, of androgen independent prostate cancer cell growth via the compounds of this invention. PC-3 cells were plated in RPMI + 10% csFBS at 6000 cells per well of a 96 well plate. Medium was changed to RPMI + 1% csFBS without phenol red and then treated for 72 hrs with increasing concentrations of SERMs.

**Fig. 9:** Effect of the indicated compounds on ER-$\alpha$ transactivation. COS or 293 cells plated in DME without phenol red + 10% csFBS per well of a 24 well plate were transfected with 0.25 $\mu$g ERE-LUC, 0.02 $\mu$g CMV-LUC (Renilla) and 25 ng of ER-$\alpha$ by lipofectamine. Twenty four hours after transfection, the cells were treated as indicated in the figure, harvested 48 hrs after transfection and were assayed for firefly and renilla luciferase.

**Fig 10:** Effect of the indicated compounds on ER-$\beta$ transactivation. COS or 293 cells plated in DME +10% csFBS at 90,000 cells per well of a 24 well plate were transfected with 0.25 $\mu$g ERE-LUC, 0.02 $\mu$g CMV-LUC (Renilla) and 50 ng of ER- $\beta$ by lipofectamine. Twenty four hours after transfection, the cells were treated as indicated in the figure, harvested 48 hrs after transfection and were assayed for firefly and renilla luciferase.

**Fig. 11:** Agonistic activity of the indicated compounds in MCF-7 cells. MCF-7 cells were plated at 500,000 cells per well of a 6 well plate. The cells were starved for 3 days and then were treated or not treated as indicated in the figure for 16 hrs. RNA was isolated and the message levels of pS2 gene measured and normalized to 18S ribosomal RNA by realtime rtPCR.

**Fig 12:** Estrogenic activity of compound 3d, as compared to toremifene (Tor) and estradiol (E2), as measured by *in vivo* increased uterine tissue weight (mg).

**Fig 13:** Estrogenic activity of compounds 4a and 4h, as compared to toremifene (Tor) and estradiol (E2), as measured by *in vivo* increased uterine tissue weight (mg).

**Fig 14:** Effects of the indicated compounds on TRAP positive multinucleated osteoclasts.

**Fig 15:** Depicts circulating lipid levels ofprostate cancer patients subjected to ADT, after treatment by toremifene.

## DETAILED DESCRIPTION OF THE PRESENT INVENTION

The present invention provides, in some embodiments, novel NRBAs, which in some embodiments are SERM compounds, and compositions comprising the same.

This invention provides, in some embodiments, NRBAs, which in one embodiment are SERMs. In one embodiment, the phrase "Selective Estrogen Receptor Modulator" or "SERM" refers to a compound that affects estrogen receptor activity. In one embodiment, a SERM exhibits activity as an agonist, or, in another embodiment, as an antagonist, or in another embodiment, as a partial agonist, or in another embodiment, as a partial antagonist of the estrogen receptor. In one embodiment, the SERM exerts its effects on the estrogen receptor (e.g., ER$\alpha$, ER$\beta$ or ERRs) in a tissue-dependent manner. In some embodiments, the SERMs of this invention can act as estrogen receptor agonists in some tissues (e.g., bone, brain, and/or heart) and as antagonists in other tissue types, for example in the breast and/or uterine lining.

In one embodiment, a SERM of this invention will have an $IC_{50}$ or $EC_{50}$ with respect to ER$\alpha$ and/or ER$\beta$ of up to about 10 $\mu$M as determined using the ER$\alpha$ and/or ER$\beta$ transactivation assays, as known in the art, or, in other embodiments, as described herein (Example 1, 2). In some embodiments, the SERMs exhibit $IC_{50}$ or $EC_{50}$ values (as agonists or antagonists) of not more than about 5 $\mu$M. Representative compounds of the present invention have been discovered to exhibit agonist or antagonist activity with respect to the estrogen receptor. Compounds of the present invention exhibit, in some embodiments, an antagonist or agonist $IC_{50}$ or $EC_{50}$ with respect to ER$\alpha$ and/or ER$\beta$ of no more than about 5 $\mu$M, or in some embodiments, no more than about 500 nM, or in other embodiments, not more than about 1 nM, as measured in ER$\alpha$ and/or ER$\beta$ transactivation assays. The term "$IC_{50}$" refers, in some embodiments, to a concentration of the SERM which reduces the activity of a target (e.g., ER$\alpha$ or ER$\beta$) to half-maximal level. The term "$EC_{50}$" refers, in some embodiments, to a concentration of the SERM that produces a half-maximal effect

In some embodiments of this invention, the compounds of this invention are characterized by a structure comprising a phenyl group added to bisphenolic agonists, thus forming triphenyl agents. In some embodiments, the triphenyl groups are rigidly held by an amide bond. In addition to the triphenyl moiety, in some embodiments, the SERMs may be characterized by a structure comprising a basic side chain (tertiary amine), which in some embodiments, is present as an *N*-substituted ethanolamine sidechain appended to one or two of the phenolic ether groups.

***SERMs:***

In one embodiment, the present invention provides a SERM compound or its prodrug, analog, isomer, metabolite, derivative, pharmaceutically acceptable salt, pharmaceutical product, polymorph, crystal, impurity, *N*-oxide, ester, hydrate or any combination thereof, represented by the structure of formula I:

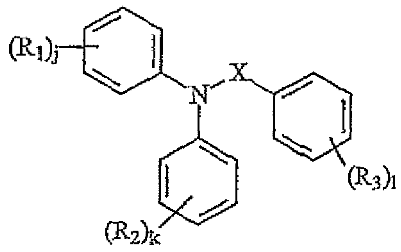

(I)

wherein

X is CO, CS, $(CH_2)_q$, branched alkyl, branched alkyl with haloalkyl side chain, haloalkyl, $(C)O(CH_2)_q$, SO, or $SO_2$;

$R_1$, $R_2$ and $R_3$ are independently, hydrogen, halogen, aldehyde, COOH, CHNOH, $CH{=}CHCO_2H$, hydroxyalkyl, hydroxyl, alkoxy, cyano, nitro, $CF_3$, $NH_2$, NHR, NHCOR, $N(R)_2$, sulfonamide, $SO_2R$, alkyl, aryl, protected hydroxyl, $OCH_2CH_2NR_4R_5$, Z-Alk-Q, Z-Alk-$NR_4R_5$, Z-Alk-heterocycle or $OCH_2CH_2$-heterocycle in which the heterocycle is a 3-7 membered substituted or unsubstituted heterocyclic ring, optionally aromatic,

or $R_1$, $R_2$ or $R_3$ together with the benzene ring to which the R-group is attached comprises a fused ring system represented by structure A

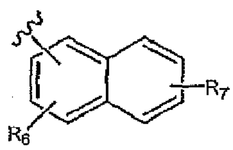

A

wherein

$R_6$ and $R_7$ are independently $R_1$, $R_2$ or $R_3$;

R is alkyl, hydrogen, haloalkyl, dihaloalkyl, trihaloalkyl, $CH_2F$, $CHF_2$, $CF_3$, $CF_2CF_3$, aryl, phenyl, halogen, alkenyl, CN, $NO_2$, or OH;

$R_4$ and $R_5$ are independently hydrogen, phenyl, an alkyl group of 1 to 6 carbon atoms, a 3 to 7 membered cycloalkyl, a 3 to 7 membered heterocycloalkyl, or a 3 to 7 membered heteroaryl group;

Z is O, NH, $CH_2$, or

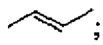 ;

Q is $SO_3H$, $CO_2H$, $CO_2R$, $NO_2$, tetrazole, $SO_2NH_2$, or $SO_2NHR$; j, k, l are independently 1-5;

q is 1-5;

Alk is linear alkyl of 1-7 carbons, branched alkyl of 1-7 carbons, or cyclic alkyl of 3-8 carbons; and

if X is $(CH_2)_q$, CO or $C(O)(CH_2)_q$, and $R_2$ is $OCH_2CH_2NR_4R_5$, or $OCH_2CH_2$-heterocycle when k is 1, then $R_1$ or $R_3$ is not hydrogen, lower alkyl (1-4 carbons), lower alkoxy (1-4 carbons), halogen, nitro or amino;

if X is $(CH_2)_q$, CO or $C(O)(CH_2)_q$, and $R_3$ is $OCH_2CH_2NR_4R_5$, or $OCH_2CH_2$-heterocycle when l is one, then $R_1$ or $R_2$ is not hydrogen, lower alkyl (1-4 carbons), lower alkoxy (1-4 carbons), halogen, nitro or amino.

In another embodiment, this invention provides a SERM compound or its prodrug, analog, isomer, metabolite, derivative, pharmaceutically acceptable salt, pharmaceutical product, polymorph, crystal, impurity, *N*-oxide, ester, hydrate or any combination thereof, represented by the structure of formula I:

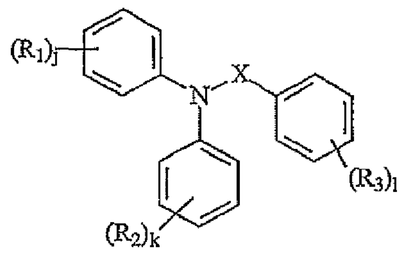

(I)

wherein

X is CS, branched alkyl, branched alkyl with haloalkyl side chain, haloalkyl, SO, or $SO_2$;

$R_1$, $R_2$ and $R_3$ are independently, hydrogen, halogen, aldehyde, COOH, CHNOH, $CH=CHCO_2H$, hydroxyalkyl, hydroxyl, alkoxy, cyano, nitro, $CF_3$, $NH_2$, NHR, NHCOR, $N(R)_2$, sulfonamide, $SO_2R$, alkyl, aryl, protected hydroxyl, $OCH_2CH_2NR_4R_5$, $Z(CH_2)_qQ$, Z-Alk-$NR_4R_5$, Z-Alk-heterocycle or $OCH_2CH_2$-heterocycle in which the heterocycle is a 3-7 membered substituted or unsubstituted heterocyclic ring, optionally aromatic,

or $R_1$, $R_2$ or $R_3$ together with the benzene ring to which the R-group is attached comprises a fused ring system represented by structure A

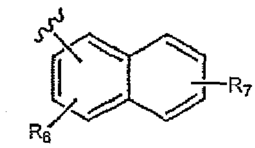

A

wherein $R_6$ or $R_7$ are independently $R_1$, $R_2$ or $R_3$;

R is alkyl, hydrogen, haloalkyl, dihaloalkyl, trihaloalkyl, $CH_2F$, $CHF_2$, $CF_3$, $CF_2CF_3$, aryl, phenyl, halogen, alkenyl, CN, $NO_2$, or OH;

$R_4$ and $R_5$ are independently hydrogen, phenyl, an alkyl group of I to 6 carbon atoms, a 3 to 7 membered cycloalkyl, a 3 to 7 membered heterocycloalkyl, or a 3 to 7 membered

heteroaryl group;

Z is O, NH, $CH_2$, or

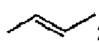;

Q is $SO_3H$, $CO_2H$, $CO_2R$, $NO_2$, tetrazole, $SO_2NH_2$, or $SO_2NHR$;

j, k, l are independently 1-5;

q is 1-5;

Alk is linear alkyl of 1-7 carbons, branched alkyl of 1-7 carbons, or cyclic alkyl of 3-8 carbons.

In another embodiment this invention provides a SERM compound or its prodrug, analog, isomer, metabolite, derivative, pharmaceutically acceptable salt, pharmaceutical product, polymorph, crystal, impurity, *N*-oxide, ester, hydrate or any combination thereof, represented by the structure of formula II:

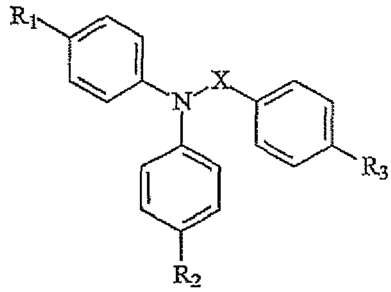

(II)

X is CO, CS, $(CH_2)_q$, branched alkyl, branched alkyl with haloalkyl, side chain, haloalkyl, $C(O)(CH_2)_q$, SO, or $SO_2$;
$R_1$, $R_2$ and $R_3$ are independently, hydrogen, halogen, aldehyde, COOH, CHNOH, $CH=CHCO_2H$, hydroxyalkyl, hydroxyl, alkoxy, cyano, nitro, $CF_3$, $NH_2$, NHR, NHCOR, $N(R)_2$, sulfonamide, $SO_2R$, alkyl, aryl, protected hydroxyl, $OCH_2CH_2NR_4R_5$, Z-Alk-Q, Z-Alk-$NR_4R_5$, Z-Alk-heterocycle or $OCH_2CH_2$-heterocycle in which the heterocycle is a 3-7 membered substituted or unsubstituted heterocyclic ring, optionally aromatic,
or $R_1$, $R_2$ or $R_3$ together with the benzene ring to which the R-group is attached comprises a fused ring system represented by structure A

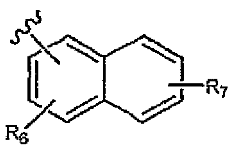

A

wherein
$R_6$ and $R_7$ are independently $R_1$, $R_2$ or $R_3$;
R is alkyl, hydrogen, haloalkyl, dihaloalkyl, trihaloalkyl, $CH_2F$, $CHF_2$, $CF_3$, $CF_2CF_3$, aryl, phenyl, halogen, alkenyl, CN, $NO_2$, or OH;
$R_4$ and $R_5$ are independently hydrogen, phenyl, an alkyl group of 1 to 6 carbon atoms, a 3 to 7 membered cycloalkyl, a 3 to 7 membered heterocycloalkyl, or a 3 to 7 membered heteroaryl group;
Z is O, NH, $CH_2$, or

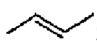;

Q is $SO_3H$, $CO_2H$, $CO_2R$, $NO_2$, tetrazole, $SO_2NH_2$, or $SO_2NHR$;
q is 1-5;
Alk is linear alkyl of 1-7 carbons, branched alkyl of 1-7 carbons, or cyclic alkyl of 3-8 carbons; and
if X is $(CR_2)_q$, CO or $C(O)(CH_2)_q$, and $R_2$ is $OCH_2CH_2NR_4R_5$, or $OCH_2CH_2$-heterocycle when k is 1, then $R_1$ or $R_3$ is not hydrogen, lower alkyl (1-4 carbons), lower alkoxy (1-4 carbons), halogen, nitro or amino;
if X is $(CH_2)_q$, CO or $C(O)(CH_2)_q$, and $R_3$ is $OCH_2CH_2NR_4R_5$, or $OCH_2CH_2$-heterocycle when I is one, then $R_1$ or $R_2$ is not hydrogen, lower alkyl (1-4 carbons), lower alkoxy (1-4 carbons), halogen, nitro or amino.

In another embodiment, according to this aspect of the invention, X is CO and $R_1$, $R_2$ and $R_3$ are OH, or in another embodiment X is CO and $R_1$ is $OCH_2CH_2$-piperidine HCl salt, $R_2$ is H and $R_3$ is OH.

In another embodiment this invention provides a SERM compound or its prodrug, analog, isomer, metabolite, derivative, pharmaceutically acceptable salt, pharmaceutical product, polymorph, crystal, impurity, *N*-oxide, ester, hydrate or any combination thereof, represented by the structure of formula II:

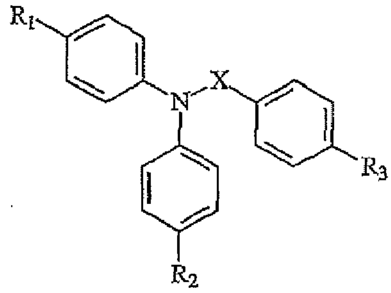

(II)

wherein

X is CS, branched alkyl, branched alkyl with haloalkyl side chain, haloalkyl, SO, or $SO_2$;

$R_1$, $R_2$ and $R_3$ are independently, hydrogen, halogen, aldehyde, COOH, CHNOH, $CH{=}CHCO_2H$, hydroxyalkyl, hydroxyl, alkoxy, cyano, nitro, $CF_3$, $NH_2$, NHR, NHCOR, $N(R)_2$, sulfonamide, $SO_2R$, alkyl, aryl, protected hydroxyl, $OCH_2CH_2NR_4R_5$, Z-Alk-Q, Z-Alk-$NR_4R_5$, Z-Alk-heterocycle or $OCH_2CH_2$-heterocycle in which the heterocycle is a 3-7 membered substituted or unsubstituted heterocyclic ring, optionally aromatic,

or $R_1$, $R_2$ or $R_3$ together with the benzene ring to which the R-group is attached comprises a fused ring system represented by structure A

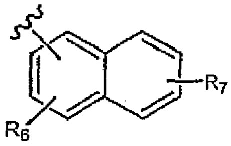

A

wherein $R_6$ and $R_7$ are independently $R_1$, $R_2$ or $R_3$;

R is alkyl, hydrogen, haloalkyl, dihaloalkyl, trihaloalkyl, $CH_2F$, $CHF_2$, $CF_3$, $CF_2CF_3$, aryl, phenyl, halogen, alkenyl, CN, $NO_2$, or OH;

$R_4$ and $R_5$ are independently hydrogen, phenyl, an alkyl group of 1 to 6 carbon atoms, a 3 to 7 membered cycloalkyl, a 3 to 7 membered heterocycloalkyl, or a 3 to 7 membered heteroaryl group;

Z is O, NH, $CH_2$, or

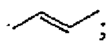;

Q is $SO_3H$, $CO_2H$, $CO_2R$, $NO_2$, tetrazole, $SO_2NH_2$, or $SO_2NHR$;

Alk is linear alkyl of 1-7 carbons, branched alkyl of 1-7 carbons, or cyclic alkyl of 3-8 carbons.

In another embodiment the present invention provides, a SERM compound or its prodrug, analog, isomer, metabolite, derivative, pharmaceutically acceptable salt, pharmaceutical product, polymorph, crystal, impurity, *N*-oxide, ester, hydrate or any combination thereof, represented by the structure of formula III:

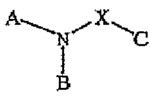

(III)

wherein

A is a ring selected from

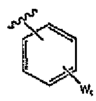 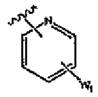 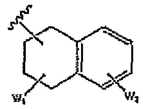

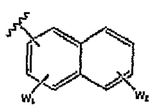 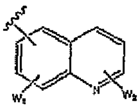 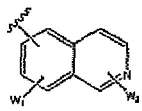

B is a ring selected from

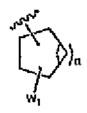 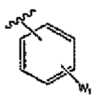 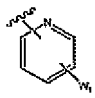 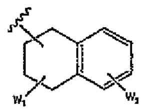

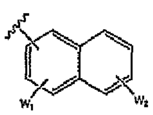 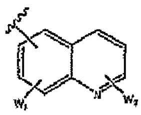 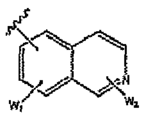

C is a ring selected from

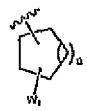 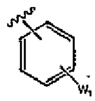 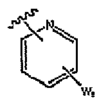 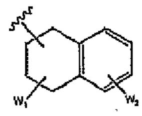

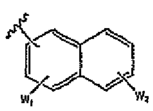 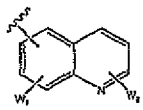 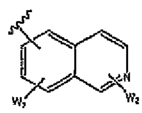

X is CO, CS, $(CH_2)_q$, branched alkyl, branched alkyl with haloalkyl side chain, haloalkyl, $C(O)(CH_2)_q$, SO, or $SO_2$;
$W_1$ and $W_2$ are independently, hydrogen, halogen, hydroxyl, aldehyde, COOH, CHNOH, $CH{=}CHCO_2H$, hydroxyalkyl, alkoxy, cyano, nitro, $CF_3$, $NH_2$, NHR, NHCOR, $N(R)_2$, sulfonamide, $SO_2R$, alkyl, aryl, protected hydroxyl, $OCH_2CH_2NR_4R_5$, Z-Alk-Q, Z-Alk-$NR_4R_5$, Z-Alk-heterocycle or $OCH_2CH_2$-heterocycle in which the heterocycle is a 3-7 membered substituted or unsubstituted heterocyclic ring, optionally aromatic;
R is alkyl, hydrogen, haloalkyl, dihaloalkyl, trihaloalkyl, $CH_2F$, $CHF_2$, $CF_3$, $CF_2CF_3$, aryl, phenyl, halogen, alkenyl, CN, $NO_2$, or OH;
$R_4$ and $R_5$ are independently hydrogen, phenyl, an alkyl group of 1 to 6 carbon atoms, a 3 to 7 membered cycloalkyl, a 3 to 7 membered heterocycloalkyl, or a 3 to 7 membered heteroaryl group;
Z is O, NH, $CH_2$, or

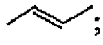;

Q is $SO_3H$, $CO_2H$, $CO_2R$, $NO_2$, tetrazole, $SO_2NH_2$, or $SO_2NHR$;
q is 1-5;
n is 0-5;
Alk is linear alkyl of 1-7 carbons, branched alkyl of 1-7 carbons, or cyclic alkyl of 3-8 carbons;
and A, B and C cannot simultaneously be a benzene ring; and
if X is $(CH_2)_q$, CO or $C(O)(CH_2)_q$, A is pyridyl ring and B and C are phenyl rings and C is substituted with $OCH_2CH_2NR_4R_5$, or $OCH_2CH_2$-heterocycle, then A or B is not substituted with hydrogen, lower alkyl (1-4 carbons), lower alkoxy (1-4 carbons), halogen, nitro or amino;
If X is $(CH_2)_q$, CO or $C(O)(CH_2)_q$, A is pyridyl ring and B and C are phenyl rings and B is substituted with $OCH_2CH_2NR_4R_5$, or $OCH_2CH_2$-heterocycle, then A or C is not substituted with hydrogen, lower alkyl (1-4 carbons), lower alkoxy (1-4

carbons), halogen, nitro or amino.

In another embodiment this invention provides a SERM compound or its prodrug, analog, isomer, metabolite, derivative, pharmaceutically acceptable salt, pharmaceutical product, polymorph, crystal, impurity, *N*-oxide, ester, hydrate or any combination thereof, represented by the structure of formula IV:

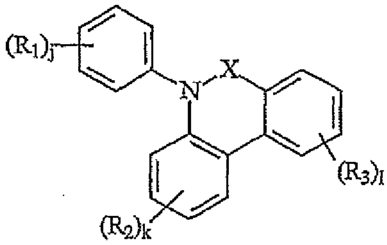

(IV)

wherein

X is CO, CS, $(CH_2)_q$, branched alkyl, branched alkyl with haloalkyl side chain, haloalkyl, $C(O)(CH_2)_q$, SO, or $SO_2$;

$R_1$, $R_2$ and $R_3$ are independently, hydrogen, halogen, hydroxyl, aldehyde, COOH, CHNOH, $CH=CHCO_2H$, hydroxyalkyl, alkoxy, cyano, nitro, $CF_3$, $NH_2$, NHR, NHCOR, $N(R)_2$, sulfonamide, $SO_2R$, alkyl, aryl, protected hydroxyl, $OCH_2CH_2NR_4R_5$, Z-Alk-Q, Z-Alk-$NR_4R_5$, Z-Alk-heterocycle or $OCH_2CH_2$-heterocycle in which the heterocycle is a 3-7 membered substituted or unsubstituted heterocyclic ring, optionally aromatic,

or $R_1$, $R_2$ and $R_3$ together with the benzene ring to which the R-group is attached comprises a fused ring system represented by structure A

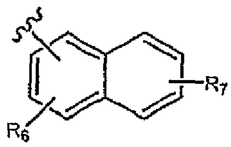

A

wherein

$R_6$ and $R_7$ are independently $R_1$, $R_2$ or $R_3$;

R is alkyl, hydrogen, haloalkyl, dihaloalkyl, trihaloalkyl, $CH_2F$, $CHF_2$, $CF_3$, $CF_2CF_3$, aryl, phenyl, halogen, alkenyl, CN, $NO_2$, or OH;

$R_4$ and $R_5$ are independently hydrogen, phenyl, an alkyl group of 1 to 6 carbon atoms, a 3 to 7 membered cycloalkyl, a 3 to 7 membered heterocycloalkyl, or a 3 to 7 membered heteroaryl group;

Z is O, NH, $CH_2$, or

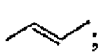;

Q is $SO_3H$, $CO_2H$, $CO_2R$, $NO_2$, tetrazole, $SO_2NH_2$, or $SO_2NHR$;

j, k, l are independently is 1-5;

q is 1-5;

Alk is linear alkyl of 1-7 carbons, branched alkyl of 1-7 carbons, or cyclic alkyl of 3-8 carbons.

In another embodiment, this invention provides, a SERM compound or its prodrug, analog, isomer, metabolite, derivative, pharmaceutically acceptable salt, pharmaceutical product, polymorph, crystal, impurity, *N*-oxide, ester, hydrate or any combination thereof, represented by the structure of formula V:

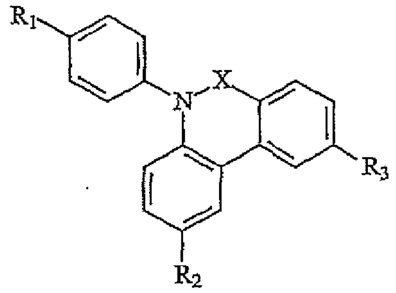

(V)

X is CO, CS, $(CH_2)_q$, branched alkyl, branched alkyl with haloalkyl side chain, haloalkyl, $C(O)(CH_2)_q$, SO, or $SO_2$;
$R_1$, $R_2$ and $R_3$ are independently, hydrogen, aldehyde, COOH, CHNOH, $CH{=}CHCO_2H$, hydroxyalkyl, halogen, hydroxyl, alkoxy, cyano, nitro, $CF_3$, $NH_2$, NHR, NHCOR, $N(R)_2$, sulfonamide, $SO_2R$, alkyl, aryl, protected hydroxyl group, $OCH_2CH_2NR_4R_5$, Z-Alk-Q, Z-Alk-$NR_4R_5$, Z-Alk-heterocycle or $OCH_2CH_2$-heterocycle in which the heterocycle is a 3-7 membered substituted or unsubstituted heterocyclic ring, optionally aromatic,
or $R_1$, $R_2$ and $R_3$ together with the benzene ring to which the R-group is attached comprises a fused ring system represented by structure A

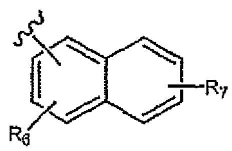
A

wherein
$R_6$ or $R_7$ are independently $R_1$, $R_2$ or $R_3$;
R is alkyl, hydrogen, haloalkyl, dihaloalkyl, trihaloalkyl, $CH_2F$, $CHF_2$, $CF_3$, $CF_2CF_3$, aryl, phenyl, halogen, CN, $NO_2$, alkenyl or OH;
$R_4$ and $R_5$ are independently hydrogen, phenyl, an alkyl group of 1 to 6 carbon atoms, a 3 to 7 membered cycloalkyl, a 3 to 7 membered heterocycloalkyl, or a 3 to 7 membered heteroaryl group;
Z is O, NH, $CH_2$, or

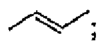;

Q is $SO_3H$, $CO_2H$, $CO_2R$, $NO_2$, tetrazole, $SO_2NH_2$, or $SO_2NHR$;
q is 1-5;
Alk is linear alkyl of 1-7 carbons, branched alkyl of 1-7 carbons, or cyclic alkyl of 3-8 carbons.

In another embodiment, this invention provides a SERM compound or its prodrug, analog, isomer, metabolite, derivative, pharmaceutically acceptable salt, pharmaceutical product, polymorph, crystal, impurity, *N*-oxide, ester, hydrate or any combination thereof, represented by the structure of formula VI:

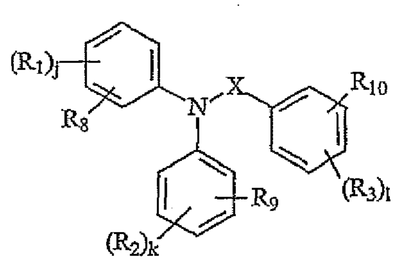

(VI)

wherein

X is CO, CS, $(CH_2)_q$, branched alkyl, branched alkyl with haloalkyl side chain, haloalkyl, $C(O)(CH_2)_q$, SO, or $SO_2$;

$R_1$, $R_2$, $R_3$, $R_8$, $R_9$, and $R_{10}$ are independently hydrogen, halogen, aldehyde, COOH, CHNOH, $CH{=}CHCO_2H$, hydroxyalkyl, hydroxyl, alkoxy, cyano, nitro, $CF_3$, $NH_2$, NHR, NHCOR, $N(R)_2$, sulfonamide, $SO_2R$, alkyl, aryl, protected hydroxyl, $OCH_2CH_2NR_4R_5$, Z-Alk-Q, Z-Alk-$NR_4R_5$, Z-Alk-heterocycle or $OCH_2CH_2$-heterocycle in which the heterocycle is a 3-7 membered substituted or unsubstituted heterocyclic ring, optionally aromatic,

or $R_1$, $R_2$ or $R_3$ together with the benzene ring to which the R-group is attached comprises a fused ring system represented by structure A

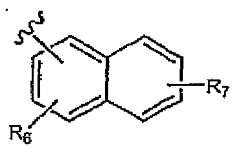

A

wherein

$R_6$ and $R_7$ are independently is $R_1$, $R_2$ or $R_3$;

R is alkyl, hydrogen, haloalkyl, dihaloalkyl, trihaloalkyl, $CH_2F$, $CHF_2$, $CF_3$, $CF_2CF_3$, aryl, phenyl, halogen, CN, $NO_2$, alkenyl or OH;

$R_4$ and $R_5$ are independently hydrogen, phenyl, an alkyl group of 1 to 6 carbon atoms, a 3 to 7 membered cycloalkyl, a 3 to 7 membered heterocycloalkyl, or a 3 to 7 membered heteroaryl group;

Z is O, NH, $CH_2$, or

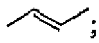 ;

Q is $SO_3H$, $CO_2H$, $CO_2R$, $NO_2$, tetrazole, $SO_2NH_2$, or $SO_2NHR$;

j, k, 1 are independently 1-4;

q is 1-5;

Alk is linear alkyl of 1-7 carbons, branched alkyl of 1-7 carbons, or cyclic alkyl of 3-8 carbons; and

if X is $(CH_2)_q$, CO or $C(O)(CH_2)_q$ , and $R_2$ is $OCH_2CH_2NR_4R_5$, or $OCH_2CH_2$-heterocycle when k is 1, and $R_9$ is hydrogen, lower alkyl (1-4 carbons), lower alkoxy (1-4 carbons), halogen, nitro or amino, then $R_1$ or $R_3$ is not hydrogen, lower alkyl (1-4 carbons), lower alkoxy (1-4carbons), halogen, nitro or amino;

if X is $(CH_2)_q$, CO or $C(O)(CH_2)_q$, and $R_3$ is $OCH_2CH_2NR_4R_5$, or $OCH_2CH_2$-heterocycle when 1 is 1, and $R_{10}$ is hydrogen, lower alkyl (1-4 carbons), lower alkoxy (1-4 carbons), halogen, nitro or amino;

then $R_1$ or $R_2$ is not hydrogen, lower alkyl (1-4 carbons), lower alkoxy (1-4 carbons), halogen, nitro or amino.

In one embodiment of this invention , the 3-7 membered substituted or unsubstituted heterocyclic ring, optionally aromatic, is represented by the structure of formula B:

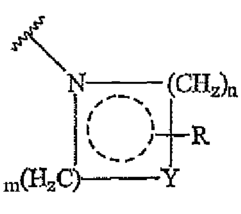

B

wherein Y is $CH_2$, CH, bond, O, S, NH, N or NR;

R is alkyl, hydrogen, haloalkyl, dihaloalkyl, trihaloalkyl, $CH_2F$, $CHF_2$, $CF_3$, $CF_2CF_3$, aryl, phenyl, halogen, CN, $NO_2$, alkenyl or OH;

if B is aryl then z is 1; and if B is cycloalkyl z is 2;

m is 0-4;

n is 0-4;

wherein m and n cannot both be zero.

In one embodiment B is substituted or unsubstituted piperidine; in another embodiment B is substituted or unsubstituted pyrrolidine; in another embodiment B is substituted or unsubstituted morpholine; in another embodiment

B is substituted or unsubstituted piperazine.

In one embodiment this invention provides a SERM compound or its prodrug, analog, isomer, metabolite, derivative, pharmaceutically acceptable salt, pharmaceutical product, polymorph, crystal, impurity, *N*-oxide, ester, hydrate or any combination thereof, represented by the structure of formula VII:

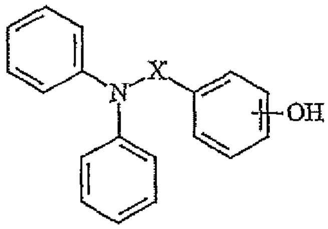

(VII)

wherein X is CS, $(CH_2)_q$, branched alkyl, branched alkyl with haloalkyl side chain, haloalkyl, $C(O)(CH_2)_q$, SO, or $SO_2$; or X is CO, and OH is meta or ortho.

In another embodiment this invention provides a selective estrogen receptor modulator (SERM) compound or its prodrug, analog, isomer, metabolite, derivative, pharmaceutically acceptable salt, pharmaceutical product, polymorph, crystal, impurity, *N*-oxide, ester, hydrate or any combination thereof, represented by the structure of formula VIII:

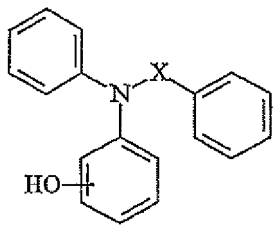

(VIII)

wherein X is CS, $(CH_2)_q$, branched alkyl, branched alkyl with haloalkyl side chain, haloalkyl, $C(O)(CH_2)_q$, SO, or $SO_2$; or X is CO, and OH is meta or ortho.

In another embodiment, this invention provides a SERM compound or its prodrug, analog, isomer, metabolite, derivative, pharmaceutically acceptable salt, pharmaceutical product, polymorph, crystal, impurity, *N*-oxide, ester, hydrate or any combination thereof, represented by the structure of formula IX:

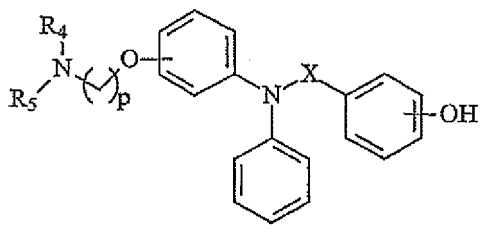

IX

wherein X is CO, CS, $(CH_2)_q$, branched alkyl, branched alkyl with haloalkyl side chain, haloalkyl, $C(O)(CH_2)_q$, SO, or $SO_2$; $R_4$ and $R_5$ are independently hydrogen, phenyl, an alkyl group of 1 to 6 carbon atoms, a 3 to 7 membered cycloalkyl, a 3 to 7 membered heterocycloalkyl, a 3 to 7 membered heteroaryl group, or $R_4$ and $R_5$ form together with the nitrogen atom a 3-7 heterocyclic ring, optionally aromatic, is represented by the structure of formula B:

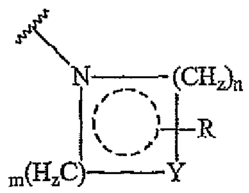

B

wherein Y is $CH_2$, CH, bond, O, S, NH, N or NR;
if B is aryl then z is 1; and if B is cycloalkyl z is 2;
m is 0-4;
is 0-4;
wherein m and n cannot both be zero;
q is 1-5;
p is 1-4;
R is alkyl, hydrogen, haloalkyl, dihaloalkyl, trihaloalkyl, $CH_2F$, $CHF_2$, $CF_3$, $CF_2CF_3$, aryl, phenyl, halogen, alkenyl, CN, $NO_2$ or OH.

In one embodiment B is substituted or unsubstituted piperidine; in another embodiment B is substituted or unsubstituted pyrrolidine; in another embodiment B is substituted or unsubstituted morpholine; in another embodiment B is substituted or unsubstituted piperazine.

In another embodiment, this invention provides a SERM compound or its prodrug, analog, isomer, metabolite, derivative, pharmaceutically acceptable salt, pharmaceutical product, polymorph, crystal, impurity, *N*-oxide, ester, hydrate or any combination thereof, represented by the structure of formula X:

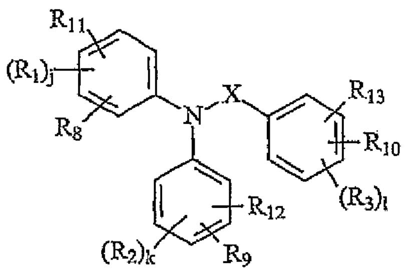

X

wherein
X is CO, CS, $(CH_2)_q$, branched alkyl, branched alkyl with haloalkyl side chain, haloalkyl, $C(O)(CH_2)_q$, SO, or $SO_2$;
$R_1$, $R_2$, $R_3$, $R_8$, $R_9$, $R_{10}$, $R_{11}$, $R_{12}$ and $R_{13}$ are independently hydrogen, halogen, aldehyde, COOH, CHNOH, $CH{=}CHCO_2H$, hydroxyalkyl, hydroxyl, alkoxy, cyano, nitro, $CF_3$, $NH_2$, NHR, NHCOR, $N(R)_2$, sulfonamide, $SO_2R$, alkyl, aryl, protected hydroxyl, $OCH_2CH_2NR_4R_5$, Z-Alk-Q, Z-Alk-$NR_4R_5$, Z-Alk-heterocycle or $OCH_2CH_2$-heterocycle in which the heterocycle is a 3-7 membered substituted or unsubstituted heterocyclic ring, optionally aromatic,
or $R_1$, $R_2$ or $R_3$ together with the benzene ring to which the R-group is attached comprises a fused ring system represented by structure A

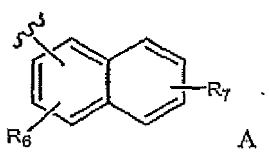

A

wherein
$R_6$ and $R_7$ are independently is $R_1$, $R_2$ or $R_3$;
R is alkyl, hydrogen, haloalkyl, dihaloalkyl, trihaloalkyl, $CH_2F$, $CHF_2$, $CF_3$, $CF_2CF_3$, aryl, phenyl, halogen, CN, $NO_2$, alkenyl or OH;
$R_4$ and $R_5$ are independently hydrogen, phenyl, an alkyl group of 1 to 6 carbon atoms, a 3 to 7 membered cycloalkyl, a

3 to 7 membered heterocycloalkyl, or a 3 to 7 membered heteroaryl group; or $R_4$ and $R_5$ form together with the nitrogen atom a 3-7 heterocyclic ring, optionally aromatic, is represented by the structure of formula B:

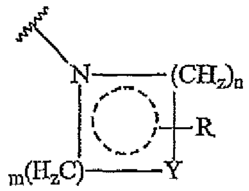

B

wherein Y is $CH_2$, CH, bond, O, S, NH, N or NR;
ifB is aryl then z is 1; and if B is cycloalkyl z is 2;
m is 0-4;
n is 0-4;
wherein m and n cannot both be zero;
q is 1-5;
p is 1-4;
R is alkyl, hydrogen, haloalkyl, dihaloalkyl, trihaloalkyl, $CH_2F$, $CHF_2$, $CF_3$, $CF_2CF_3$, aryl, phenyl, halogen, alkenyl, CN, $NO_2$ or OH.
Z is O, NH, $CH_2$, or

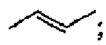 ;

Q is $SO_3H$, $CO_2H$, $CO_2R$, $NO_2$, tetrazole, $SO_2NH_2$, or $SO_2NHR$;
j, k, l are independently 1-3;
q is 1-5;
Alk is linear alkyl of 1-7 carbons, branched alkyl of 1-7 carbons, or cyclic alkyl of 3-8 carbons; and
if X is $(CH_2)_q$, CO or $C(O)(CH_2)_q$, and $R_2$ is $OCH_2CH_2NR_4R_5$, or $OCH_2CH_2$-heterocycle when k is 1, and $R_9$ and $R_{12}$ are hydrogen, lower alkyl (1-4 carbons), lower alkoxy (1-4 carbons), halogen, nitro or amino, then
$R_1$ or $R_3$ is not hydrogen, lower alkyl (1-4 carbons), lower alkoxy (1-4 carbons), halogen, nitro or amino;
if X is $(CH_2)_q$, CO or $C(O)(CH_2)_q$ , and $R_3$ is $OCH_2CH_2NR_4R_5$, or $OCH_2CH_2$-heterocycle when 1 is one, and $R_{10}$ and $R_{13}$ are hydrogen, lower alkyl (1-4 carbons), lower alkoxy (1-4 carbons), halogen, nitro or amino;
then $R_1$ or $R_2$ is not hydrogen, lower alkyl (1-4 carbons), lower alkoxy (1-4 carbons), halogen, nitro or amino.

In one embodiment the NRBA or SERM compound of this invention is 4-methoxy-*N,N*-bis-(4-methoxyphenyl)-benzamide (2a). In one embodiment the NRBA or SERM compound of this invention is 3-methoxy-*N,N*-bis-(4-methoxyphenyl)-benzamide (2b). In one embodiment the NRBA or SERM compound of this invention is 4-methoxy-*N*-(4-methoxyphenyl)-*N*-(3-methoxyphenyl)-benzamide (2c). In one embodiment the NRBA or SERM compound of this invention is *N,N*-bis-(4-methoxyphenyl)-benzamide (2d). In one embodiment the NRBA or SERM compound of this invention is 4-Methoxy-*N,N*-diphenyl-benzamide (2e). In one embodiment the NRBA or SERM compound of this invention is 3-methoxy-*N,N*-diphenyl-benzamide (2f). In one embodiment the NRBA or SERM compound of this invention is *N,N*-diphenyl-benzamide (2g). In one embodiment the NRBA or SERM compound of this invention is *N*-(4-methoxyphenyl)-*N*-phenyl-benzamide (2h). In one embodiment the NRBA or SERM compound of this invention is *N*-(3-methoxyphenyl)-*N*-phenylbenzamide (2i). In one embodiment the NRBA or SERM compound of this invention is 4-methoxy-*N*-(4-methoxyphenyl)-*N*-phenyl-benzamide (2j). In one embodiment the NRBA or SERM compound of this invention is 4-methoxy-*N*-(3-methoxyphenyl)-*N*-phenyl-benzamide (2k). In one embodiment the NRBA or SERM compound of this invention is *N,N*-bis(4-methoxyphenyl)-4-fluorobenzamide (21). In one embodiment the NRBA or SERM compound of this invention is 4-methoxy-*N,N*-diphenyl-sulfonamide (2m). In one embodiment the NRBA or SERM compound of this invention is 4-methoxy-*N*-(4-methoxyphenyl)-*N*-(4-fluorophenyl)-benzamide (2n). In one embodiment the NRBA or SERM compound of this invention is 4-methoxy-*N*-(4-methoxyphenyl)-*N*-(1-naphthyl)-benzamide (2o). In one embodiment the NRBA or SERM compound of this invention is *N*-(4-methoxyphenyl)-*N*-(4-benzyloxyphenyl)-1-naphthylamide (2p). In one embodiment the NRBA or SERM compound of this invention is 4-chloro-*N*-(4-methoxyphenyl)-*N*-(4-benzyloxyphenyl)-benzamide (2q). In one embodiment the NRBA or SERM compound of this invention is 4-cyano-*N*-(4-methoxyphenyl)-*N*-(4-benzyloxyphenyl)-benzamide (2r). In one embodiment the NRBA or SERM compound of this invention is *N*-(4-methoxyphenyl)-*N*-(4-benzyloxyphenyl)-2-naphthylamide (2s). In one embodiment the NRBA or SERM compound of this invention is 4-(benzyloxy)-*N*-[4-(benzyloxy)phenyl]-*N*-(4-methoxyphenyl)benzamide (2t). In one embodiment the NRBA or SERM compound of this invention is *N*-[4-(benzyloxy)phenyl]-4-methoxy-*N*-(4-methoxyphenyl) benzamide (2u). In one embodiment the NRBA or SERM compound of this invention is *N*-[4-(benzyloxy)phenyl]-*N*-biphenyl-4-yl-4-methoxybenzamide (2v). In one embodiment the NRBA or SERM compound of this invention is 4-cyano-*N*-(4-methoxyphenyl)-*N*-phenylbenzamide (2w). In one embodiment the NRBA or SERM compound of this invention is 3-methoxy-*N*-(4-methoxyphenyl)-*N*-phenylbenzamide (2x). In one embodiment the NRBA or SERM compound of this invention is 4-cyano-*N*-(3-methoxyphenyl)-*N*-phenylbenzamide (2y). In one embodiment the NRBA or SERM compound of this invention is 4-cyano-*N,N*-diphenylbenzamide (2z). In another embodiment the NRBA or SERM compound of this invention is 4-hydroxy-*N,N*-bis-(4-hydroxyphenyl)-benzamide (3a). In one embodiment the NRBA or SERM compound of this invention is 3-hydroxy-*N*-bis-(4-hydroxyphenyl)-benzamide (3b). In one embodiment the NRBA or SERM compound of this invention is 4-hydroxy-*N*-(4-hydroxyphenyl)-*N*-(3-hydroxyphenyl)-benzamide (3c). In one embodiment the NRBA or SERM compound of this invention is *N,N*-bis-(4-hydroxyphenyl)-benzamide (3d). In another embodiment the NRBA or SERM compound of this invention is 4-hydroxy-*N,N*-diphenyl-benzamide (3e). In another embodiment the NRBA or SERM compound of this invention is 3-hydroxy-*N,N*-diphenyl-benzamide (3f). In another embodiment the NRBA or SERM compound of this invention is *N*-(4-hydroxyphenyl)-*N*-phenylbenzamide (3g). In another embodiment the NRBA or SERM compound of this invention is *N*-(3-hydroxyphenyl)-*N*-phenyl-benzamide (3h). In another embodiment the NRBA or SERM compound of this invention is 4-hydroxy-*N*-(4-hydroxyphenyl)-*N*-phenyl-benzamide (3i). In one embodiment the NRBA or SERM compound of this invention is 4-hydroxy-*N*-(3-hydroxyphenyl)-*N*-phenyl-benzamide (3j). In one embodiment the NRBA or SERM compound of this invention is *N,N*-bis(4-hydroxyphenyl)-4-fluoro-benzamide (3k). In one embodiment the NRBA or SERM compound of this invention is 4-hydroxy-*N,N*-diphenyl-phenyl-sulfonamide (31). In another embodiment the NRBA or SERM compound of this invention is 4-hydroxy-*N*-(4-hydroxyphenyl)-*N*-(fluorophenyl)-benzamide (3m). In another -embodiment the NRBA or SERM compound of this invention is *N,N*-bis(4-hydroxyphenyl)-1-naphthylamide (3n). In one embodiment the NRBA or SERM compound of this invention is 4-hydroxy-*N*-(1-naphthyl)-*N*-(4-hydroxyphenyl)-benzamide (3o). In one embodiment the NRBA or SERM compound of this invention is 4-cyano-*N,N*-bis(4-hydroxyphenyl)-benzamide (3p). In one embodiment the NRBA or SERM compound of this invention is 3-Cyano-*N,N*-bis(4-hydroxyphenyl)-benzamide (3q).In another embodiment the NRBA or SERM compound of this invention is *N,N*-bis(4-hydroxyphenyl)-2-naphthylamide (3r). In one embodiment the NRBA or SERM compound of this invention is 4-cyano-*N*-[4-(2-piperidin-1-ylethoxy)-phenyl]-*N*-(4-hydroxyphenyl)-benzamide (3s). In another embodiment the NRBA or SERM compound of this invention is 3-chloro-*N*-[4-(2-piperidin-1-ylethoxy)-phenyl]-*N*-(4-hydroxyphenyl)-benzamide (3t). In one embodiment the NRBA or SERM compound of this invention is *N*-biphenyl-4-yl-*N*-(4-hydroxyphenyl)-4-methoxybenzamide (3u). In one embodiment the NRBA or SERM compound of this invention is *N*-biphenyl-4-yl-4-hydroxy-*N*-(4-hydroxyphenyl)-benzamide (3v). In one embodiment the NRBA or SERM compound of this invention is 4-hydroxy-*N*-(4-hydroxyphenyl)-*N*-[4-(2-piperidin-1-ylethoxy)phenyl]-benzamide (3w). In another embodiment the NRBA or SERM compound of this invention is 3-hydroxy-*N*-(4-hydroxyphenyl)-*N*-phenylbenzamide (3x). In one embodiment the NRBA or SERM compound of this invention is *N*-biphenyl-4-yl-4-hydroxy-*N*-[4-(2-piperidin-1-ylethoxy)phenyl]-benzamide (3y). In another embodiment the NRBA or SERM compound of this invention is *N*-(4-hydxoxyphenyl)-*N*-[4-(2-piperidin-1-ylethoxy)-phenyl]-benzamide (4a). In another embodiment the NRBA or SERM compound of this invention is *N*-(phenyl)-*N*-[4-(2-piperidin-1-ylethoxy)-phenyl]-benzamide (4b). In another embodiment the NRBA or SERM compound of this invention is *N,N*-diphenyl-[3-(2-piperidinylethoxy)]-benzamide hydrochloride (4c). In another embodiment the NRBA or SERM compound of this invention is *N,N*-diphenyl-[3-(2-piperidinylethoxy)]-benzamide hydrochloride (4d). In another embodiment the NRBA or SERM compound of this invention is *N*-(4-hydroxyplienyl)-*N*-phenyl-[4-(2-piperidin-1-ylethoxy)]-benzamide hydrochloride (4e). In one embodiment the NRBA or SERM compound of this invention is *N,N*-diphenyl-bis[4-(2-piperidin-1-ylethoxy)-phenyl]-sulfonamide hydrochloride (4f). In another embodiment the NRBA or SERM compound of this invention is *N*-(4-fluorophenyl)-*N*-[4-hydroxyphenyl]-[4-(2-piperidin-1-ylethoxy)]-benzamide (4g). In another embodiment the NRBA or SERM compound of this invention is *N*-(4-hydroxyphenyl)-*N*-[4-(2-piperidin-1-ylethoxy)-phenyl]-4-fluroro-benzamide hydrochloride (4h). In one embodiment the NRBA or SERM compound of this invention is 3-(2-piperidin-1-ylethoxy)-*N,N*-bis(4-hydroxyphenyl)-benzamide (4i). In another embodiment the NRBA or SERM compound of this invention is 4-cyano-*N*-[4-(2-piperidin-1-ylethoxy)-phenyl]-*N*-(4-mathoxyphenyl)-benzamide (4j). In another embodiment the NRBA or SERM compound of this invention is 4-chloro-*N*-[4-(2-piperidin-1-ylethoxy)-phenyl]-*N*-(4-methoxyphenyl)-benzamide (4k) In one embodiment the NRBA or SERM compound of this invention is 4-cyano-*N*-[4-(2-piperidin-1-ylethoxy)-phenyl]-*N*-(4-methoxyphenyl)-benzamide (41). In another embodiment the NRBA or SERM compound of this invention is 3-chloro-*N*-[4-(2-piperidin-1-ylethoxy)-phenyl]-*N*-(4-methoxyphenyl)-benzamide (4m). In another embodiment the NRBA or SERM compound of this invention is 4-methoxy-*N*-(4-methoxyphenyl)-*N*-[4-(2-piperidin-1-ylethoxy)phenyl]-benzamide (4n). In one embodiment the NRBA or SERM compound of this invention is *N*-biphenyl-4-yl-*N*-(4-hydroxyphenyl)-4-(2-piperidin-1-ylethoxy)-benzamide (4o). In another embodiment the NRBA or SERM compound of this invention is 4-methoxy-*N*-phenyl-*N*-[4-(2-piperidin-1-ylethoxy)phenyl]-benzamide (4p). In another embodiment the NRBA or SERM compound of this invention is *N*-(4-hydroxyphenyl)-*N*-phenyl-3-(2-piperidin-1-ylethoxy)-benzamide (4q). In another embodiment the NRBA or SERM compound of this invention is *N*-(4-fluorophenyl)-*N*-[4-(2-piperidin-1-ylethoxy)-phenyl]-[4-(2-piperidin-1-yl-ethoxy)]-benzamide dihydrochloride (4r). In another embodiment the NRBA or SERM compound of this invention is *N,N*-bis[4-(2-piperidin-1-ylethoxy)-phenyl]-4-fluoro-benzamide dihydrochloride (4s). In another embodiment the NRBA or SERM compound of this invention is *N,N*-bis[4-(2-piperidin-1-ylethoxy)-phenyl]-benzamide dihydrochloride (4t). In one embodiment the NRBA or SERM compound of this invention is *N*-[4-(2-piperidin-1-ylethoxy)-phenyl]- *N*-phenyl-[4-(2-piperidin-1-ylethoxy)]-benzamide dihydrochloride (4u). In one embodiment the NRBA or SERM compound of this invention is 4-chloro-*N*-[4-hydroxyphenyl]-*N*-(4-mathoxyphenyl)-benzamide (5a). In one embodiment the NRBA or SERM compound of this invention is 4-cyano-*N*-[4-hydroxyphenyl]-*N*-(4-methoxyphenyl)-benzamide (5b). In one embodiment the NRBA or SERM compound of this invention is 3-chloro-*N*-[4-hydroxyphenyl]-*N*-(4-methoxyphenyl)-benzamide (5c). In one embodiment the NRBA or SERM compound of this invention is 4-hydroxy-*N*-(4-hydroxyphenyl)-*N*-(4-mothoxyphenyl)-benzamide (5d). In one embodiment the NRBA or SERM compound of this invention is *N*-(4-hydroxyphenyl)-4-methoxy-*N*-(4-methoxyphenyl)-benzamide (5e). In one embodiment the NRBA or SERM compound of this invention is 2-(*N*-(4-methoxyphenyl)-4-methylphenylsulfonamido)ethyl 4-methylbenzenesulfonate (6a). In one embodiment the NRBA or SERM compound of this invention is (*R*)-3-bromo-2-hydroxy-*N*-(4-methoxyphenyl)-2-methylpropanamide (6b). In one embodiment the NRBA or SERM compound of this invention is (*S*)-2-hydroxy-*N*,3-bis(4-methoxyphenyl)-2-methylpropanamide (6c). In one embodiment the NRBA or SERM compound of this invention is (*S*)-2-hydroxy-3-(4-methoxyphenoxy)-*N*-(4-methoxyphenyl)-2-methylpropanamide (6d). In one embodiment the NRBA or SERM compound of this invention is (*R*)-3-bromo-2-hydroxy-*N*-(4-hydroxyphenyl)-2-methylpropanamide (6e). In one embodiment the NRBA or SERM compound of this invention is (*S*)-2-hydroxy-3-(4-hydroxyphenoxy)-*N*-(4-hydroxyphenyl)-2-methylpropanamide (6f). In one embodiment the NRBA or SERM compound of this invention is (*S*)-2-hydroxy-*N*,3-bis(4-hydroxyphenyl)-2-methylpropanamide (6g). In another embodiment the NRBA or SERM compound of this invention is 5-[4-methoxy-phenyl]-5*H*-phenanthridin-6-one (7a). In another embodiment the NRBA or SERM compound of this invention is 5-[4-hydroxy-phenyl]-5*H*-phenanthridin-6-one (7b). In one embodiment the NRBA or SERM compound of this invention is 5-[4-(2-piperidin-1-ylethoxy)-phenyl]-5*H*-phenanthridin-6-one (7c). In another embodiment the NRBA or SERM compound of this invention is cyclohexane-carboxylic acid bis(4-hydroxyphenyl)-amide(8b). In another embodiment the NRBA or SERM compound of this invention is 4-cyano-*N*-(4-hydroxyphenyl)-*N*-phenylbenzamide (10a). In another embodiment the NRBA or SERM compound of this invention is *N*-(biphenyl-4-yl)-4-cyano-*N*-(4-mothoxyphenyl)-benzamide (10b). In another embodiment the NRBA or SERM compound of this invention is *N,N*-bis(4-hydroxyphenyl)biphenyl-4-carboxamide (10c). In another embodiment the NRBA or SERM compound of this invention is *N,N*-bis(4-hydroxyphenyl)-3,4-dimethylbenzamide (10d). In another embodiment the NRBA or SERM compound of this invention is *N*-(biphenyl-4-yl)-4-cyano-*N*-(4-hydroxyphenyl)-benzamide (10e). In another embodiment the NRBA or SERM compound of this invention is 3-fluoro-4-hydroxy-*N*-(4-hydroxyphenyl)-*N*-phenylbenzamide (10f). In another embodiment the NRBA or SERM compound of this invention is 4-fluoro-3-hydroxy-*N,N*-bis(4-hydroxyphenyl)-benzamide (10g). In another embodiment the NRBA or SERM compound of this invention is 4-hydroxy-*N,N*-bis(4-hydroxyphenyl)-3,5-dimethylbenzamide (10i). In another embodiment the NRBA or SERM compound of this invention is *N,N*-bis(4-hydroxyphenyl)-2,3-dimethylbenzamide (10j). In another embodiment the NRBA or SERM compound of this invention is 3-fluoro-4-hydroxy-*N,N*-bis(4-hydroxyphenyl)-benzamide (10k). In another embodiment the NRBA or SERM compound of this invention is *N,N*-bis(4-hydroxyphenyl)-4-propylbenzamide (101). In another embodiment the NRBA or SERM compound of this invention is 3,4-dihydroxy-*N,N*-bis(4-hydroxyphenyl)-benzamide (10m). In another embodiment the NRBA or SERM compound of this invention is 4-hydroxy-*N,N*-bis(4-hydroxyphenyl)-3-methylbenzamide (10n). In another embodiment the NRBA or SERM compound of this invention is *N*-(4-hydroxyphenyl)-*N*-(4-(2-(piperidin-1-yl)ethoxy)phenyl)-4-propylbenzamide (10o). In another embodiment the NRBA or SERM compound of this invention is *N*-(4-hydroxyphenyl)-2,3-dimethyl-*N*-(4-(2-(piperidin-1-yl)ethoxy) phenyl)-benzamide (10p). In another embodiment the NRBA or SERM compound of this invention is *N,N*-bis(4-hydroxyphenyl)-2,4-dimethylbenzamide (10q). In another embodiment the NRBA or SERM compound of this invention is *N, N*-bis(4-hydroxyphenyl)-3,5-dimethylbenzamide (10r). In another embodiment the NRBA or SERM compound of this invention is *N,N*-bis(4-hydroxyphenyl)-4-methylbenzamide (10s). In another embodiment the NRBA or SERM compound of this invention is 4,4'-(2,3-dimethyl-benzylazanediyl)diphenol (10t). In another embodiment the NRBA or SERM compound of this invention is 4-formyl-*N,N*-bis(4-hydroxyphenyl)-benzamide (10u). In another embodiment the NRBA or SERM compound of this invention is N-cyclohexyl-4-hydroxy-N-(4-hydroxyphenyl)benzamide (10w). In another embodiment the NRBA or SERM compound of this invention is 4-((4-fluorophenyl)(4-hydroxybenzyl)amino)phenol (10x). In another embodiment the NRBA or SERM compound of this invention is *N*-(4-(2-(dimethylamino)ethoxy)phenyl)-*N*-(4-hydroxyphenyl)-benzamide (10y). In another embodiment the NRBA or SERM compound of this invention is 3-Cyano-*N*-(4-hydroxyphenyl)-*N*-(4-(2-(piperidin-1-yl)ethoxy)phenyl)-benzamide (10z). In another embodiment the NRBA or SERM compound of this invention is *N*-(4-hydroxyphenyl)-*N*-(4-(2-(pyrrolidin-1-yl)ethoxy)phenyl)benzamide (11a). In another embodiment the NRBA or SERM compound of this invention is *N,N*-bis(4-hydroxyphenyl)-4-(trifluoromethyl)-benzamide (11b). In another embodiment the NRBA or SERM compound of this invention is *N*-(4-hydroxyphenyl)-*N*-(4-(2-(piperidin-1-yl)ethoxy)phenyl)-4-(trifluoromethyl)-benzamide (11c). In another embodiment the NRBA or SERM compound of this invention is *N,N*-bis(4-hydroxyphenyl)-4-nitro-benzamide (11d). In another embodiment the NRBA or SERM compound of this invention is 3-fluoro-*N,N*-bis(4-hydroxyphenyl)-benzamide (11e). In another embodiment the NRBA or SERM compound of this invention is *N*-(4-hydroxyphenyl)-*N*-(4-(2-(piperidin-1-yl)ethoxy)phenyl)-1-naphthamide (11f). In another embodiment the NRBA or SERM compound of this invention is 3-fluoxo-*N*-(4-hydroxy-phenyl)-*N*-(4-(2-(piperidin-1-yl)ethoxy)phenyl)benzamide (11g). In another embodiment the NRBA or SERM compound of this invention is *N*-(4-hydroxyphenyl)-4-nitro-*N*-(4-(2-(piperidin-1-yl)ethoxy)phenyl)benzamide (11h). In another embodiment the NRBA or SERM compound of this invention is *N,N*-bis(4-hydroxyphenyl)-4-methoxy-1-naphthamide (11i). In another embodiment the NRBA or SERM compound of this invention is *N*-(4-hydroxyphenyl)-*N*-(4-(2-(piperidin-1-yl)ethoxy)phenyl)-2-naphthamide (11j). In another embodiment the NRBA or SERM compound of this invention is 2-hydroxy-*N,N,*2-tris(4-hydroxyphenyl)-propanamide (11k). In another embodiment the NRBA or SERM compound of this invention is 4-((hydroxyimino)methyl)-*N,N*-bis(4-hydroxyphenyl)benzamide (111), In another embodiment the NRBA or SERM compound of this invention is *N*-(4-hydroxyphenyl)-2,4-dimethyl-*N*-(4-(2-(piperidin-1-yl)ethoxy)phenyl)benzamide (11m). In another embodiment the NRBA or SERM compound of this invention is *N*-(4-hydroxyphenyl)-3,5-dimethyl-*N*-(4-(2-(piperidin-1-yl)ethoxy)phenyl)benzamide (11n). In another embodiment the NRBA or SERM compound of this invention is 4-((2,3-dimethylbenzyl)(4-(2-(piperidin-1-yl)ethoxy)phenyl)amino)phenol (11o). In another embodiment the NRBA or SERM compound of this invention is *N,N*-bis(4-hydroxyphenyl)-4-pentylbenzamide (11p). In another embodiment the NRBA or SERM compound of this invention is *N*-(4-hydroxyphenyl)-4-pentyl-*N*-(4-(2-(piperidin-1-yl)ethoxy)phenyl)benzamide (11q). In another embodiment the NRBA or SERM compound of this invention is 4-*tert*-butyl-*N,N*-bis(4-hydroxyphenyl)benzamide (11r). In another embodiment the NRBA or SERM compound of this invention is 4-*tert*-butyl-*N*-(4-hydroxyphenyl)-*N*-(4-(2-(piperidin-1-yl)ethoxy)phenyl)benzamide (11s). In another embodiment the NRBA or SERM compound of this invention is 3-{4-[bis-(4-hydroxy-phenyl)-carbamoyl]-phenyl}-acrylic acid (11t). In another embodiment the NRBA or SERM compound of this invention is 3-{4-[bis-(4-hydroxy-phenyl)-carbamoyl]-phenyl}-propionic acid (11u). In another embodiment the NRBA or SERM compound of this invention is *N,N*-bis-(4-hydroxy-phenyl)-4-(3-hydroxy-propyl)-benzamide (11v). In another embodiment the NRBA or SERM compound of this invention is *N*-(4-hydroxyphenyl)-4-(3-hydroxypropyl)-*N*-(4-methoxyphenyl)-benzamide (11w). In another embodiment the NRBA or SERM compound of this invention is 4-fluoro-*N,N*-bis(4-hydroxyphenyl)-2-(trifluoromethyl)-benzamide (11x). In another embodiment the NRBA or SERM compound of this invention is 3-fluoro-*N*-(4-fluorophenyl)-4-hydroxy-*N*-(4-hydroxyphenyl)benzamide (11y). In another embodiment the NRBA or SERM compound of this invention is *N*-(4-hydroxyphenyl)-4-methyl-*N*-(4-(2-(piperidin-1-yl)ethoxy)phenyl)benzamide (11z). In another embodiment the NRBA or SERM compound of this invention is *N,N*-bis(4-hydroxyphenyl)-isonicotin-amide (11aa). In another embodiment the NRBA or SERM compound of this invention is *N*-(4-hydroxyphenyl)-*N*-(4-(2-(piperidin-1-yl) ethoxy)phenyl)-isonicotinamide (11ab). In another embodiment, this invention provides a composition comprising a NRBA or SERM compound as described herein, or any combination thereof.

In another embodiment, the SERM compound for use in the methods of this invention may be represented by the structure of formula XI:

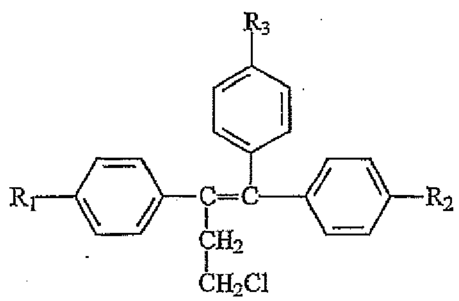

(XI)

wherein $R_1$ and $R_2$, which can be the same or different, are H or OH, $R_3$ is $OCH_2CH_2OH$, $OCH_2CH_2NR_4R_5$, wherein $R_4$ and $R_5$, which can be the same or different, are H, an alkyl group of 1 to about 4 carbon atoms or forms together with the nitrogen a cyclic 5-8 membered ring; and their pharmaceutically acceptable carrier, diluents, salts, esters, or N-oxides, and mixtures thereof.

In another embodiment, the SERM compound is toremifene

The term "alkyl" refers, in one embodiment, to a saturated aliphatic hydrocarbon, including straight-chain, branched-chain and cyclic alkyl groups. In one embodiment, the alkyl group has 1-12 carbons. In another embodiment, the alkyl group has 1-7 carbons. In another embodiment, the alkyl group has 1-6 carbons. In another embodiment, the alkyl group has 1-4 carbons. In another embodiment, the cyclic alkyl group has 3-8 carbons. In another embodiment, the cyclic alkyl group has 3-12 carbons. In another embodiment, the branched alkyl is an alkyl substituted by alkyl side chains of 1 to 5 carbons. In another embodiment, the branched alkyl is an alkyl substituted by haloalkyl side chains of

1 to 5 carbons. The alkyl group may be unsubstituted or substituted by a halogen, haloalkyl, hydroxyl, alkoxy carbonyl, amido, alkylamido, dialkylamido, nitro, amino, alkylamino, dialkylamino, carboxyl, thio and/or thioalkyl.

An "alkenyl" group refers, in another embodiment, to an unsaturated hydrocarbon, including straight chain, branched chain and cyclic groups having one or more double bonds. The alkenyl group may have one double bond, two double bonds, three double bonds, etc. In another embodiment, the alkenyl group has 2-12 carbons. In another embodiment, the alkenyl group has 2-6 carbons. In another embodiment, the alkenyl group has 2-4 carbons. Examples of alkenyl groups are ethenyl, propenyl, butenyl, cyclohexenyl, etc. The alkenyl group may be unsubstituted or substituted by a halogen, hydroxy, alkoxy carbonyl, amido, alkylamido, dialkylamido, nitro, amino, alkylamino, dialkylamino, carboxyl, thio and/or thioalkyl.

A "haloalkyl" group refers, in another embodiment, to an alkyl group as defined above, which is substituted by one or more halogen atoms, e.g. by F, Cl, Br or I.

An "aryl" group refers, in another embodiment, to an aromatic group having at least one carbocyclic aromatic group or heterocyclic aromatic group, which may be unsubstituted or substituted by one or more groups selected from halogen, haloalkyl, hydroxy, alkoxy carbonyl, amido, alkylamido, dialkylamido, nitro, amino, alkylamino, dialkylamino, carboxy or thio or thioalkyl. Nonlimiting examples of aryl rings are phenyl, naphthyl, pyranyl, pyrrolyl, pyrazinyl, pyrimidinyl, pyrazolyl, pyridinyl, furanyl, thiophenyl, thiazolyl, imidazolyl, isoxazolyl, and the like.

A "hydroxyl" group refers, in another embodiment, to an OH group. It is understood by a person skilled in the art that when $R_1$, $R_2$ or $R_3$ in the compounds of the present invention is OR, then R is not OH.

In one embodiment, the term "halo" refers to a halogen, such as F, Cl, Br or I.

In another embodiment, the phrase "phenol" refers to an alcohol (OH) derivative of benzene.

A "heterocycle" group refers, in one embodiment, to a ring structure comprising in addition to carbon atoms, sulfur, oxygen, nitrogen or any combination thereof, as part of the ring. In another embodiment the heterocycle is a 3-12 membered ring. In another embodiment the heterocycle is a 6 membered ring. In another embodiment the heterocycle is a 5-7 membered ring. In another embodiment the heterocycle is a 4-8 membered ring. In another embodiment, the heterocycle group may be unsubstituted or substituted by a halogen, haloalkyl, hydroxyl, alkoxy, carbonyl, amido, alkylamido, dialkylamido, cyano, nitro, $CO_2H$, amino, alkylamino, dialkylamino, carboxyl, thio and/or thioalkyl. In another embodiment, the heterocycle ring maybe fused to another saturated or unsaturated cycloalkyl or heterocyclic 3-8 membered ring. In another embodiment, the heterocyclic ring is a saturated ring. In another embodiment, the heterocyclic ring is an unsaturated ring.

Reference to protected hydroxyl, in some embodiments, includes the incorporation of a substituent bonded to the oxygen moiety of the benzene ring, wherein the substituent may be readily removed. In some embodiments, phenolic protecting groups may comprise a: methyl ether, methoxymethyl (MOM) ether, benzoyloxymethyl (BOM) ether, methoxyethoxymethyl (MEM) ether, 2-(trimethylsilyl)ethoxymethyl(SEM) ether, methylthiomethyl (MTM) ether, phenylthiomethyl (PTM) ether, azidomethyl ether, cyanomethyl ether, 2,2-dichloro-1,1-difluoroethyl ether, 2-chloroethyl ether, 2-bromoethyl ether, tetrahydropyranyl (THP) ether, 1-ethoxyethyl (EE) ether, phenacyl ether, 4-bromophenacyl ether, cyclopropylmethyl ether, allyl ether, propargyl ether, isopropyl ether, cyclohexyl ether, *t*-butyl ether, benzyl ether, 2,6-dimethylbenzyl ether, 4-methoxybenzyl ether, o-nitrobenzyl ether, 2,6 dichlorobenzyl ether, 3,4 dichlorobenzyl ether, 4-(dimethylamino)carbonylbenzyl ether, 4-methylsulfinylbenzyl ether, 4-anthrylmethyl ether, 4-picolyl ether, heptafluoro-p-tolyl, tetrafluoro-4-pyridyl ether, trimethylsilyl (TMS) ether , *t*-butyldimethylsilyl (TBDMS) ether, t-butyldiphenylsilyl (TBDPS) ether, triisopropylsilyl (TIPS) ether, aryl formate, arylacetate, aryl levulinate, arylpivaloate, aryl benzoate, aryl 9-fluorencarboxylate, aryl methyl carbonate, 1-adamantyl carbonate, *t*-butyl carbonate, 4-methylsulfinylbenzyl carbonate, 2,4-dimethylpent-3-yl carbonate, aryl 2,2,2 trichloroethyl carbonate, aryl benzyl carbonate, aryl carbamate, dimethylphosphinyl ester (Dmp-OAr), dimethylphosphinothionyl ester (Mpt-OAr), diphenylphosphinothionyl ester (Dpt-OAr), aryl methanesulfonate, aryl toluenesulfonate or aryl 2-formylbenzenesulfonate.

In one embodiment, this invention provides a NRBA or SERM compound and/or analog, derivative, isomer, metabolite, pharmaceutically acceptable salt, pharmaceutical product, hydrate, *N*-oxide, prodrug, ester, polymorph, impurity or crystal or combinations thereof. In one embodiment, this invention provides an analog of the NRBA or SERM compound. In another embodiment, this invention provides a derivative of the NRBA or SERM compound. In another embodiment, this invention provides an isomer of the NRBA or SERM compound. In another embodiment, this invention provides a metabolite of the NRBA or SERM compound. In another embodiment, this invention provides a pharmaceutically acceptable salt of the NRBA or SERM compound. In another embodiment, this invention provides a pharmaceutical product of the NRBA or SERM compound. In another embodiment, this invention provides a hydrate of the NRBA or SERM compound. In another embodiment, this invention provides an *N*-oxide of the NRBA or SERM compound. In another embodiment, this invention provides a prodrug of the NRBA or SERM compound. In another embodiment, this invention provides an ester of the NRBA or SERM compound. In another embodiment, this invention provides a polymorph of the NRBA or SERM compound. In another embodiment, this invention provides a crystal of the NRBA or SERM compound. In another embodiment, this invention provides an impurity of the NRBA or SERM compound. In another embodiment, this invention provides composition comprising a NRBA or SERM compound, as described herein, or, in

another embodiment, a combination of an analog, derivative, isomer, metabolite, pharmaceutically acceptable salt, pharmaceutical product, hydrate, *N*-oxide, prodrug, polymorph, ester, impurity or crystal of the NRBA or SERM compounds of the present invention.

In one embodiment, the term "isomer" includes, but is not limited to, optical isomers and analogs, structural isomers and analogs, conformational isomers and analogs, and the like.

In one embodiment, the term "isomer" is meant to encompass optical isomers of the NRBA or SERM compound. It will be appreciated by those skilled in the art that the NRBA or SERMs of the present invention contain at least one chiral center. Accordingly, the NRBA or SERMs used in the methods of the present invention may exist in, and be isolated in, optically-active or racemic forms. Some compounds may also exhibit polymorphism. It is to be understood that the present invention encompasses any racemic, optically-active, polymorphic, or stereroisomeric form, or mixtures thereof, and use of these for any application is to be considered within the scope of this invention.

In one embodiment, the NRBAs or SERMs are the pure (R)-isomers. In another embodiment, the NRBAs or SERMs are the pure (*S*)-isomers. In another embodiment, the NRBAs or SERMs are a mixture of the (R) and the (*S*) isomers. In another embodiment, the NRBAs or SERMs are a racemic mixture comprising an equal amount of the (R) and the (*S*) isomers. It is well known in the art how to prepare optically-active forms (for example, by resolution of the racemic form by recrystallization techniques, by synthesis from optically-active starting materials, by chiral synthesis, or by chromatographic separation using a chiral stationary phase), and such methods are to be considered as part of this invention. In some embodiments, synthesis of such racemic forms may be accomplished by methods described and exemplified herein, or via appropriate modification thereof, as will be understood by one skilled in the art.

In one embodiment, the NRBAs or SERMs are the pure (*E*)-isomers. In another embodiment, the NRBAs or SERMs are the pure (Z)-isomers. In another embodiment, the NRBAs or SERMs are a mixture of the (*E*) and the (*Z*) isomers.

The invention includes "pharmaceutically acceptable salts" of the NRBAs or SERMs of this invention, which may be produced, in one embodiment, using an amino-substituted NRBA or SERM and organic and inorganic acids, for example, citric acid and hydrochloric acid. In one embodiment, the pharmaceutically acceptable salt of a NRBA compound comprising a piperidine ring is an HCl salt. In another embodiment, the pharmaceutically acceptable salt of a NRBA compound comprising a pyrrolidine ring is an HCl salt. In another embodiment, the pharmaceutically acceptable salt of a NRBA compound comprising a morpholine ring is an HCl salt. In another embodiment, the pharmaceutically acceptable salt of a NRBA compound comprising a piperazine ring is an HCl salt.

Pharmaceutically acceptable salts can be prepared from the phenolic compounds, in other embodiments, by treatment with inorganic bases, for example, sodium hydroxide. In another embodiment, esters of the phenolic compounds can be made with aliphatic and aromatic carboxylic acids, for example, acetic acid and benzoic acid esters.

This invention provides, in some embodiments, derivatives of the NRBA or SERM compounds. In one embodiment, the term "derivatives" refers to ether derivatives, acid derivatives, amide derivatives, ester derivatives or others, as known in the art. In another embodiment, this invention further includes hydrates of the NRBA or SERM compounds. In one embodiment, the term "hydrate" refers to hemihydrate, monohydrate, dihydrate, trihydrate or others, as known in the art.

This invention provides, in other embodiments, metabolites of the NRBA or SERM compounds. In one embodiment, the term "metabolite" refers to any substance produced from another substance by metabolism or a metabolic process.

In some embodiments, a NRBA or SERM of this invention will comprise the compounds listed in Table 1. In some embodiments, the NRBAs or SERMs of this invention will have an affinity for a nuclear hormone receptor, with varying affinity. In some embodiments of this invention, NRBAs or SERMs of this invention will vary in terms of their activity, for example, some NRBAs or SERMs possessing greater anabolic activity, some exhibiting greater activity with regard to anti-estrogenic activity, etc. It is to be understood that all such NRBAs or SERMs are to be considered as part of this invention.

In some embodiments, the NRBAs or SERMs of this invention may exibit affinity for or binding to a nuclear receptor, which in some embodiments, is an estrogen receptor $\alpha$ and/or estrogen receptor $\beta$ molecule. In some embodiments, the NRBAs or SERMs of this invention may exhibit agonist activity. In some embodiments, the NRBAs or SERMs of this invention may exhibit antagonist activity. Agonist and antagonist activity for representative NRBAs are exemplified in the Examples herein, wherein such agonist and/or antagonist activity under specific experimental conditions is provided, representing only some embodiments of this invention. It is to be understood that while the indicated compounds may exhibit a particular activity (for example, compound 3v is an agonist) under the experimental conditions employed, as a function, in some embodiments of the particular cells utilized, etc., such compounds may possess alternate, varied, or partial activity in different experimental settings. In some embodiments, the NRBAs or SERMs of this invention may exhibit agonist activity for a particular receptor, and antagonist activity for a different receptor, or vice versa, or in some embodiments, the NRBAs or SERMs of this invention may exhibit agonist activity for a particular receptor under certain experimental conditions, yet exhibit antagonist activity for the same receptor under different experimental conditions, or

vice versa, or in some embodiments, the NRBAs or SERMs of this invention may exibit agonist activity for a particular receptor in a particular tissue, yet exhibit antagonist activity for the same receptor in a different tissue, or vice versa, etc.

Steroid nuclear hormone receptors are known to have rapid, tissue-specific effects that are mediated by cell-surface and cytosolic receptors through protein-protein interaction or phosphorylation of kinases, which are known as non-genomic effects. For instance, SERMs are known to have distinct rapid effects in the cardiovascular and central nervous systems which may be mediated by distinct receptors. Putative receptors for these non-genomic effects include a variety of G-protein coupled receptors (GPCRs) such as GPR130 for SERMs, as well as cell-membrane associated or cytosolic nuclear receptors. NRBA and SERMs of this invention may also bind to receptors involved in these non-genomic effects allowing differential pharmacological exploitation of genomic, non-genomic, and tissue-selective steroid receptor activities. As such these NRBA and SERMs may have a wide variety of specific and targeted steroid responses broadening their potential to have beneficial medical properties

In some embodiments, a NRBA of this invention is a non-genomic agonist, or in some embodiments, a non-genomic antagonist, or in some embodiments, a non-genomic partial agonist of a nuclear receptor. In some embodiments, the NRBAs of this invention are tissue selective, non-genomic nuclear receptors, such as for example, estrogen or androgen receptor agonists, or in some embodiments, tissue selective, non-genomic nuclear receptor antagonists, or in some embodiments, tissue selective, non-genomic nuclear receptor partial agonists. In some embodiments, the NRBAs of this invention are non-selective non-genomic nuclear receptors, such as for example, estrogen or androgen receptor agonists, or in some embodiments, non-selective non-genomic nuclear receptor antagonists, or in some embodiments, non-selective non-genomic nuclear receptor partial agonists. In some embodiments, the NRBAs of this invention are non-selective genomic nuclear receptors, such as for example, estrogen or androgen receptor agonists, or in some embodiments, antagonists, or in some embodiments, partial agonists. In some embodiments, the NRBAs of this invention are tissue selective genomic nuclear receptor modulators, such as for example, estrogen or androgen receptor agonists, or in some embodiments, antagonists, or in some embodiments, partial agonists. In some embodiments, the NRBAs of this invention are genomic agents which selectively transactivate nuclear receptor-regulated genes. In some embodiments, selective transactivation is in a tissue selective manner. In some embodiments, the NRBAs of this invention are genomic agents which selectively transrepress nuclear receptor-regulated genes. In some embodiments, selective tranrepression is in a tissue selective manner,

This invention provides, in other embodiments, pharmaceutical products of the NRBA or SERM compounds. The term "pharmaceutical product" refers, in other embodiments, to a composition suitable for pharmaceutical use (pharmaceutical composition), for example, as described herein.

In one embodiment, this invention provides a method of binding any NRBA or SERM compound of this invention to an estrogen receptor or an estrogen related receptors, comprising the step of contacting an estrogen receptor with said NRBA or SERM. In another embodiment, this invention provides a method of binding any NRBA or SERM compound of this invention to a nuclear hormone receptor or one related thereto.

***<u>COMPOSITIONS:</u>***

In some embodiments, any of the compositions of this invention will comprise a NRBA or SERM compound, in any form or embodiment as described herein. In some embodiments, any of the compositions of this invention will consist of a NRBA or SERM compound, in any form or embodiment as described herein. In some embodiments, of the compositions of this invention will consist essentially of a NRBA or SERM compound, in any form or embodiment as described herein. In some embodiments, the term "comprise" refers to the inclusion of the indicated active agent, such as the NRBA or SERM compound, as well as inclusion of other active agents, and pharmaceutically acceptable carriers, excipients, emollients, stabilizers, etc., as are known in the pharmaceutical industry. In some embodiments, the term "consisting essentially of" refers to a composition, whose only active ingredient is the indicated active ingredient, however, other compounds may be included which are for stabilizing, preserving, etc. the formulation, but are not involved directly in the therapeutic effect of the indicated active ingredient. In some embodiments, the term "consisting essentially of" may refer to components which facilitate the release of the active ingredient, or other active ingredients, however the primary compound mediating a therapeutic effect is the indicated active ingredient. In some embodiments, the term "consisting" refers to a composition, which contains the active ingredient and a pharmaceutically acceptable carrier or excipient.

In another embodiment, the invention provides a composition comprising a NRBA or SERM, as herein described, or its prodrug, analog, isomer, metabolite, derivative, pharmaceutically acceptable salt, pharmaceutical product, polymorph, crystal, impurity, *N*-oxide, ester, hydrate or any combination thereof and a suitable carrier or diluent.

An active component can be formulated into the composition as neutralized pharmaceutically acceptable salt forms. Pharmaceutically acceptable salts include the acid addition salts, which are formed with inorganic acids such as, for example, hydrochloric or phosphoric acids, or such organic acids as acetic, oxalic, tartaric, mandelic, and the like. Salts formed from the free carboxyl groups can also be derived from inorganic bases such as, for example, sodium,

potassium, ammonium, calcium, or ferric hydroxides, and such organic bases as isopropylamine, trimethylamine, 2-ethylamino ethanol, histidine, procaine, and the like.

The compositions of the present invention are formulated in one embodiment for oral delivery, wherein the active compounds may be incorporated with excipients and used in the form of ingestible tablets, buccal tables, troches, capsules, elixirs, suspensions, syrups, wafers, and the like. The tablets, troches, pills, capsules and the like may also contain the following: a binder, as gum tragacanth, acacia, cornstarch, or gelatin; excipients, such as dicalcium phosphate; a disintegrating agent, such as corn starch, potato starch, alginic acid and the like; a lubricant, such as magnesium stearate; and a sweetening agent, such as sucrose, lactose or saccharin may be added or a flavoring agent, such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar, or both. Syrup of elixir may contain the active compound, sucrose as a sweetening agent methyl, and propylparabens as preservatives, a dye and flavoring, such as cherry or orange flavor. In addition, the active compounds may be incorporated into sustained-release, pulsed release, controlled release or postponed release preparations and formulations.

In another embodiment, the compositions of this invention comprise one or more, pharmaceutically acceptable carrier materials.

In one embodiment, the carriers for use within such compositions are biocompatible, and in another embodiment, biodegradable. In other embodiments, the formulation may provide a relatively constant level of release of one active component. In other embodiments, however, a more rapid rate of release immediately upon administration may be desired. In other embodiments, release of active compounds may be event-triggered. The events triggering the release of the active compounds may be the same in one embodiment, or different in another embodiment. Events triggering the release of the active components may be exposure to moisture in one embodiment, lower pH in another embodiment, or temperature threshold in another embodiment. The formulation of such compositions is well within the level of ordinary skill in the art using known techniques. Illustrative carriers useful in this regard include microparticles of poly(lactide-co-glycolide), polyacrylate, latex, starch, cellulose, dextran and the like. Other illustrative postponed-release carriers include supramolecular biovectors, which comprise a non-liquid hydrophilic core (e.g., a cross-linked polysaccharide or oligosaccharide) and, optionally, an external layer comprising an amphiphilic compound, such as phospholipids. The amount of active compound contained in one embodiment, within a sustained release formulation depends upon the site of administration, the rate and expected duration of release and the nature of the condition to be treated suppressed or inhibited.

In one embodiment it will be desirable to deliver the compositions disclosed herein parenterally, intravenously, intramuscularly, or even intraperitoneally. Such approaches are well known to the skilled artisan, some of which are further described, for example, in U.S. Pat. No. 5,543,158; U.S. Pat. No. 5,641,515 and U.S. Pat. No. 5,399,363, all of which are fully incorporated by reference. In certain embodiments, solutions of the active compounds as free base or pharmacologically acceptable salts may be prepared in water suitably mixed with a surfactant, such as hydroxypropylcellulose. Dispersions may also be prepared in glycerol, liquid polyethylene glycols, and mixtures thereof and in oils. It must be stable under the conditions of manufacture and storage and must be preserved against the contaminating action of microorganisms, such as bacteria and fungi.

In another embodiment, it will be preferable to include isotonic agents, for example, sugars or sodium chloride. In other embodiments, prolonged absorption of the injectable compositions will be desirable. Prolonged absorption of the injectable compositions can be brought about by the use of agents delaying absorption, for example, aluminum monostearate and gelatin, in the compositions.

Parenteral vehicles include in certain embodiments sodium chloride solution, Ringer's dextrose, dextrose and sodium chloride, lactated Ringer's and fixed oils. Intravenous vehicles include fluid and nutrient replenishers, electrolyte replenishers such as those based on Ringer's dextrose, and the like. Preservatives and other additives may also be present, such as, for example, antimicrobials antioxidants, collating agents, inert gases and the like.

In some embodiments, NRBAs or SERMs of this invention may be administered at various dosages to a subject, which in one embodiment, is a human subject In one embodiment, the NRBA or SERM is administered at a dosage of 0.1 - 200 mg per day. In one embodiment, the NRBA or SERM is administered at a dose of 0.1 - 10 mg, or in another embodiment, 0.1 - 25 mg, or in another embodiment, 0.1- 50 mg, or in another embodiment, 0.3 - 15 mg, or in another embodiment, 0.3 - 30 mg, or in another embodiment, 0.5 - 25 mg, or in another embodiment, 0.5 - 50 mg, or in another embodiment, 0.75 - 15 mg, or in another embodiment, 0.75 - 60 mg, or in another embodiment, 1 - 5 mg, or in another embodiment, 1 - 20 mg, or in another embodiment, 3 - 15 mg, or in another embodiment, 1 - 30 mg, or in another embodiment, 30 - 50 mg, or in another embodiment, 30 - 75 mg, or in another embodiment, 100 - 2000 mg. In some embodiments, the NRBAs or SERMs may be administered at different dosages, as a function of time, or disease/symptom/condition severity, or age, or other factors, as will be appreciated by one skilled in the art.

The NRBAs or SERMs of this invention may be administered at various dosages. In one embodiment, the NRBA or SERM is administered at a dosage of 1 mg. In another embodiment the NRBA or SERM is administered at a dosage of 5 mg, or in another embodiment, 3 mg, or in another embodiment 10 mg, or in another embodiment 15 mg,

or in another embodiment 20 mg, or in another embodiment 25 mg, or in another embodiment 30 mg, or in another embodiment 35 mg, or in another embodiment 40 mg, or in another embodiment 45 mg, or in another embodiment 50 mg, or in another embodiment 55 mg, or in another embodiment 60 mg, or in another embodiment 65 mg, or in another embodiment 70 mg, or in another embodiment 75 mg, or in another embodiment 80 mg, or in another embodiment 85 mg, or in another embodiment 90 mg, or in another embodiment 95 mg or in another embodiment 100 mg.

In one embodiment, the methods of this invention make use of the NRBA or SERM as described at a dose as described herein, given orally, once a day.

While the compounds of the invention can be administered as the sole active pharmaceutical agent, they can also be used in combination with one or more other compound as described herein, and/or in combination with other agents used in the treatment and/or prevention of the diseases, disorders and/or conditions, as described herein, as will be understood by one skilled in the art. In another embodiment, the compounds of the present invention can be administered sequentially with one or more such agents to provide sustained therapeutic and prophylactic effects. In another embodiment, the compounds may be administered via different routes, at different times, or a combination thereof. It is to be understood that any means of administering combined therapies which include the NRBAs or SERMs of this invention are to be considered as part of this invention, in an embodiment thereof.

Suitable agents include, but are not limited to, other SERMs as well as traditional estrogen agonists and antagonists. Representative agents useful in combination with the compounds of the invention include, for example, tamoxifen, 4-hydroxytamoxifen, idoxifene, toremifene, 4-hydroxytoremifene ospemifene, droloxifene, raloxifene, arzoxifene, bazedoxifene, PPT (1,3,5-tris(4-hydroxyphenyl)-4-propyl-1*H*-pyrazole), DPN [2,3-bis(4-hydroxyphenyl)propanenitrile], lasofoxifene, pipendoxifene, EM-800, EM-652, nafoxidine, zindoxifene, tesmilifene, miproxifene phosphate, RU58,688, EM 139, ICI 164,384, ICI 182,780, clomiphene, MER-25, diethylstibestrol, coumestrol, genistein, GW5638, LY353581, zuclomiphene, enclomiphene, delmadinone acetate, tibolone, DPPE, (*N,N*-diethyl-2-(4-(phenylmethyl)-phenoxy}ethanamine), TSE-424, WAY-070, WAY—292, WAY-818, prinaberel, ERB-041, WAY-397, WAY-244, ERB-196, WAY-169122, MF-101, ERb-002, ERB-037, ERB-017, BE-1060, BE-380, BE-381, WAY-358, [$^{18}$F]FEDNP, LSN-500307, AA-102, CT-101, CT-102, VG-101, and the like.

In some embodiments, other agents that can be combined with one or more of the compounds of the invention include aromatase inhibitors such as, but not limited to, letrozole, anastrazole, atamestane, fadrozole, minamestane, exemestane, plomestane, liarozole, NKS-01, vorozole, YM-511, finrozole, 4-hydroxyandrostenedione, aminogluethimide, rogletimide.

In some embodiments, other agents useful for combination with the compounds of the invention include, but are not limited to antineoplastic agents, such as alkylating agents. In some embodiments, the NRBAs or SERMs of this invention may be administered in conjunction with antibiotics, hormonal antineoplastics and/or antimetabolites. Examples of useful alkylating agents include alkyl sulfonates such as busulfan, improsulfan and piposulfan; aziridines, such as a benzodizepa, carboquone, meturedepa and uredepa; ethylenimines and methylmelamines such as altretamine, triethylenemelamine, triethylenephosphoramide, triethylenethiophosphoramide and trimethylolmelamine; nitrogen mustards such as chlorambucil, chlomaphazine, cyclophosphamide, estramustine, iphosphamide, mechlorethamine, mechlorethamine oxide hydrochloride, melphalan, novembichine, phenesterine, prednimustine, trofosfamide, and uracil mustard; nitroso ureas, such as carmustine, chlorozotocin, fotemustine, lomustine, nimustine, ranimustine, dacarbazine, mannomustine, mitobronitol, mitolactol and pipobroman. More such agents will be known to those having skill in the medicinal chemistry and oncology arts.

In some embodiments, other agents suitable for combination with the compounds of the present invention include protein synthesis inhibitors such as abrin, aurintricarboxylic acid, chloramphenicol, coficin E3, cycloheximide, diphtheria toxin, edeine A, emetine, erythromycin, ethionine, fluoride, 5-fluorohyptophan, fusidic acid, guanylyl methylene diphosphonate and guanylyl imidodiphosphate, kanamycin, kasugamycin, kirromycin, and *O*-methyl threonine, modeccin, neomycin, norvaline, pactamycin, paromomycin, puromycin, ricin, $\alpha$-sarcin, shiga toxin, showdomycin, sparsomycin, spectinomycin, streptomycin, tetracycline, thiostrepton and trimethoprim. Inhibitors of DNA synthesis, including alkylating agents such as dimethyl sulfate, mitomycin C, nitrogen and sulfur mustards, MNNG and NMS; intercalating agents such as acridine dyes, actinomycins, adriamycin, anthracenes, benzopyrene, ethidium bromide, propidium diiodide-intertwining, and agents such as distamycin and netropsin, can also be combined with compounds of the present invention in pharmaceutical compositions. DNA base analogs such as acyclovir, adenine, $\beta$-1-*D*-arabinoside, amethopterin, aminopterin, 2-aminopurine, aphidicolin, 8-azaguanine, azaserine, 6-azauracil, 2'-azido-2'-deoxynucliosides, 5-bromodeoxycytidine, cytosine, $\beta$-1-*D*-arabinoside, diazooxynorleucine, dideoxynucleosides, 5-fluorodeoxycytidine, 5-fluorodeoxyuridine, 5-fluorouracil, hydroxyurea and 6-mercaptopurine also can be used in combination therapies with the compounds of the invention. Topoisomerase inhibitors, such as coumermycin, nalidixic acid, novobiocin and oxolinic acid, inhibitors of cell division, including colcemide, colchicine, vinblastine and vincristine; and RNA synthesis inhibitors including actinomycin D, $\alpha$-amanitine and other fungal amatoxins, cordycepin (3′-deoxyadenosine), dichlororibofuranosyl benzimidazole, rifampicine, streptovaricin and streptolydigin also can be combined with the compounds of the invention to provide pharmaceutical compositions. In some embodhnents, immunostimulatory agents are coadministered with the

NRBAs or SERMs of this invention.

In addition, the compounds of the present invention can be used, either singly or in combination as described above, in combination with other modalities for preventing or treating conditions, diseases or disorders, as described herein. In some embodiments, such other treatment modalities may include without limitation, surgery, radiation, hormone supplementation, diet regulation, wound debridement, etc., as will be appropriate for the condition being treated. These can be performed sequentially (e.g., treatment with a compound of the invention following surgery or radiation) or in combination (e.g., in addition to a diet regimen).

In another embodiment, the present invention includes compounds and compositions in which a compound of the invention is either combined with, or covalently bound to, a cytotoxic agent bound to a targeting agent, such as a monoclonal antibody (e.g., a murine or humanized monoclonal antibody). It will be appreciated that the latter combination may allow the introduction of cytotoxic agents into cancer cells with greater specificity. Thus, the active form of the cytotoxic agent (i.e., the free form) will be present only in cells targeted by the antibody. Of course, the compounds of the invention may also be combined with monoclonal antibodies that have therapeutic activity against cancer.

The additional active agents may generally be employed in therapeutic amounts as indicated in the PHYSICIANS' DESK REFERENCE (PDR) 53rd Edition (1999), or such therapeutically useful amounts as would be known to one of ordinary skill in the art. The compounds of the invention and the other therapeutically active agents can be administered at the recommended maximum clinical dosage or at lower doses. Dosage levels of the active compounds in the compositions of the invention may be varied to obtain a desired therapeutic response depending on the route of administration, severity of the disease and the response of the patient. The combination can be administered as separate compositions or as a single dosage form containing both agents. When administered as a combination, the therapeutic agents can be formulated as separate compositions that are given at the same time or different times, or the therapeutic agents can be given as a single composition.

In some embodiments, the pharmaceutical compositions of this invention will comprise a NRBA or SERM compound of formula (I)-(X) or its prodrug, analog, isomer, ester, metabolite, derivative, pharmaceutically acceptable salt, pharmaceutical product, polymorph, crystal, impurity, *N*-oxide, hydrate or any combination thereof.

The pharmaceutical composition can comprise the NRBA or SERM compound alone or can further include a pharmaceutically acceptable carrier and can be in solid or liquid form such as tablets, powders, capsules, pellets, solutions, suspensions, elixirs, emulsions, gels, creams, or suppositories, including rectal and urethral suppositories. Pharmaceutically acceptable carriers include gums, starches, sugars, cellulosic materials, and mixtures thereof. The pharmaceutical preparation containing the NRBA or SERM compound can be administered to a subject by, for example, subcutaneous implantation of a pellet; in a further embodiment, the pellet provides for controlled release of NRBA or SERM compound over a period of time. The preparation can also be administered by intravenous, intraarterial, or intramuscular injection of a liquid preparation, oral administration of a liquid or solid preparation, or by topical application. Administration can also be accomplished by use of a rectal suppository or a urethral suppository. The pharmaceutical composition can also be a parenteral formulation; in one embodiment, the formulation comprises a liposome that includes a complex of a NRBA or SERM compound.

The pharmaceutical composition of the invention can be prepared by known dissolving, mixing, granulating, or tablet-forming processes. For oral administration, the NRBA or SERM compound or their physiologically tolerated derivatives such as salts, esters, *N*-oxides, and the like are mixed with additives customary for this purpose, such as vehicles, stabilizers, or inert diluents, and converted by customary methods into a suitable form for administration, such as tablets, coated tablets, hard or soft gelatin capsules, aqueous, alcoholic or oily solutions. Examples of suitable inert vehicles are conventional tablet bases such as lactose, sucrose, or cornstarch in combination with binders like acacia, cornstarch, gelatin, or with disintegrating agents such as cornstarch, potato starch, alginic acid, or with a lubricant such as stearic acid or magnesium stearate. Examples of suitable oily vehicles or solvents are vegetable or animal oils such as sunflower oil or fish-liver oil. Preparations can be effected both as dry and as wet granules. For parenteral administration (subcutaneous, intravenous, intraarterial, or intramuscular injection), the chemopreventive agents or their physiologically tolerated derivatives such as salts, esters, *N*-oxides, and the like are converted into a solution, suspension, or emulsion, if desired with the substances customary and suitable for this purpose, for example, solubilizers or other auxiliaries. Examples are: sterile liquids such as water and oils, with or without the addition of a surfactant and other pharmaceutically acceptable adjuvants. Illustrative oils are those of petroleum, animal, vegetable, or synthetic origin, for example, peanut oil, soybean oil, or mineral oil. In general, water, saline, aqueous dextrose and related sugar solutions, and glycols such as propylene glycols or polyethylene glycol are preferred liquid carriers, particularly for injectable solutions.

The preparation of pharmaceutical compositions which contain an active component is well understood in the art. Typically, such compositions are prepared as an aerosol of the polypeptide delivered to the nasopharynx or as injectables, either as liquid solutions or suspensions, however, solid forms suitable for solution in, or suspension in, liquid prior to injection can also be prepared. The preparation can also be emulsified. The active therapeutic ingredient is often mixed with excipients which are pharmaceutically acceptable and compatible with the active ingredient. Suitable excipients are, for example, water, saline, dextrose, glycerol, ethanol, or the like and combinations thereof. In addition,

if desired, the composition can contain minor amounts of auxiliary substances such as wetting or emulsifying agents, or pH buffering agents which enhance the effectiveness of the active ingredient.

An active component can be formulated into the composition as neutralized pharmaceutically acceptable salt forms. Pharmaceutically acceptable salts include the acid addition salts (formed with the free amino groups of the polypeptide or antibody molecule) and are formed with inorganic acids such as, for example, hydrochloric or phosphoric acids, or such organic acids as acetic, oxalic, tartaric, mandelic, and the like. Salts formed from the free carboxyl groups can also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

For topical administration to body surfaces using, for example, creams, gels, drops, and the like, the NRBA or SERM compound or their physiologically tolerated derivatives such as salts, esters, *N*-oxides, and the like are prepared and applied as solutions, suspensions, or emulsions in a physiologically acceptable diluent with or without a pharmaceutical carrier.

In another embodiment, the active compound can be delivered in a vesicle, in particular a liposome (see Langer, Science 249:1527-1533 (1990); Treat et al., in Liposomes in the Therapy of Infectious Disease and Cancer, Lopez-Berestein and Fidler (eds.), Liss, New York, pp. 353-365 (1989); Lopez-Berestein, ibid., pp. 317-327; see generally ibid).

This invention also provides in one embodiment, methods of use of the compositions mentioned hereinabove.

In one embodiment, this invention provides methods of treatment using a NRBA, NRBAs, SERM or SERMs or composition/s of this invention. In one embodiment, the terms "treating" or "treatment", includes preventative as well as disorder remitative treatment. The terms "reducing,", "suppressing" and "inhibiting" have their commonly understood meaning of lessening or decreasing, in another embodiment, or delaying, in another embodiment, or reducing, in another embodiment the incidence, severity or pathogenesis of a disease, disorder or condition. In embodiment, the term treatment refers to delayed progression of, prolonged remission of, reduced incidence of, or amelioration of symptoms associated with the disease, disorder or condition. In one embodiment, the terms "treating" "reducing", "suppressing" or "inhibiting" refer to a reduction in morbidity, mortality, or a combination thereof, in association with the indicated disease, disorder or condition. In one embodiment, the term "progression" refers to an increasing in scope or severity, advancing, growing or becoming worse. The term "recurrence" means, in another embodiment, the return of a disease after a remission. In one embodiment, the methods of treatment of the invention reduce the severity of the disease, or in another embodiment, symptoms associated with the disease, or in another embodiment, reduces the number of biomarkers expressed during disease.

In one embodiment, the term "treating" and its included aspects, refers to the administration to a subject with the indicated disease, disorder or condition, or in some embodiments, to a subject predisposed to the indicated disease, disorder or condition. The term "predisposed to" is to be considered to refer to, *inter alia,* a genetic profile or familial relationship which is associated with a trend or statistical increase in incidence, severity, etc. of the indicated disease. In some embodiments, the term term "predisposed to" is to be considered to refer to *inter alia,* a lifestyle which is associated with increased risk of the indicated disease. In some embodiments, the term "predisposed to" is to be considered to refer to *inter alia,* the presence of biomarkers which are associated with the indicated disease, for example, in cancer, the term "predisposed to" the cancer may comprise the presence of precancerous precursors for the indicated cancer,

The term "administering", in another embodiment, refers to bringing a subject in contact with a compound of the present invention. Administration can be accomplished *in vitro,* i.e. in a test tube, or *in vivo,* i.e. in cells or tissues of living organisms, for example humans. In one embodiment, the present invention encompasses administering the compounds of the present invention to a subject.

***SOME EMBODIMENTS OF USES OF THE NRBAs or SERMs OF THIS INVENTION:***

***Prostate Carcinogenesis:***

Prostate cancer is one of the most frequently occurring cancers among men in the United States, with hundreds of thousands of new cases diagnosed each year. As a result prostate cancer is the second leading cause of cancer deaths. Unfortunately, over sixty percent of newly diagnosed cases of prostate cancer are found to be pathologically advanced, with no cure and a dismal prognosis. One approach to this problem is to detect prostate cancer earlier through screening programs and thereby reduce the number of advanced prostate cancer patients. Another strategy, however, is to develop drugs to prevent the development of prostate cancer. One third of all men over 50 years of age have a latent form of prostate cancer that may be activated into the life-threatening clinical prostate cancer form. The frequency of latent prostatic tumors has been shown to increase substantially with each decade of life from the 50s (5.3-14%) to the 90s (40-80%). The number of people with latent prostate cancer is the same across all cultures, ethnic groups, and races, yet the frequency of clinically aggressive cancer is markedly different. This suggests that environmental factors

may play a role in activating latent prostate cancer. Thus, the development of chemoprevention strategies against prostate cancer may have the greatest overall impact both medically and economically in the fight against prostate cancer.

Further, as prostate intraepithelial neoplasia is in the direct causal pathway to prostate cancer and its presence specifically portends an increased risk of prostate cancer, men diagnosed with prostate intraepithelial neoplasia have dramatic changes in their quality of life. The only way to diagnose prostate intraepithelial neoplasia is by prostate biopsy. Once the diagnosis of prostate intraepithelial neoplasia is made, however, the standard of medical care is that the patient must be subjected to more frequent biopsies and physician visits. In addition, there is great patient and physician anxiety because the diagnosis of prostate cancer is imminent. Currently, there is no treatment available for patients who have prostate intraepithelial neoplasia.

This invention relates to the prevention and treatment of hyperplasia. In some embodiments, this invention is directed to a method of a) treating, preventing, suppressing, inhibiting or reducing the incidence of benign prostate hyperplasia (BPH) in a male subject; and b) treating a subject suffering from hair loss, comprising administering a therapeutically effective amount of a nuclear hormone binding agent (NRBA) of this invention, which in some embodiments, is a selective estrogen receptor modulator (SERM) compound and/or its analog, derivative, isomer, metabolite, pharmaceutically acceptable salt, pharmaceutical product, hydrate, *N*-oxide, prodrug, polymorph, crystal, or any combination thereof, as described herein.

**[0122]** [00117] In one embodiment, this invention provides a method of treating, preventing, suppressing, preventing the recurrence of, reducing the incidence of, reducing the severity of, reducing or preventing metastasis of prostate cancer, BPH or precancerous precursors of prostate cancer, via the administration of a NRBA/SERM or NRBAs/SERMs of this invention, or a composition comprising the same.

The NRBAs/SERMs and pharmaceutical compositions comprising the same of the present invention are particularly useful for treating a subject having an elevated risk of developing prostate cancer. High-risk subjects Include, for example, those having benign prostatic hyperplasia, prostatic intraepithelial neoplasia (PIN), an abnormally high level of circulating prostate specific antibody (PSA), or who have a family history of prostate cancer.

Intermediate endpoint biomarkers are measurable biologic alterations in tissue that occur between the initiation of and the development of frank neoplasia. A biomarker is validated if the final endpoint, cancer incidence, is also reduced by the putative chemopreventive agent. Intermediate biomarkers in cancer may be classified into the following groups: histologic, proliferation, differentiation and biochemical markers. In any chemoprevention strategy, the availability of histologically recognizable and accepted precancerous lesions constitutes an important starting point. For the prostate, a histological marker is a precancerous precursor of prostatic adenocarcinoma of which prostatic intraepithelial neoplasia (PIN) is an example. PIN appears as an abnormal proliferation within the prostatic ducts of premalignant foci of cellular dysplasia and carcinoma *in situ* without stromal invasion. PIN and histological prostate cancer are morphometrically and phenotypically similar. Thus, the development of high-grade PIN represents an important step in the progression pathway whereby the normal prostate develops PIN, histological prostate cancer, invasive clinical prostate cancer, and metastases.

Prostate intraepithelial neoplasia has been shown to be a precancerous lesion, or precursor of prostatic adenocarcinoma. Prostate intraepithelial neoplasia is the abnormal proliferation within the prostatic ducts of premalignant foci of cellular dysplasia and carcinoma, *in situ* without stromal invasion. Prostate intraepithelial neoplasia is the most accurate and reliable marker of prostate carcinogenesis and may be used as an acceptable endpoint in prostate chemoprevention trials. Prostate intraepithelial neoplasia has a high predictive value as a marker for adenocarcinoma, and its identification warrants repeat biopsy for concurrent or subsequent invasive carcinoma. Most studies suggest that most patients with prostate intraepithelial neoplasia will develop carcinoma within 10 years. Interestingly, prostate intraepithelial neoplasia does not contribute to serum PSA, which is not surprising, since unlike prostate cancer, prostate intraepithelial neoplasia has not yet invaded the vasculature of the prostate to leak PSA into the blood steam Thus, prostate intraepithelial neoplasia may precede even prostate cancer related serum PSA elevations.

In some embodiments, any NRBA, which may be characterized by the structure of a formula presented herein may be used for treating, preventing, suppressing, reducing incidence of, preventing latency of, suppressing or prolonging latency of prostate cancer, benign prostate cancer or precancerous precursors of the same, and is to be considered as part of this invention. In one embodiment, NRBAs/SERMs useful for effecting all aspects of prostate carcinogenesis based their activity in receptor binding studies, ER transactivation studies, *in vitro* studies of their effects on prostate cancer cell growth, and in *vivo* studies.

In some embodiments, NRBAs/SERMs are useful in treating, reducing, preventing side effects associated with treatments for prostate cancer. In some embodiments, such SERMs include, based their activity in receptor binding studies, ER transactivation studies, *in vitro* studies of their effects on prostate cancer cell growth, and *in vivo* studies.

In some embodiments ER $\alpha$ antagonists and ER $\beta$ agonist are useful in treating, reducing, preventing side effects associated with treatments for prostate cancer and benign prostatic hyperplasia. In another embodiment ER $\alpha$ antagonists of this invention include *inter alia* 10m, 4a, 11f or 11g. In another embodiment ER $\beta$ agonist of this invention include *inter alia* 10d, 10f, 101 or 11p, listed in Table 1 or any combination thereof.

Further, the NRBAs/SERMs may be administered in combination with other cytokines or growth factors that include but are not limited to: IFN-γ, IFN-α or IFN-β; interleukin (IL) 1, IL-2, IL-4, IL-6, IL-7, IL-12, tumor necrosis factor (TNF) α, TNF-β, granulocyte colony stimulating factor (G-CSF), granulocyte/macrophage CSF (GMCSF); accessory molecules, including members of the integrin superfamily and members of the Ig superfamily such as, but not limited to, LFA-1, LFA-3, CD22, and B7-1, B7-2, and ICAM-1 T cell costimulatory molecules.

In another embodiment, the NRBA/SERM may be administered in combination with other compounds which, in one embodiment, inhibit prostate carcinogenesis, such as 5 alpha-reductase inhibitors, or inhibitors of other enzymes involved in the androgen biosynthetic pathway, such as, for example, 3α-hydroxysteroid dehydrogenase, 17-ketoreductase, 17β-hydroxysteroid dehydrogenase, 17β-aldoketoreductase, 3β-DHΔ4,6-isomerase, 3β-DHΔ4,5-isomerase, 17,20 desmolase, p450c17, p450ssc, 17,20-lyase, and others, for affecting prostate intraepithelial neoplasia (PIN), prostate carcinogenesis, conditions related to excess androgen production, such as polycystic ovarian disease, infertility, hot flashes, gynecomastia, for example when such conditions are in subjects undergoing androgen-deprivation therapy, or other hormone-related conditions, including diabetes, some of which are further described herein below.

The chemopreventive agent may precede or follow a DNA damaging agent treatment by intervals ranging from minutes to weeks. Protocols and methods are known to those skilled in the art DNA damaging agents or factors are known to those skilled in the art and refer to any chemical compound or treatment method that induces DNA damage when applied to a cell. Such agents and factors include radiation and waves that induce DNA damage, such as gamma-irradiation, X-rays, UV-irradiation, microwaves, electronic emissions, and the like. A variety of chemical compounds, also described as "chemotherapeutic agents", function to induce DNA damage, all of which are intended to be of use in the combined treatment methods disclosed herein. Chemotherapeutic agents contemplated to be of use include, e.g., adriamycin, 5-fluorouracil (5FU), etoposide (VP-16), camptothecin, actinomycin-D, mitomycin C, cisplatin (CDDP) and even hydrogen peroxide. The invention also encompasses the use of a combination of one or more DNA damaging agents, whether radiation-based or actual compounds, such as the use of X-rays with cisplatin or the use of cisplatin with etoposide.

In another embodiment one may irradiate the localized tumor site with DNA damaging radiation such as X-rays, UV-light, gamma -rays or even microwaves. Alternatively, the tumor cells may be contacted with the DNA damaging agent by administering to the subject a therapeutically effective amount of a pharmaceutical composition comprising a DNA damaging compound, such as adriamycin, 5-fluorouracil, etoposide, camptothecin, actinomycin-D, mitomycin C, or more preferably, cisplatin. Agents that damage DNA also include compounds that interfere with DNA replication, mitosis and chromosomal segregation. Such chemotherapeutic compounds include adriamycin, also known as doxorubicin, etoposide, verapamil, podophyllotoxin, and the like.

Other factors that cause DNA damage and have been used extensively include what are commonly known as gamma-rays, X-rays, and/or the directed delivery of radioisotopes to tumor cells. Other forms of DNA damaging factors are also contemplated, such as microwaves and UV-irradiation. It is most likely that all of these factors Effect a broad range of damage to DNA, on the precursors of DNA, the replication and repair of DNA, and the assembly and maintenance of chromosomes.

In another embodiment, the invention provides for the use of the NRBAs/SERMs, or compositions comprising the same for treating, suppressing, inhibiting precancerous precursors of prostate adenocarcinoma lesions in a mammalian subject.

In another embodiment, the invention provides for the use of the NRBAs/SERMs, or compositions comprising the same for treating, preventing, suppressing, inhibiting, or reducing the incidence of osteoporosis, hot flashes, gynecomastia, and/or hair loss in male human subjects having prostate cancer. In some embodiments, the osteoporosis, hot flashes, gynecomastia, and/or hair loss in male human subjects with prostate cancer arise as a consequence of androgen deprivation therapy (ADT) initiated in the subject. According to this aspect, and in one embodiment, the NRBA/SERM is useful in treating these ADT-induced conditions, while concurrently not aggravating prostate carcinogenesis in the subjects. In another embodiment, according to this aspect of the invention, the MRBAs/SERMs/compositions of this invention both treat, suppress, inhibit, etc., prostate carcinogenesis and concurrently treat ADT-induced conditions in these subjects.

In another embodiment, the invention provides a method of treating, suppressing, inhibiting or reducing the risk of developing prostate cancer in a subject with prostate cancer comprising administering a pharmaceutical composition comprising a SERM compound of formula (I)-(X), to said subject.

In another embodiment, the invention provides a method of suppressing or inhibiting prostate cancer in a mammalian subject comprising administering a pharmaceutical composition comprising a SERM compound of formula (I)-(X) or its prodrug, ester, analog, isomer, metabolite, derivative, pharmaceutically acceptable salt, pharmaceutical product, polymorph, crystal, impurity, *N*-oxide, hydrate or any combination thereof, to said subject.

In another embodiment, the invention provides a method of reducing the risk of developing prostate cancer in a mammalian subject comprising administering a pharmaceutical composition comprising a SERM/NRBA compound of formula (I)-(X) or its prodrug, analog, isomer, metabolite, derivative, pharmaceutically acceptable salt, pharmaceutical

product, ester, polymorph, crystal, impurity, *N*-oxide, hydrate or any combination thereof, thereby reducing the risk of developing prostate cancer in said subject.

In another embodiment, the invention provides a method of treating precancerous precursors of prostate adenocarcinoma lesions in a mammalian subject comprising administering a pharmaceutical composition comprising SERM/NRBA compound of formula (I)-(X) or its prodrug, ester, analog, isomer, metabolite, derivative, pharmaceutically acceptable salt, pharmaceutical product, polymorph, crystal, impurity, *N*-oxide, hydrate or any combination thereof, thereby treating precancerous precursors of prostate adenocarcinoma lesions in said to the subject. In another embodiment, the precancerous precursor of prostate adenocarcinoma is prostate intraepithelial neoplasia

In one embodiment, according to these aspects of the invention, the methods are appropriate for treating, suppressing, inhibiting, reducing the risk of developing, etc., latent prostate cancer.

*Colon Cancer*

Colon cancer is the second most frequently diagnosed malignancy in the United States, as well as the second most common cause of cancer death. Cholesterolrich diets have had a significant epidemiological association with cancers of the colon, which in turn may be influenced by the administration of compounds which modulate nuclear hormone binding agents, in particular, compounds which modulate receptors binding components of the steroidogenic pathway, in particular SERM compounds, as described herein.

In one embodiment, the invention provides a method of treating, preventing, inhibiting or reducing the incidence of colon cancer conditions in a subject, comprising administering a pharmaceutical composition comprising a NRBA compound of formula (I)-(X), which in some embodiments is a SERM, or its prodrug, analog, isomer, metabolite, derivative, pharmaceutically acceptable salt, pharmaceutical product, polymorph, crystal, impurity, *N*-oxide, ester, hydrate or any combination thereof to the subject.

In some embodiments ER-β agonists are useful in treating, preventing, inhibiting reducing the incidence of inflammatory conditions in a subject. In another embodiment ER- β agonist of this invention include *inter alia* 10d, 10f, 101, or 11p, listed in Table 1 or any combination thereof

In some embodiments, treatment may comprise administration of other agents, which treat colon cancer, such as, for example, azetidinone-based cholesterol absorption inhibitors, or others, as known to those skilled in the art. In some embodiments, such treatment may precede, or follow that of the NRBA compounds of this invention, or be concurrent therewith.

*ADT-induced* ***conditions:***

In one embodiment, this invention provides methods of 1) improving the lipid profile of a subject; 2) reducing the circulating lipid levels in a subject; 3) increasing high density lipoprotein (HDL) cholesterol levels in a subject; 4) altering ratios of low density lipoprotein to high density lipoprotein levels in a subject; wherein said subject has prostate cancer and is undergoing or has undergone ADT, wherein said method comprises administering to said subject a composition comprising a SERM compound or its pharmaceutically acceptable salt, hydrate, N-oxide, or any combination thereof; In another embodiment, the SERM, compound is of formula (I -XI) or its prodrug, ester, analog, isomer, metabolite, derivative, pharmaceutically acceptable salt, pharmaceutical product, polymorph, crystal, impurity, *N*-oxide, hydrate or any combination thereof.

In another embodiment, the subject is undergoing or has undergone ADT. The terms "has undergone," "undergoing ," and the like refer, in one embodiment, to subjects that have recently (within the last 6 months) or are currently receiving any treatment or therapy known in the art that reduces androgen levels in general or testosterone levels in particular. In another embodiment, the terms refer to a subject that received such a treatment or therapy more than 6 months previously. In one embodiment, the treatment or therapy is surgical. In another embodiment, the treatment or therapy is medical. In another embodiment, the treatment or therapy eliminates an androgen or a testosterone entirely, or below detectable levels. In another embodiment, the ADT is a side effect of a treatment or therapy not intended to reduce androgen or testosterone levels. Each of these possibilities represents a separate embodiment of the present invention.

In another embodiment, ADT is used for treating prostate cancer, for delaying the progression of prostate cancer, and for preventing and/or treating the recurrence of prostate cancer, which comprise administering LHRH analogs, reversible antiandrogens (such as bicalutamide or flutamide), anti-estrogens, anticancer drugs, 5-alpha reductase inhibitors, aromatase inhibitors, progestins, selective androgen receptor modulators (SARMS) or agents acting through other nuclear hormone receptors. In another embodiment, ADT is administered monthly, or every 3, 4, 6 or 12 month. In another embodiment, ADT is administered every two weeks in the first month, then every four weeks.

In some embodiments, this invention provides methods comprising administering a SERM compound to a subject that has prostate cancer and is undergoing or has undergone ADT. In one embodiment, the SERM compound

of the present invention can be administered prior to the ADT. In another embodiment, the SERM compound of the present invention can be administered with the ADT. In another embodiment, the SERM compound of the present invention can be administered after the ADT.

In some embodiments, the methods of this invention comprise administering the SERM compound of this invention in combination with the ADT, prior to the ADT or after the ADT as a preventive for all diseases in this invention. In one embodiment the SERM is administered between 1-2 weeks before ADT. In another embodiment the SERM is administered between 2-4 weeks prior to ADT. In another embodiment the SERM is administered between 1-2 months before ADT. In another embodiment the SERM is administered between 2-4 months before ADT. In another embodiment the SERM is administered between 4-6 months before ADT. In one embodiment the SERM is administered between 1-2 weeks after ADT. In another embodiment the SERM is administered between 2-4 weeks after ADT. In another embodiment the SERM is administered between 1-2 months after ADT. In another embodiment the SERM is administered between 2-4 months after ADT. In another embodiment the SERM is administered between 4-6 months after ADT.

In other embodiments, the present invention provides a method of treating any disease, disorder, or symptom associated with ADT. In other embodiments, the present invention provides a method of treating any disease, disorder, or symptom associated with androgen deprivation. In other embodiments, the present invention provides a method of treating any disease, disorder, or symptom associated with testosterone deprivation. Each disease, disorder, or symptom represents a separate embodiment of the present invention.

*Immune System Disorders:*

Cross-talk has been shown to occur between endocrine-disrupting chemicals and cytokine signaling through estrogen receptors, suggesting a role for SERMs and/or other nuclear hormone binding agents in the modulation of the immune system and/or diseases thereof.

For example, tamoxifen, clomiphene and nafoxidine cause a decrease in viability of the estrogen receptor-negative T-lymphoblastic leukemia cell line CCRF/CEM, suggesting a role for antiestrogens in the clinical treatment of leukemia.

Leukemia is a malignant cancer of the bone marrow and blood and comprises acute or chronic myelogenous, or acute or chronic lymphocytic type disease.

Standard treatment for leukemia usually involves chemotherapy and/or bone marrow transplantation and/or radiation therapy. Chemotherapy usually involves a combination of two or more anti-cancer drugs, with common combinations including cytarabine with either doxorubicin or daunorubicin or mitoxantrone or thioguanine, mercaptopurine with methotrexate, mitroxantrone with etoposide, asparaginase with vincristine, daunorubicin and prednisone, cyclophosphamide with vincristine, cytarabine and prednisone, cyclophosphamide with vincristine and prednisone, daunorubicin with cytarabine and thioguanine and daunorubicin with vincristine and prednisone.

In one embodiment, the invention provides a method of treating, preventing, inhibiting or reducing the incidence of leukemia in a subject, comprising administering a pharmaceutical composition comprising a SERM compound of formula (I)-(X) or its prodrug, analog, isomer, metabolite, derivative, pharmaceutically acceptable salt, pharmaceutical product, polymorph, crystal, impurity, *N*-oxide, ester, hydrate or any combination thereof, to the subject.

In some embodiments ER-β agonists are useful in treating, preventing, inhibiting or reducing the incidence of leukemia in a subject. In another embodiment ER-β agonist of this invention include *inter alia* 10d, 10f, 101, or 11p, listed in Table 1 or any combination thereof.

In another embodiment ER α antagonists are useful in treating, preventing, inhibiting or reducing the incidence of leukemia in a subject. In some embodiments, the ER α antagonists of this invention include *inter* 10m, 4a, 11f, or 11g.

In some embodiments the NRBA/SERMs of this invention, which in one embodiment are ER-β agonists, are useful in treating, preventing, inhibiting or reducing the incidence of inflammatory diseases, disorders or conditions in a subject. In another embodiment ER- β agonists of this invention include, *inter alia, alia* 10d, 10f, 101, or 11p, listed in Table 1 or any combination thereof.

*Neurological Diseases, **Disorders** or Conditions:*

Estrogens may be protective against brain injury, neurodegeneration, and cognitive decline. Estrogens may also be protective of cortical and hippocampal neurons in ischemic injury and damage due to seizures, and in some embodiments, estrogen receptor (ER) α agonists may play a more dominant role in mediating neuroprotection.

In one embodiment, the invention provides a method of treating, preventing, inhibiting reducing the incidence of neurological diseases, disorders or conditions in a subject, comprising administering a pharmaceutical composition comprising a NRBA or SERM compound of formula (I)-(X) or its prodrug, analog, isomer, metabolite, derivative, pharmaceutically acceptable salt, pharmaceutical product, polymorph, crystal, impurity, *N*-oxide, ester, hydrate or any combination thereof to the subject.

In some embodiments, the term "neurological" is to be understood as including of or belonging to the central nervous system.

In some embodiments both ER-β and ER-α agonists of this invention are useful in treating, preventing, inhibiting reducing the incidence of inflammatory conditions in a subject. In one embodiment an ER-β agonist of this invention useful in this context includes, *inter alia, alia* 10d, 10f, 101, or 11p, listed in Table 1 or any combination thereof. In another embodiment an ER-α agonist of this invention useful in this context includes *inter alia* 3v, 3b, 3k, or 10x, listed in Table 1, or any combination thereof.

In some embodiments, the neurological disease disorder or condition comprises a demyelinating disease, such as multiple sclerosis, idiopathic demyelinating polyneuropathy, chronic inflammatory demyelinating polyneuropathy or Guillain-Barre syndrome. In some embodiments, the neurological disease disorder or condition comprises ischemia resulting from the narrowing of a blood vessel or vessels supplying the brain, infection of the central nervous system, such as, for example, in meningitis, or encephalomyelitis, amyotrophic lateral sclerosis (ALS), Alzheimer's disease, pain, Huntington's disease (HD), myasthenia gravis (MG), fronto-temporal dementia (FTD), stroke, traumatic brain injury, age-related retinal degeneration, mood disorders or depression, anoxic/hypoxic damage (e.g., cardiopulmonary resuscitation, drowning), spinal cord injury, local anesthetic-induced seizure activity, ischemic stroke, ischemic neurodegeneration of the retina, epilepticus, Tourette's syndrome, obsessive-compulsive disorder, drug-induced (e.g., nerve gas-induced) CNS injury, chronic pain syndromes, acute and chronic neurodegenerative disorders (e.g., lateral sclerosis, Alzheimer's disease, AIDS dementia syndrome, cocaine addiction, or neurodegenerative disorders such as hypoglycemia, cerebral palsy, transient cerebral ischemic attack, perinatal asphyxia, psychosis, Parkinson's disease, Olivopontocerebellar atrophy, and pathogen-induced, for example viral-induced neurodegeneration such as in acquired immunodeficiency syndrome and its associated dementia.

It is to be understood that any neurological disease, disorder or condition, which can be treated, or ameliorated with the administration of a compound or composition of this invention is to be considered part of this invention.

*Ocular Disorders*

The compounds of this invention may be useful for the treatment or amelioration of conditions affecting the neural retina. Estrogen may have neuroprotective effects in the retina (see for example Invest Ophthal Vis Sci 38: 1193-1202 (1997) and Invest Ophthal Vis Sci 44(7):3155-3162 (2003)), and estrogen receptors are found in the inner retina as well as the choroid (Br J Ophthalmol 85:877-882 (2001). The NRBAs of the present invention may be useful in treating the eye for, or protecting against local ischemia or degenerative events that include, but are not limited to, macular degeneration, glaucoma, diabetic retinopathy, retinitis pigmentosa and other retinal degeneration resulting from genetic defects, trauma or environmental exposure.

In one embodiment, this invention provides a method of treating eye diseases selected from glaucoma, high tension glaucoma, normal tension glaucoma, central chorioretinopathy, senile macular degeneration, macular hole, cataract, senile cataract, chorioretinal hemorrhage, central retinal artery or vein occlusion, arteriosclerosis of retinal artery, photopsia, diabetic retinopathy, chorioretinal atrophy, retinal and choroidal neovascular diseases, cataract due to removal of ovary, cataract due to TGFβ, macular fibrosis, macular epiretinal membrane, retinal tear, retinal detachment, retinitis proliferans, pigmentary retinal degeneration, keratitis, corneal opacity, corneal erosion, detachment of corneal epithelium, corneal ulcer, corneal endothelial cell degeneration and dystrophy or loss of endothelial cell, corneal dystrophy or degeneration, epidemic keratoconjunctivitis, chalazion, iritis, uveitis, autoimmune disease, chorioretinitis, iridocyclitis, asthenopia, narrowing of visual field due to various kinds of diseases, optic nerve atrophy, optic neuritis, anterior ischemic optic neuropathy, lowering in dynamic visual activity, abnormal color vision, refractive error, presbyopia, myopia, hyperopia, astigmatism, central nerve diseases, psychosis, hysteria, diseases due to cerebral pituitary gland disorder and imbalance of hormones, diseases due to gene disorder and diseases due to immune disorder, the method comprising administering a pharmaceutical composition comprising a NRBA or SERM compound of formula (I)-(X) or its prodrug, analog, isomer, metabolite, derivative, pharmaceutically acceptable salt, pharmaceutical product, polymorph, crystal, impurity, *N*-oxide, ester, hydrate or any combination thereof to the subject

In another embodiment, the methods of treating eye diseases comprise administering a composition comprising the compounds of this invention to the subject, wherein the composition is in the form of eye drops, eye wash, ointments, conjunctival injections, or contact lens adsorbents. In another embodiment, the methods of treating eye diseases comprises administering a composition comprising the compounds of this invention in the form of a tablet, capsule, liquid, syrup, injection, hap, ointment, eye drops, suppository, and the like, and administered orally, or non-orally such as injection, locally such as dropping to eye, etc. The effective ingredient may be vaporized and inhaled, for example through the nose, mouth or trachea.

In some embodiment, the methods of treating eye diseases comprise administering a composition comprising the compounds of this invention and any other compound, which is useful in treating the indicated conditions, as known in the art.

In some embodiment, eye drops and eye wash comprise water-solubilized compounds (I) — (X) of this invention, which are, in one embodiment, dissolved in sterilized distilled water, BSS Plus, and/or physiological saline. In another embodiment, additives are added comprising excipients, carriers, pH controllers, isotonic agents, preservatives, glutathione, glucose, various kind of salt(s), stabilizers, refrigerants, antioxidants, antiseptic agents, or any combination thereof In another embodiment, the eye drops and eye wash comprise hydroxypropylmethyl cellulose, carboxymethyl cellulose or its sodium salt, polypyrrolidone, polyvinylpyrrolidone (this is added and heated), or any combination thereof.
In some embodiments, the compounds of this invention have low solubility in water. In one embodiment, the compounds may be water solubilized by using cyclodextrin. In another embodiment a —cyclodextrin is used. In another embodiment β cyclodextrin is used. In another embodiment, γ cyclodextrin is used. In another embodiment, hydroxyalkylated β cyclodextrin is used.

*Bladder Cancer*

Bladder cancer is the second most common genitourinary tumor in human populations. SERMs have been shown to induce apoptosis in bladder cancer cell lines, expressing estrogen receptors.
In some embodiments this invention provides a method of treating, suppressing, reducing the incidence or severity of, or prolonging remission of bladder cancer in a subject, the method comprising administering a pharmaceutical composition comprising a NRBA or SERM compound of formula (I)-(X) or its prodrug, analog, isomer, metabolite, derivative, pharmaceutically acceptable salt, pharmaceutical product, polymorph, crystal, impurity, *N*-oxide, ester, hydrate or any combination thereof to the subject.
In some embodiments, according to this aspect of the invention, the methods comprise administering an ER-β ligand, such as, but not limited to, compounds 10d, 10f, 101, or 11p, listed in Table 1 or any combination thereof.
Existing thereapies for bladder cancer may be combined with the therapies provided herein, including, cystectomy with or without administration of methotrexate, vinbiastine, doxorubicin, or cisplatin (M-VAC), or others as known in the art.

*Bone-related diseases or disorders*

In one embodiment, the invention provides a method of treating, preventing or reducing the severity of a bone-related disease or disorder in a subject, comprising administering a NRBA, which in some embodiments is a SERM, of this invention to the subject. In one embodiment, the subject is administered a NRBA/SERM or composition comprising the same, wherein the NRBA/SERM is a compound of formula (I)-(X) or its prodrug, ester, analog, isomer, metabolite, derivative, pharmaceutically acceptable salt, pharmaceutical product, polymorph, crystal, impurity, *N*-oxide, hydrate or any combination thereof.
In one embodiment, the bone-related disease or disorder is osteoporosis, and the methods of the present invention for the prevention and/or treatment of osteoporosis, reduction of the incidence, inhibition of, suppression of, or treatment of osteoporosis, or in another embodiment, androgen-deprivation induced osteoporosis, bone fractures and/or loss of bone mineral density (BMD) in a subject. In one embodiment, the subject is female, and in some embodiments, the bone-related disease or disorder occurs in post-menopausal women. In some embodiments, the subject is female, and the bone-related disease or disorder occurs in women with breast cancer, or other neoplastic conditions. In some embodiments, the subject is female, has a neoplastic condition and the bone-related disease or disorder is a by-product of a therapy to treat the neoplastic condition. According to this aspect, and in some embodiments, the treatment of the bone-related disease or disorder is also suppressive, or participates in treating the neoplastic condition in the subject. In another embodiment, the subject is female, and the bone-related disease or disorder is a result of aging in the subject.
In one embodiment, the subject is male, and in some embodiments, the bone-related disease or disorder occurs as a function of advanced age or audropause in the subject. In some embodiments, the subject is male, and the bone-related disease or disorder occurs in men with prostate cancer, or other neoplastic conditions. In some embodiments, the subject is male, has a neoplastic condition and the bone-related disease or disorder is a by-product of a therapy to treat the neoplastic condition. In some embodiments, according to this aspect, the therapy is androgen deprivation therapy (ADT). In some embodiments, the treatment of the bone-related disease or disorder is also suppressive, or participates in treating the neoplastic condition in the subject.
Bone loss due to glucocorticoid excess is diffuse, affecting both cortical and cancellous bone, with a predilection for the axial skeleton. Spontaneous fractures of the vertebrae or ribs are, therefore, often presenting manifestations of the disorder. A cardinal feature of glucocoricoid-induced osteoporosis is decreased bone formation. In addition, patients receiving long-term glucocorticoid therapy sometimes develop collapse of the femoral head (osteonecrosis).
In some embodiments, the SERMs of this invention are useful in treating glucocorticoid-induced osteoporosis in a subject. In some embodiments, the SERMs are useful in preventing, ameliorating, treating, suppressing, delaying

incidence of, reducing severity of bone loss in a subject, associated with glucocorticoid administration in the subject. In some embodiments, the glucocorticoid is any such compound known in the art, including, *inter-alia,* hydrocortisone, cortisone acetate, prednisolone, prednisone, methylprednisolone, triamcinolone, paramethasone, betamethasone, dexamethasone , fludrocortisone, or any combination thereof.

Osteoporosis is a systemic skeletal disease, characterized by low bone mass and deterioration of bone tissue, with a consequent increase in bone fragility and susceptibility to fracture. In osteoporotic patients, bone strength is abnormal, with a resulting increase in the risk of fracture. Osteoporosis depletes both the calcium and the protein collagen normally found in the bone, resulting in either abnormal bone quality or decreased bone density. Bones that are affected by osteoporosis can fracture with only a minor fall or injury that normally would not cause a bone fracture. The fracture can be either in the form of cracking (as in a hip fracture) or collapsing (as in a compression fracture of the spine). The spine, hips, and wrists are common areas of osteoporosis bone fractures, although fractures can also occur in other skeletal areas.

BMD is a measured calculation of the true mass of bone. The absolute amount of bone as measured by bone mineral density (BMD) generally correlates with bone strength and its ability to bear weight. By measuring BMD, it is possible to predict fracture risk in the same manner that measuring blood pressure can help predict the risk of stroke.

BMD in one embodiment can be measured by known bone-mineral content mapping techniques. Bone density of the hip, spine, wrist, etc., may be measured by a variety of techniques. The preferred method of BMD measurement is dual-energy x-ray densitometry (DXA). BMD of the hip, antero-posterior (AP) spine, lateral spine, and wrist can be measured using this technology. Measurement at any site predicts overall risk of fracture, but information from a specific site is the best predictor of fracture at that site. Quantitative computerized tomography (QCT) is also used to measure BMD of the spine. See for example, "Nuclear Medicine: "Quantitative Procedures". by Wahner H W, Dunn W L, Thorsen H C, et al, published by Toronto Little, Brown & Co., 1983, (see pages 107-132). An article entitled "Assessment of Bone Mineral Part 1" appeared in the Journal of Nuclear Medicine, pp 1134-1141, (1984). Another article entitled "Bone Mineral Density of The Radius" appeared in Vol. 26, No. 11, (1985) Nov. Journal of Nuclear Medicine at pp 13-39. Abstracts on the use of gamma cameras for bone-mineral content measurements are (a) S. Hoory et al, Radiology, Vol. 157(P), p. 87 (1985), and (b) C. R. Wilson et al, Radiology, Vol. 157(P), p. 88 (1985).

The present invention provides a safe and effective method for treating, preventing, suppressing, inhibiting or reducing the risk of developing osteoporosis and/or loss of BMD. In one embodiment, the methods include use in the treatment of male subjects suffering from prostate cancer, having an elevated risk of developing androgen-deprivation induced osteoporosis. In another embodiment, the methods are employed in female subjects, during menopause. In another embodiment, the methods include use in the treatment in female subjects suffering from post menopausal osteoporosis. In another embodiment, the female subject is genetically predisposed to developing osteoporosis. In another embodiment, the female subject developed osteoporosis as a result of taking a medication, for example, Depo-Provera. In one embodiment, the subject is a mammalian subject, or in another embodiment, the subject is a human subject.

In one embodiment, the NRBAs/SERMs and/or compositions presented herein are effective at treating, suppressing or inhibiting osteopenia accompanied by bone loss. "Osteopenia" refers, in one embodiment, to decreased calcification or density of bone. In one embodiment, the term encompasses all skeletal systems in which such a condition is noted.

In another embodiment, the invention provides, a method of reducing the incidence, inhibiting, suppressing, and treating osteoporosis, bone fractures and/or loss of bone mineral density (BMD) in a subject, comprising administering a pharmaceutical composition comprising a NRBA/SERM compound of formula (I)-(X), or its prodrug, ester, analog, isomer, metabolite, derivative, pharmaceutically acceptable salt, pharmaceutical product, polymorph, crystal, impurity, *N*-oxide, hydrate or any combination thereof, thereby reducing the incidence, inhibiting, suppressing, and treating osteoporosis, bone fractures and/or loss of bone mineral density (BMD) in the subject.

In one embodiment, the bone-related diseases or conditions being treated with the NRBAs, such as the SERMs of this invention, are a result of androgen-deprivation therapy, which in one embodiment, is carried out in a male patient with prostate cancer. In another embodiment, the bone-related diseases or conditions being treated with the SERMs of this invention are a result of andropause, in an aging male, or in another embodiment, in a male subject with an endocrine disorder which results in diminished androgen production. In another embodiment, bone-related diseases or conditions being treated with the NRBAs/SERMs of this invention, are a result of menopause, in women. In another embodiment, bone-related diseases or conditions being treated with the SERMs of this invention are a result of an endocrine disorder in women, or in another embodiment, a by-product of hormonal manipulation, contraception, or others.

In one embodiment, the SERM used to treat, prevent, inhibit, etc., bone-related diseases or conditions based on their pharmacologic activity as demonstrated in receptor binding studies, estrogen receptor transactivation, *in vitro* studies of osteoblast and osteoclast activity, and *in vivo* studies.

In some embodiments ER-$\alpha$ ligandss are useful in treating, preventing, inhibiting, bone-related diseases or conditions. In another embodiment ER-$\alpha$ ligandsof this invention include *inter alia* 3v, 3b, 3k, or 10x, listed in Table 1,

or any combination thereof.

***Hormone-Related** Disease*

In some embodiments this invention provides a method of treating, preventing, suppressing, delaying onset or severity of a hormone related disease.

In another embodiment, this invention provides a method for treating and/or preventing, etc., post menopausal, or post-andropausal conditions.

In another embodiment, this invention provides a method for treating and/or preventing, etc., diseases, disorders or conditions arising as a result of hormone manipulation or endocrine disorders, resulting in diminished or abrogated circulating hormone levels, for example, diminished or abrogated estrogen or androgen levels.

Such diseases, disorders or conditions may include hot flashes, night sweats, loss of libido, weight gain, hair loss, hypertension, depression, anxiety, gastrointestinal distress, or any combination thereof.

In some embodiments, such diseases, disorders or conditions arise as a result of menopause, andropause, androgen deprivation therapy, or other therapies for related conditions, for example, in treating breast or prostate cancer, in females or males, respectively.

In some embodiments, the treatment for conditions associated with androgen deprivation therapy may include treating or ameliorating hot flashes, gynecomastia, osteoporosis, decreased lean muscle mass, depression and other mood changes, loss of libido, and erectile dysfunction in these subjects.

In some embodiments, the hormone-dependent disease or condition which may be affected via the methods and/or using the NRBAs, which in some embodiments are SERMs and/or compositions of this invention include sex hormone dependent cancers (e.g., prostate cancer, uterine cancer, breast cancer, pituitary gland tumor and the like), prostatic hypertrophy, endometriosis, hysteromyoma, precocious puberty, dysmenorrhea, amenorrhea, premenstrual syndrome, multilocular ovarian syndrome, polycystic ovarian syndrome, postoperative recurrence of the aforementioned cancers, dwarfism, Alzheimer's disease, climacteric disturbance, indefinite complaints, metastasis of the aforementioned cancers, sex hormone-dependent diseases such as calcium/phosphorus bone metabolism disorder and the like, contraception, infertility, or other diseases and/or conditions which develop, as a consequence of the diseases/conditions/ syndromes listed herein.

In one embodiment, the cancer being treated, prevented, suppressed, etc., is breast cancer. This includes but is not limited to hormone sensitive, hormone refractory, or metastatic breast cancer. In one embodiment, the breast cancer arises in the lining of the milk ducts of the breast (ductal carcinoma), or in the lobules where breast milk is produced (lobular carcinoma). Other forms of breast cancer include Inflammatory Breast Cancer and Recurrent Breast Cancer. Inflammatory breast cancer is a rare, but very serious, aggressive type of breast cancer.

In one embodiment, the term "breast cancer" refers to a condition characterized by anomalous rapid proliferation of abnormal cells in one or both breasts of a subject. The abnormal cells often are referred to as "neoplastic cells," which refers to, in some embodiments, transformed cells that can form a solid tumor. The term "tumor", in some embodiments, refers to an abnormal mass or population of cells (i.e. two or more cells) that result from excessive or abnormal cell division, whether malignant or benign, and pre-cancerous and cancerous cells. Malignant tumors are distinguished from benign growths or tumors in that, in addition to uncontrolled cellular proliferation, they can invade surrounding tissues and can metastasize.

In breast cancer, neoplastic cells may be identified in one or both breasts only and not in another tissue or organ, in one or both breasts and one or more adjacent tissues or organs (e.g. lymph node), or in a breast and one or more non-adjacent tissues or organs to which the breast cancer cells have metastasized.

The term "invasion", in some embodiments, refers to the spread of cancerous cells to adjacent surrounding tissues. The term "metastasis", in some embodiments, refers to a process in which cancer cells travel from one organ or tissue to another non-adjacent organ or tissue. Cancer cells in the breast(s) can spread to tissues and organs of a subject, and conversely, cancer cells from other organs or tissue can invade or metastasize to a breast. Cancerous cells from the breast(s) may invade or metastasize to any other organ or tissue of the body. Breast cancer cells often invade lymph node cells and/or metastasize to the liver, brain and/or bone and spread cancer in these tissues and organs.

In some embodiments, the NRBAs, which in some embodiments of this invention are SERMs, and/or compositions comprising the same, of this invention, are useful in affecting/treating/suppressing, etc., prostate cancer, ovarian cancer, cervical cancer, uterine cervical cancer, endometrial carcinoma, vulval cancer or breast cancer.

In one embodiment, the NRBAs, which in some embodiments of this invention are SERMs and/or compositions comprising the same can be used for the prophylaxis or treatment of the aforementioned hormone-dependent diseases.

In another embodiment, this invention provides a method of suppressing, inhibiting, reducing the risk of developing, preventing or treating a subject with endometrial carcinoma, comprising the step of administering to said subject a composition of this invention, in an amount effective to suppress, inhibit, reduce the risk of developing, prevent or treat endometrial carcinoma in said subject.

In some embodiments, the NRBAs, which in some embodiments of this invention are SERMs of this invention are useful for treating endometrial carcinoma, or, in another embodiment, breast cancer, or in another embodiment, any hormone-related disease, condition or disorder, due to overproduction or dysregulated production of estrogen. According to this aspect of the invention, and in one embodiment, such a disease, condition or disorder may be positively affected by the administration of a NRBA, which in some embodiments of this invention is a SERM, is chosen based on its pharmacologic activity as demonstrated in receptor binding studies, estrogen receptor transactivation, *in vitro* studies of osteoblast and osteoclast activity, and *in vivo* studies.

In some embodiment, ER-$\alpha$ antagonists are useful in treating, suppressing, inhibiting or reducing breast cancer and endometrial cancer. In another embodiment, ER-$\beta$ showed increased proliferation of mammary epithelium suggesting that ER-$\beta$ regulates the growth of mammary gland and agonists to ER-$\beta$ would reduce growth of mammary gland. Also, ER-$\beta$ selective ligands have anti-estrogenic activity and thereby prevent the actions of estrogens in mammary carcinoma growth, and are useful in applications thereof, and represent embodiments of this invnetion.

In one embodiment such ER-$\alpha$ antagonists of this invention include *inter alia* 10m, 4a, 11f or 11g. In another embodiment, such ER $\beta$ agonists include *inter alia* 10d, 10f, 101 or 11p, listed in Table 1 or any combination thereof.

In another embodiment, this invention provides a method of suppressing, inhibiting, reducing the risk of developing, preventing or treating a subject with polycystic ovarian syndrome, comprising the step of administering to said subject a compound or composition of this invention, in an amount effective to suppress, inhibit, reduce the risk of developing, prevent or treat polycystic ovarian syndrome in said subject.

In another embodiment, this invention provides a method of suppressing, inhibiting, delaying onset or preventing diabetes, breast cancer, endometrial carcinoma or cardiovascular disease in a female subject suffering from polycystic ovarian syndrome, comprising the step of administering to said subject a compound or composition of this invention, in an amount effective to suppress, inhibit, delay onset, or prevent diabetes, breast cancer, endometrial carcinoma or cardiovascular disease in the subject.

In one embodiment, the NRBAs, which in one embodiment are SERMs and/or compositions comprising the same can be used for the prophylaxis or treatment of sexual dysfunction.

In one embodiment, the sexual dysfunction treated by the methods with the NRBAs, which in one embodiment are SERMs and/or compositions of this invention is in men, and in another embodiment, includes, in the main, erectile dysfunction, male orgasmic disorder, inhibited or hypoactive sexual desire and priapism. Inhibited or hypoactive sexual desire refers to a decrease in desire for, or interest in, sexual activity and can result from a variety of causes, including physical illness, depression, hormonal abnormality or medications that affect libido.

In one embodiment, the NRBAs, which in one embodiment are SERMs and/or compositions comprising the same can be used for the prophylaxis or treatment of sexual dysfunction in women. In one embodiment, the women are premenopausal. In one embodiment, the women are postmenopausal women. According to this aspect of the invention, and in one embodiment, the NRBAs, which in one embodiment are SERMs and/or compositions comprising the same can be used for the prophylaxis or treatment of symptoms associated with the estrogen deprivation of menopause, such as vaginal dryness/lack of lubrication and consequent pain associated with intercourse, which can be closely associated in turn with diminished sexual desire. Other postmenopausal symptoms such as night sweats, hot flushes, insomnia, depression, nervousness, urinary incontinence, irritability and anxiety are also likely to be associated with diminished sexual desire, and may be treated with the NRBAs, which in one embodiment are SERMs of this invention and/or compositions comprising the same.

In another embodiment, the invention provides a contraceptive, and/or a method of use thereof, the contraceptive comprising a composition comprising a NRBA, which in one embodiment is a SERM compound of formula (I)-(X) or its prodrug, ester, analog, isomer, metabolite, derivative, pharmaceutically acceptable salt, pharmaceutical product, polymorph, crystal, impurity, *N*-oxide, hydrate or any combination thereof. In one embodiment, the invention provides a method for providing post-coital contraception by administering the composition comprising a NRBA, which in one embodiment is a SERM compound of formula (I)-(X) or its prodrug, ester, analog, isomer, metabolite, derivative, pharmaceutically acceptable salt, pharmaceutical product, polymorph, crystal, impurity, *N*-oxide, hydrate or any combination thereof.

In one embodiment this invention provides a method of treating a subject suffering from post menauposal conditions, said method comprising the step of administering to said subject a NRBA, which in one embodiment is a SERM compound and/or its pharmaceutically acceptable salt, hydrate, *N*-oxide, or any combination thereof.

In another embodiment this invention provides a method of suppressing, inhibiting or reducing the risk of post menopausal conditions, said method comprising the step of administering to said subject a NRBA, which in one embodiment is a SERM compounds and/or its pharmaceutically acceptable salt, hydrate, *N*-oxide, or any combination thereof.

In another embodiment, the invention provides a method of treating, preventing, suppressing, inhibiting, or reducing the incidence of hot flashes, gynecomastia, and/or hair loss in female subjects, or in another embodiment, in male human subjects. In one embodiment, invention provides a method of treating, preventing, suppressing, inhibiting, or reducing the incidence of hot flashes, gynecomastia, and/or hair loss in a male subject having prostate cancer,

comprising administering a pharmaceutical composition comprising a NRBA, which in one embodiment is a SERM compound of formula (I)-(X) or its prodrug, ester, analog, isomer, metabolite, derivative, pharmaceutically acceptable salt, pharmaceutical product, polymorph, crystal, impurity, *N*-oxide, hydrate or any combination thereof, thereby treating, preventing, suppressing, inhibiting, or reducing the incidence of hot flashes, gynecomastia, and/or hair loss in said male human subjects.

In one embodiment, the term "hot flashes" refers to the following: sudden feeling of heat in the upper part or all of the body, face and neck flush, red blotches appearing on the chest, back and arms, heavy sweating, cold shivering, etc.

It is to be understood that any sex hormone-dependent disease, disorder or condition may be treated via the methods of this invention, using the SERMs/compositions of this invention.

In one embodiment, hot flashes can be treated with any SERM, which has a structure characterized by any of the formulas, as described herein. In one embodiment, hot flashes may be treated, prevented, alleviated with the following SERMs chosen based on their pharmacologic activity as demonstrated in receptor binding studies, estrogen receptor transactivation, *in vitro* studies of osteoblast and osteoclast activity, and *in vivo* studies

In some embodiments ER-α agonists and/or ER-β agonists are useful in treating, suppressing, inhibiting or reducing the risk post menopausal conditions such as hot flashes, hot sweats, night sweats, hot flush, depression and/or anxiety. In another embodiment ER-α agonists of this invention include *inter alia* 3v, 3b, 3k or 10x, or any combination thereof. In another embodiment ER-β agonist of this invention include *inter alia* 10d, 10f, 101 or 11p, listed in table 1, or any combination thereof.

Hot flash is mediated by both ER-α and ER-β. To overcome this, tissue selective agonists of both the isoforms can be used. In some embodiments, side effects associated with some ER-α agonists such as thromboembolism, mammary carcinogenesis and uterine cancer, may be obviated via selection of specific ER-β agonists for this indication.

*Cardiovascular Disease and Obesity*

Cardiovascular cells, as well as reproductive tissues, bone, liver, and brain, express both of the known estrogen receptors, estrogen receptor-α (ER-α) and estrogen receptor-β (ER- β). These receptors are important targets for endogenous estrogen, estrogen replacement therapy (ERT), and pharmacological estrogen agonists. Estrogen—estrogen receptor complexes serve as transcription factors that promote gene expression with a wide range of vascular effects, including regulation of vasomotor tone and response to injury, which may be protective against development of atherosclerosis and ischemic diseases. Estrogen receptors in other tissues, such as the liver, may mediate both beneficial effects (e.g., changes in apoprotein gene expression that improve lipid profiles) and adverse effects (e.g., increases in gene expression of coagulation proteins and/or decreases in fibrinolytic proteins). Two general estrogen-mediated vascular effects are recognized. Rapid, transient vasodilation occurs within a few minutes after estrogen exposure, independently of changes in gene expression. Longer-term effects of estrogen on the vasculature, such as those related to limiting the development of atherosclerotic lesions or vascular injury, occur over hours to days after estrogen treatment and have as their hallmark alterations in vascular gene expression. Progesterone and other hormonal receptors are also expressed in the vasculature.

In one embodiment, the invention provides a method of treating, preventing, reducing the risk of mortality from cardiovascular and/or cerebrovascular disease in a subject, comprising administering a pharmaceutical composition comprising a NRBA, which in one embodiment is a SERM, compound of formula (I) -(X) or its prodrug, ester, analog, isomer, metabolite, derivative, pharmaceutically acceptable salt, pharmaceutical product, polymorph, crystal, impurity, *N*-oxide, hydrate or any combination thereof.

In some embodiments ER-β agonists are useful in treating, preventing, reducing the risk of mortality from cardiovascular and/or cerebrovascular disease in a subject. In another embodiment ER-β agonist of this invention include *inter alia* 10d, 10f, 101 or 11p listed in Table 1 or any combination thereof.

In one embodiment, cardiovascular disease comprises, *inter alia,* atherosclerosis of the coronary arteries, angina pectoris, and myocardial infarction. In one embodiment, cerebrovascular disease comprises, *inter alia,* atherosclerosis of the intracranial or extracranial arteries, stroke, syncope, and transient ischemic attacks.

In one embodiment, the methods/compounds/compositions of this invention are useful in treating a subject having one or more risk factors for cardiovascular disease or cerebrovascular disease, such as hypercholesterolemia, hypertension, diabetes, cigarette smoking, familial or previous history of coronary artery disease, cerebrovascular disease, and cardiovascular disease. Hypercholesterolemia typically is defined as a serum total cholesterol concentration of greater than about 5.2 mmol/L (about 200 mg/dL). In some embodiments, risk factors may comprise hypertension (especially with systolic pressures greater than 160 mmHg), cardiac disease, transient ischemic attacks, diabetes mellitus, carotid bruits, and sickle cell disease. Obesity, a sedentary lifestyle, tobacco use, alcohol consumption, and elevated serum cholesterol and lipid levels may also be risk factors for cerebrovascular disease.

In another embodiment, the invention provides a method of improving a lipid profile in a subject, comprising

administering a pharmaceutical composition comprising a NRBA, which in one embodiment is a SPERM compound of formula (I)-(X) or its prodrug, ester, analog, isomer, metabolite, derivative, pharmaceutically acceptable salt, pharmaceutical product, polymorph, crystal, impurity, *N*-oxide, hydrate or any combination thereof, thereby improving the lipid profile in said subject.

In some embodiments, the phrase "improving a lipid profile" may refer to lowering circulating lipid levels, lowering plaque formation in vasculature, altering circulating HDL/LDL ratios, lowering circulating cholesterol levels, preventing lipid accumulation in vasculature, or any combination thereof, or other therapeutic effects related thereto, as will be appreciated by one skilled in the art.

In one embodiment, the present invention provides NRBAs/SERMs for reducing a fat mass in a subject. In another embodiment the compound is aprodrug, ester, analog, isomer, metabolite, derivative, pharmaceutically acceptable salt, pharmaceutical product, polymorph, crystal, impurity, *N*-oxide, hydrate or any combination thereof, of the NRBAs/SERMs described herein, or a composition comprising the same.

In another embodiment, this invention provides for the use of a NRBA, which in one embodiment is a SERM compound of this invention, or its prodrug, ester, analog, isomer, metabolite, derivative, pharmaceutically acceptable salt, pharmaceutical product, polymorph, crystal, impurity, *N*-oxide, hydrate or any combination thereof, or a composition comprising the same, in treating obesity or diabetes associated with a metabolic syndrome in a subject

In another embodiment, the subject has a hormonal imbalance, disorder, or disease. In another embodiment the subject has menopause, or in another embodiment, the subject has andropause, or age-related androgen decline in a male subject.

In one embodiment, the present invention provides a use of a NRBA, which in one embodiment is a SERM compound as described for increasing a lean mass in a subject. In one embodiment the subject has a hormonal imbalance, disorder, or disease. In another embodiment the subject has a disease listed herein, which is negatively impacted by obesity, or the presence of greater fat mass, or in another embodiment, is positively affected by the increase in lean mass in the subject.

In another embodiment, this invention relates to a method of preventing, suppressing, inhibiting or reducing the incidence of obesity in a subject, comprising the step of administering to the subject a NRBA, which in one embodiment is a SERM as herein described and/or its analog, ester, derivative, isomer, metabolite, pharmaceutically acceptable salt, pharmaceutical product, hydrate, *N*-oxide, prodrug, polymorph, crystal, or any combination thereof, in an amount effective to prevent, suppress, inhibit or reduce the incidence of obesity in the subject.

In one embodiment, the NRBA, which in one embodiment is a SERM compound, when administered, alters the levels of leptin in a subject. In another embodiment, the NRBA/SERM compounds decrease the levels of leptin. In another embodiment, the NRBA/SERM compounds increase the levels of leptin in a subject. In one embodiment, the NRBAs/SERMs effect circulating, or in another embodiment, tissue levels of leptin. In one embodiment, the term 'level/s of leptin' refers to the serum level of leptin.

Since leptin is implicated in controlling appetite, weight loss, food intake, and energy expenditure, modulating and/or controlling the levels of leptin is a useful therapeutic approach in treating preventing, inhibiting or reducing the incidence of obesity in subjects suffering from obesity. Modulating the level of leptin can result in a loss of appetite, a reduction of food intake, and an increase in energy expenditure in the subject, and thus may contribute to the control and treatment of obesity.

The term "obesity" refers, in one embodiment, to an increase in body weight beyond the limitation of skeletal and physical requirement, as the result of excessive accumulation of fat in the body.

The term "obesity-associated metabolic disorder" refers, in one embodiment, to a disorder which results from, is a consequence of, is exacerbated by or is secondary to obesity. Non-limiting examples of such a disorder are osteoarthritis, Type II diabetes mellitus, increased blood pressure, stroke, and heart disease.

The term "diabetes", in one embodiment, refers to a relative or absolute lack of insulin leading to uncontrolled carbohydrate metabolism. Most patients can be clinically classified as having either insulin-dependent diabetes mellitus (IDDM or Type-I diabetes) or non-insulin-dependent diabetes mellitus (NIDDM or Type-II diabetes).

The term "increased blood pressure" or "hypertension" refers, in other embodiments, to a repeatedly high blood pressure above 140 over 90 mmHg. Chronically-elevated blood pressure can cause blood vessel changes in the back of the eye, thickening of the heart muscle, kidney failure, and brain damage.

The term "stroke" refers, in other embodiments, to damage to nerve cells in the brain due to insufficient blood supply often caused by a bursting blood vessel or a blood clot. The term "heart disease", in other embodiments, refers to a malfunction in the heart normal function and activity, including heart failure.

In one embodiment, the NRBA/s or SERM/s as described herein, bias toward lean mass versus fat accumulation, which can be applied to any of the conditions for which the methods of this invention are applicable.

According to this aspect of the invention, and in one embodiment, the NRBA/s or SERMs of this invention may be co-administered with adrenergic, anti-adrenergic, anti-androgen, anti-anginal, antihyperlipidemic, antihyperlipoproteinemic, antihypertensive, anti-inflammatory, aldosterone antagonists, amino acids, blood glucose regulators, cardioprotectants; cardiovascular agents; cholinergic agonist and antagonists, cholinesterase deactivators or inhibitors, enzyme inhibitors, free oxygen radical scavengers, hormones, antihypocholesterolemic agents, hypolipidemic agents, hypotensive agents, immunizing agents, immunostimulants, potassium channel openers, post-stroke and post-head trauma treatments, prostaglandins, sodium and calcium channel blockers, thyroid hormones and inhibitors, etc.

In another embodiment, this invention relates to a method of promoting, increasing or facilitating weight loss in a subject, comprising the step of administering to the subject a NRBA/s or SERM/s as described herein and/or an analog, derivative, isomer, metabolite, pharmaceutically acceptable salt, pharmaceutical product, hydrate, *N*-oxide, ester, prodrug, polymorph, crystal, or any combination thereof, in an amount effective to promote, increase or facilitate weight loss in the subject.

In another embodiment, this invention relates to a method of decreasing, suppressing, inhibiting or reducing appetite of a subject, comprising the step of administering to the subject a NRBA/s or SERM/s as described herein and/or its analog, derivative, isomer, metabolite, pharmaceutically acceptable salt, pharmaceutical product, hydrate, *N*-oxide, ester, prodrug, polymorph, crystal, or any combination thereof, in an amount effective to decrease, suppress, inhibit or reduce the appetite of the subject

In another embodiment, this invention relates to a method of altering the body composition of a subject, comprising the step of administering to the subject a NRBA or SERM as described herein and/or its analog, derivative, isomer, metabolite, pharmaceutically acceptable salt, pharmaceutical product, hydrate, *N*-oxide, ester, prodrug, polymorph, crystal, or any combination thereof, in an amount effective to alter the body composition of the subject. In one embodiment, altering the body composition comprises altering the lean body mass, the fat free body mass of the subject, or a combination thereof.

In another embodiment, this invention relates to a method of altering lean body mass or fat free body mass of a subject, comprising the step of administering to the subject a NRBA/s or SERM/s as described herein and/or its analog, derivative, isomer, metabolite, pharmaceutically acceptable salt, pharmaceutical product, hydrate, *N*-oxide, ester, prodrug, polymorph, crystal, or any combination thereof, in an amount effective to alter the lean body mass or fat free body mass of the subject

In another embodiment, this invention relates to a method of converting fat to lean muscle in a subject, comprising the step of administering to the subject a NRBA/s or SERM/s as described herein and/or its analog, derivative, isomer, metabolite, pharmaceutically acceptable salt, pharmaceutical product, hydrate, *N*-oxide, prodrug, polymorph, ester, crystal, or any combination thereof, in an amount effective to convert fat to lean muscle in the subject.

In another embodiment, this invention relates to a method of treating an obesity-associated metabolic disorder in a subject, comprising the step of administering to the subject a NRBA/s or SERM/s as described herein and/or its analog, derivative, isomer, metabolite, pharmaceutically acceptable salt, pharmaceutical product, hydrate, *N*-oxide, ester, prodrug, polymorph, crystal, or any combination thereof, in an amount effective to treat the obesity-associated metabolic disorder in the subject.

In another embodiment, this invention relates to a method of preventing, suppressing, inhibiting or reducing an obesity-associated metabolic disorder in a subject, comprising the step of administering to the subject a selective estrogen receptor modulator NRBA/s or SERM/s as described herein and/or its analog, derivative, isomer, metabolite, pharmaceutically acceptable salt, pharmaceutical product, hydrate, *N*-oxide, ester, prodrug, polymorph, crystal, or any combination thereof, in an amount effective to prevent, suppress, inhibit or reduce the obesity-associated metabolic disorder in the subject.

In one embodiment, the obesity-associated metabolic disorder is hypertension. In another embodiment, the disorder is osteoathritis. In another embodiment, the disorder is Type II diabetes or diabetes mellitus. In another embodiment, the disorder is increased blood pressure. In another embodiment, the disorder is stroke. In another embodiment, the disorder is heart disease.

In another embodiment, this invention relates to a method of decreasing, suppressing, inhibiting or reducing adipogenesis in a subject, comprising the step of administering to the subject a NRBA/s or SERM/s as described herein and/or its analog, derivative, isomer, metabolite, pharmaceutically acceptable salt, pharmaceutical product, hydrate, *N*-oxide, ester, prodrug, polymorph, crystal, or any combination thereof, in an amount effective to decrease, suppress, inhibit or reduce adipogenesis in the subject.

In one embodiment, the NRBA or SERM is useful in a) treating, preventing, suppressing, inhibiting, or reducing obesity; b) promoting, increasing or facilitating weight loss; c) decreasing, suppressing, inhibiting or reducing appetite; d) altering the body composition; e) altering lean body mass or fat free body mass; f) converting fat to lean muscle; g) treating, preventing, suppressmg, inhibiting, or reducing an obesity-associated metabolic disorder, for example hypertension, osteoarthritis, Type II diabetes or diabetes mellitus, increased blood pressure, stroke, or heart disease; h) decreasing, suppressing, inhibiting or reducing adipogenesis; and/or i) altering the level of leptin.

In one embodiment, the NRBA/s or SERMs of this invention find utility in treating or halting the progression of, or treating symptoms of diabetes, In one embodiment, the administration of the NRBA/s or SERM/s as described herein to a subject at risk of diabetes, or in other embodiments, other metabolic disorders, may alter progression of the disease,

or in another embodiment, prevent disease, or in another embodiment, reduce the severity, or in another embodiments, reduce symptoms associated with the disease. In one embodiment, the treatment is initiated upon first indication of a risk factor, or in another embodiment, upon evidence of early stages of disease, as will be appreciated by one skilled in the art. For example, and in one embodiment, treatment of a subject may be initiated with the subject's presentation of hyperinsulinemia, or in another embodiment, gestational diabetes, or in another embodiment, hyperglycemia, or in another embodiment, impaired glucose tolerance, etc., as will be appreciated by one skilled in the art.

In another embodiment, the NRBA/s or SERMs as described herein are useful in treating co-morbidities related to diabetes. These conditions include: hypertension, cerebrovascular disease, atherosclerotic coronary artery disease, macular degeneration, diabetic retinopathy (eye disease) and blindness, cataracts, systemic inflammation (characterized by elevation of inflammatory markers such as erythrocyte sedimentation rate or C-reactive protein), birth defects, pregnancy related diabetes, pre-ecclampsia and hypertension in pregnancy, kidney disease (renal insufficiency, renal failure etc.), nerve disease (diabetic neuropathy), superficial and systemic fungal infections, congestive heart failure, gout/ hyperuricemia, obesity, hypertriglyceridemia, hypercholesterolemia, fatty liver disease (non-alcoholic steatohepatitis, or NASH), and diabetes-related skin diseases such as necrobiosis lipoidica diabeticorum (NLD), blisters of diabetes (Bullosis Diabeticorum), eruptive xanthomatosis, digital sclerosis, disseminated granuloma annulare, and acanthosis nigricans.

In one embodiment this invention provides a method for a) treating, preventing, suppressing or inhibiting atherosclerosis b) treating, preventing, suppressing inhibiting liver damage due to fat deposits comprising the step of administering to the subject a NRBA/s or SERM/s as described herein and/or its analog, derivative, isomer, metabolite, pharmaceutically acceptable salt, pharmaceutical product, hydrate, *N*-oxide, ester, prodrug, polymorph, crystal, or any combination thereof, or a composition comprising the same, in an amount effective to treat, prevent or inhibit atherosclerosis and liver damage due to fat deposit.

In one embodiment, the NRBA/s or SERM/s as described herein is useful in a) treating, preventing, suppressing, inhibiting, or reducing atherosclerosis; b) treating, preventing, suppressing or inhibiting liver damage due to fat deposits.

In one embodiment atherosclerosis refers to a slow, complex disease that may begin with damage to the innermost layer of the artery. In another embodiment the causes of damage to the arterial wall may include a) elevated levels of cholesterol and in the blood; b) high blood pressure; c) tobacco smoke d) diabetes. In another embodiment, the condition is treatable in a smoker, despite the fact that tobacco smoke may greatly worsen atherosclerosis and speed its growth in the coronary arteries, the aorta and arteries in the legs. Similarly, in another embodiment, the methods of this invention may be useful in treating subjects with a family history of premature cardiovascular disease who have an increased risk of atherosclerosis.

In one embodiment, liver damage due to fat deposits refer to the build-up of fat in the liver cells forming a fatty liver which may be associated with or may lead to inflammation of the liver. This can cause scarring and hardening of the liver. When scarring becomes extensive, it is called cirrhosis.

In another embodiment the fat accumulates in the liver as obesity. In another embodiment fatty liver is also associated with diabetes mellitus, high blood triglycerides, and the heavy use of alcohol. In another embodiment fatty liver may occur with certain illnesses such as tuberculosis and malnutrition, intestinal bypass surgery for obesity, excess vitamin A in the body, or the use of certain drugs such as valproic acid (trade names: Depakene/Depakote) and corticosteroids (cortisone, prednisone). Sometimes fatty liver occurs as a complication of pregnancy. In one embodiment, the NRBA/s, which in some embodiments are SERM/s as described herein may be used to treat any of these conditions, and represents another embodiment of this invention. In one embodiment, use of the NRBA/s of this invention will not result in toxic effects in the liver, even with prolonged use.

In another embodiment, the invention provides a method of treating or preventing peripheral vascular disease (PVD) by use of a NRBA, which in some embodiments is a SERM of this invention to regulate the rheological behaviour of blood thereby improving microcirculation. In another embodiment, the PVD may be caused by hypertension. In another embodiment, the PVD may be caused by diabetes. In another embodiment, the PVD may be caused by a vaso-occlusive event.

In one embodiment, the phrase "vaso-occlusive event" refers to an event that is characterized by or results in a decrease in the internal diameter of blood vessels either locally or systemically to an extent which impedes blood flow in a subject and/or is of a pathological nature. In one embodiment, a vaso-occlusive event encompasses pathological narrowing or occlusion of a stent, a vascular graft or a blood vessel. In one embodiment, the phrase, "pathological narrowing or occlusion" refers to narrowing or occlusion which is abnormal and/or disease-related. A vaso-occlusive event includes events which cause blood vessel narrowing or occlusion (such as thrombotic events, thromboembolic events and intimal hyperplasia) as well as conditions which result from such blood vessel narrowing (such as myocardial infarction and ischemic stroke).

Thrombotic events including thromboembolic events can be serious medical conditions particularly since they can cause a reduction in blood flow to critical organs including the brain and myocardium. Examples of thrombotic events include but are not limited to arterial thrombosis, including stent and graft thrombosis, cardiac thrombosis, coronary thrombosis, heart valve thrombosis and venous thrombosis. Cardiac thrombosis is thrombosis in the heart. Arterial

thrombosis is thrombosis in an artery. Coronary thrombosis is the development of an obstructive thrombus in a coronary artery, often causing sudden death or a myocardial infarction. Venous thrombosis is thrombosis in a vein. Heart valve thrombosis is a thrombosis on a heart valve. Stent thrombosis is thrombosis resulting from and/or located in the vicinity of a vascular stent. Graft thrombosis is thrombosis resulting from and/or located in the vicinity of an implanted graft, particularly a vascular graft.

Examples of conditions or disorders that result from thrombotic events include but are not limited to myocardial infarction, stroke, transient ischemic attacks, amaurosis fugax, aortic stenosis, cardiac stenosis, coronary stenosis and pulmonary stenosis. Stenosis is the narrowing or stricture of a duct or canal. Coronary stenosis is the narrowing or stricture of a coronary artery. Cardiac stenosis is a narrowing or diminution of any heart passage or cavity. Pulmonary stenosis is the narrowing of the opening between the pulmonary artery and the right ventricle. Aortic stenosis is narrowing of the aortic orifice of the heart or of the aorta itself.

Vaso-occlusive events also include disorders in which the blood vessel narrowing results not necessarily from a thrombus but rather a thickening of the vessel wall such as with intimal hyperplasia. Intimal hyperplasia refers to a condition characterized by abnormal proliferation of the cells of the intimal layer of the blood vessel wall.

Thus, one aspect of the invention relates to methods/compounds/compositions for reducing the risk of a thrombotic event. In one embodiment, the method reduces the risk of stroke. Stroke is a condition resulting from the lack of oxygen to the brain, resulting from one or more occlusive thrombi. Depending on the area of the brain affected, stroke can result in a wide range of symptoms from transient ischemic attacks to death (e.g., coma, reversible or irreversible paralysis, speech problems or dementia). In preferred embodiments, the stroke is non-hemorrhagic in nature.

The methods/compounds/compositions of the invention in other embodiments relates to reducing the risk of myocardial infarction. Myocardial infarction refers to an irreversible injury to the heart muscle. Myocardial infarction generally results from an abrupt decrease in coronary blood flow following a thrombotic occlusion (e.g., a thromboembolism) of a coronary artery. The thrombus, in many instances, forms after the rupture of atherosclerotic plaques in diseased coronary arteries. Such injury is highly correlated with factors such as cigarette smoking, hypertension and lipid accumulation.

Transient ischemic attack is a transient acute neurological dysfunction resulting from a thromboembolism in the cerebral circulation. Amaurosis fugax is the temporary monocular blindness resulting from a thromboembolism in the retinal vasculature.

The methods/compounds/compositions of the invention can be used to reduce the risk of a primary or a secondary vaso-occlusive event such as a thrombotic event or to inhibit the progression of such an event A primary vaso-occlusive event refers to the first known vaso-occlusive event experienced by the subject. A secondary vaso-occlusive event refers to a vaso-occlusive event which occurs in a subject known or diagnosed as having previously experienced a vaso-occlusive event (i.e., a primary vaso-occlusive event).

It is to be understood that any of these conditions, predispositions, symptoms, byproducts, etc. of the same, may be positively affected by the administration of a compound/composition of this invention, and such use is to be considered an embodiment thereof.

In another embodiment, the invention provides a method of protection against the development of Alzheimer disease in a subject, comprising administering a pharmaceutical composition comprising a SERM compound of formula (I)-(X) or its prodrug, analog, isomer, metabolite, derivative, pharmaceutically acceptable salt, pharmaceutical product, polymorph, crystal, impurity, *N*-oxide, ester, hydrate or any combination thereof, hereby protecting against the development of Alzheimer disease in said subject.

*Anti-inflammatory effects of SERMs*

Inflammation is a common and potentially debilitating condition that occurs when the white blood cells and endogenous chemicals that can protect us from infection and foreign substances such as bacteria and viruses act on tissue surrounding a wound or infection. In some diseases, however, the body's defense system (immune system) triggers an inflammatory response when there are no foreign substances to fight off. In these diseases, called autoimmune diseases, the body's normally protective immune system causes damage to its own tissues. The body responds as if normal tissues are infected or somehow abnormal. Some, but not all types of arthritis are the result of misdirected inflammation. Arthritis is a general term that describes inflammation in joints and affects more than 2-4% of the world's population. There are many medications available to decrease swelling and inflammation and hopefully prevent or minimize the progression of the inflammatory disease. The medications include non-steroidal anti-inflammatory drugs (NSAIDs - such as aspirin, ibuprofen or naproxen), corticosteroids (such as prednisone), anti-malarial medications (such as hydroxychloroquine), and other medications including gold, methotrexate, sulfasalazine, penicillamine, cyclophosphamide and cyclosporine.

The role of estrogen receptor and its ligands as therapy for inflammation has been under consideration. The effects are regarded to be mediated by the isoform ER-β. Treatment of rats with estradiol or SERMs such as raloxifene

and tamoxifen has been shown to reduce the incidence of lipo-polysacharride induced inflammatory responses. One of the pathways through which inflammatory responses are mediated is through the activation of NFκB pathway. Nuclear receptor ligands inhibit the NFκB activity through protein protein interaction. Recently it was shown that SERMs inhibit the inflammatory responses by inhibiting the NFκB function without having estrogenic effects on other reproductive tissues.

In another embodiment, the invention provides a method of treating, preventing, inhibiting reducing the incidence of inflammatory conditions in a subject, comprising administering a pharmaceutical composition comprising a SERM compound of formula (I)-(X) or its prodrug, analog, isomer, metabolite, derivative, pharmaceutically acceptable salt, pharmaceutical product, polymorph, crystal, impurity, *N*-oxide, ester, hydrate or any combination thereof, thereby treating, preventing, inhibiting reducing the incidence of inflammatory conditions in a subject.

In some embodiments ER-β agonists are useful in treating, preventing, inhibiting reducing the incidence of inflammatory conditions in a subject. In another embodiment ER- β agonist of this invention include *inter alia* 10d, 10f, 101 or 10p, listed in Table 1 or any combination thereof.

The following examples are presented in order to more fully illustrate the preferred embodiments of the invention. They should in no way be construed, however, as limiting the broad scope of the invention.

EXAMPLES

**EXAMPLE 1:**

**Novel SERMs and their Estrogen Receptor Binding Affinities, Agonist and**

**Antagonist Activity**

**Materials and Methods:**

ER binding affinity was determined via one of the following methods:

**Method 1:**

Human recombinant ER was expressed in insect Sf9 cells and performed a radioactive competitive binding assay with tritiated estradiol. If the NRBA compounds tested showed a ≥ 50% inhibition of [$^3$H] estradiol binding at 1 μM (1000 nM) concentration, they were tested using four concentrations of the compound to give an estimated $IC_{50}$ and $K_i$ value.

**Method 2:**

Estrogen receptor (BR) binding affinity of NRBA compounds was also determined using an *in vitro* competitive radioligand-binding assay with [$^3$H]-estradiol ([$^3$H]-$E_2$, PerkinElmer), a high affinity ligand for both ERα and ERβ. The equilibrium dissociation constant ($K_d$) of [$^3$H]-$E_2$ was determined by incubating increasing concentrations of [$^3$H]-$E_2$ (0.01 to 10 nM) with bacterial expressed ERα or β ligand binding domain (LBD) at 4°C for 18h. Non-specific binding was determined by adding 1000 nM $E_2$ to the incubation mixture. It was determined that the minimum concentration of [$^3$H]-$E_2$ required to saturate ERα and ERβ binding sites in the incubation mixture was 1 nM, respectively. The binding affinity of the NRBA compounds was determined under identical conditions by incubating increasing concentrations ($3\times10^{-2}$ to 1,000 nM) of ligand with isolated ER LBD and 1 nM [$^3$H]-$E_2$. Following incubation, bound and free [$^3$H]-$E_2$ was separated by using vacuum filtration with the Harvester (PerkinElmer). Briefly, the incubation mixture was filtered through a high affinity protein binding filter, and washed several times to remove any unbound radioactivity. The filter plate was air dried and sealed on the bottom. Scintillation cocktail was added to each well and the top of the plate was sealed. Radioactivity was counted in a TopCount NXT Microplate Scintillation Counter.

Specific binding of [$^3$H]-$E_2$ (B) at each concentration of SERM was obtained by subtracting the nonspecific binding of [$^3$H]-$E_2$, and expressed as a percentage of the specific binding of [$^3$H]-$E_2$ in the absence of SERM, ($B_0$). The concentration of SERM that reduced the specific binding of [$^3$H]-$E_2$ by 50% ($IC_{50}$) was determined by computer-fitting the data by nonlinear regression analysis using SigmaPlot (SPSS Inc., Chicago, IL) to the following equation:

$$B = B_0 * [1 - C/(IC_{50} + C)]$$

where *C* is the concentration of SERM.

The equilibrium dissociation constant (*Ki*) of SERM was calculated by:

$$K_i = K_d * IC_{50}/(K_d + L)$$

where $K_d$ is the equilibrium dissociation constant of [$^3$H]-$E_2$ (ERα=0.65 nM, ERβ=1.83 nM), and *L* is the concentration of [$^3$H]-$E_2$ (1 nM).

Table 1 presents a series of NRBA compounds. Representative NRBAs are described hereinbelow, whose activity under specific experimental conditions is provided. It is to be understood that while the indicated compounds may exhibit a particular activity (for example, compound 3v is an agonist) under the experimental conditions employed, as a function, in some embodiments of the particular cells utilized, etc., such compounds may possess alternate or varied activity in different experimental settings.

Representative examples of the NRBAs of this invention and their activity under the indicated conditions are as follows:

ER alpha agonists: **3v** (*ER-α: $K_i$* =20 nM; *$EC_{50}$*=22.4 nM), **3b** (*ER-α: $K_i$* =14 nM; *$EC_{50}$*=10 nM), **3k** (*ER-α: $K_i$* =129 nM; *$EC_{50}$*= 12 nM), **10x** (*ER-α: $K_i$*=13 nM; *$EC_{50}$*=16 nM).

ER alpha antagonists: **10m** (*ER-α: $K_i$*=221 nM; $IC_{50}$=<10 nM), **4a** (*ER-α: Ki*=111 nM; $IC_{50}$=35 nM), **11f** (*ER-α: $K_i$*=60nM*;* $IC_{50}$=69 nM), and **11g** (*ER-α: $K_i$*=79 nM; $IC_{50}$=16 nM)

ER beta agonists: **10d** (*ER-β: $K_i$* =61 nM; *$EC_{50}$*= 85 nM), **10f** (*ER-β : $K_i$* =57 nM; *$EC_{50}$*= 42 nM), 101 (*ER-β: $K_i$*=82 nM; *$EC_{50}$*= 27 nM), **11p** (*ER-β: $K_i$*=108 nM; *$EC_{50}$*= <10 nM)

ER beta antagonist: **10j** (*ER-β: $K_i$* =36 nM;; $IC_{50}$ = 20 nM), **10n** (*ER-β: $K_i$* =92 nM; $IC_{50}$ = 47 nM), **10t** (*ER-β : $K_i$* = <10 nM; $IC_{50}$ =17 nM)

**Table 1:**

| **Compound # and IUPAC NAME** | **PHYSICAL CHARACTERIZATION** |
|---|---|
| Estradiol (E2) | |
| Propyl pyrazole triol (PPT) | |
| Diproplonitrile (DPN) | |
| ICI-182780 | |
| **5d**<br>4-Hydroxy-*N*-(4-hydroxyphenyl)-*N*-(4-methoxyphenyl)-benzamide | tan solid. 95 % yield. M.p.239-241 °C. $^1$H NMR (DMSO-*$d_6$*, 300 MHz) δ 9.80 (bs, 1H), 9.46 (bs, 1H), 7.23-7.21 (m, 2H), 7.08-7.05 (m, 2H), 6.96-6.93 (m, 2H), 6.87-6.84 (m, 2H), 6.68-6.65 (m, 2H), 6.60-6.57 (m, 2H), 3.72 (s, 3H). MS *mlz* 334 (M-H)$^-$. |
| **5e**<br>*N*-(4-Hydroxyphenyl)-4-methoxy-*N*-(4-methoxyphenyl)-benzamide | tan solid. 90% yield. M.p. 205-206 °C. $^1$H NMR (DMSO-*$d_6$*, 300 MHz) δ 9.48 (bs, 1H), 7.35-7.32 (m, 2H), 6.99-6.97 (m, 2H), 6.87-6.85 (m, 2H), 6.81-6.77 (in, 2H), 6.68-6.66 (m, 2H), 3.72 (s, 6H), MS *mlz* 348 (M-H)$^-$. |
| **4n**<br>4-Methoxy-*N*-(4-methoxyphenyl)-*N*-[4-(2-piperidin-1-ylethoxy)phenyl]-benzamide | white solid. 88% yield. M.p. 163-165 °C. $^1$H NMR (DMSO-*$d_6$*, 300 MHz) δ 7.36 (d, 2H, *J* = 8.69 Hz), 7.16-7.07 (m, 4H), 6.94 (d, 2H, *J* = 8.82 Hz), 6.87 (d, 2H, *J* = 8.82 Hz), 6.80 (d, 2H, *J* = 8.75 Hz), 4.38-4.35 (m, 2H), 3.69 (s, 6H), 3.48-3.44 (m, 4H), 2.51-2.50 (m, 2H), 1.78-1.66 (m, 4H), 1.41-1.37 (m, 2H). MS *m*/*z* 461 (M+H)$^+$. |
| **3u**<br>*N*-Biphenyl-4-yl-*N*-(4-hydroxyphenyl)-4-methoxybenzamide | tan solid. 21 % yield. M.p. 232-234 °C. $^1$H NMR (DMSO-*$d_6$*, 300 MHz) δ 9.55 (s, 1H). 7.65-7.62 (m, 4H), 7.45 (t, 1H, *J* = 7.69 Hz), 7.40-7.34 (m, 4H), 7.23 (d, 2H, *J* = 8.55 Hz), 7.03-7.02 (m, 2H), 6.82-6.80 (m, 2H), 6.71-6.69 (m, 2H), 3.73 (s, 3H),. MS *m*/*z* 418 (M+Na)$^+$. |

(continued)

| Compound # and IUPAC NAME | PHYSICAL CHARACTERIZATION |
|---|---|
| **3v**<br>*N*-Biphenyl-4-yl-4-hydroxy-*N*-(4-hydroxyphenyl)-benzamide | white solid. 49% yield. M.p. 253-255 °C. $^1H$ NMR (DMSO-$d_6$, 300 MHz) δ 7.65-7.61 (m, 4H), 7.45 (t, 2H, *J* = 7.69 Hz), 7.36-7.33 (m, 1H), 7.28-7.26 (m, 2H), 7.21-7.19 (m, 2H), 7,01-6.98 (m, 2H), 6.71-6.68 (m, 2H), 6.62-6.60 (m, 2H). MS *m*/*z* 404 (M+Na)$^+$. |
| **3w**<br>4-Hydroxy-*N*-(4-hydroxyphenyl)-*N*-[4-(2-piperidin-1-ylethoxy)phenyl]-benzamide | tan solid. 46% yield. M.p. 233-235 °C. $^1H$ NMR (DMSO-$d_6$, 300 MHz) δ 9.81 (bs, 1H), 9.47 (bs, 1H), 7.24-7.20 (m, 2H), 7.05-7.03 (m, 2H), 6.96-6.93 (m, 2H), 6.87-6.84 (m, 2H), 6.68-6.65 (m, 2H), 6.60-6.57 (m, 2H), 4.02-3.99 (m, 2H), 2.63-2.60 (m, 2H), 2.09-2.08 (m, 4H), 1.48-1.36 (m, 6H). MS *m*/*z* 433 (M+H)$^+$. |
| **2w**<br>4-Cyano-*N*-(4-methoxyphenyl)-*N*-phenylbenzamide | pale-yellow solid. 96% yield. M.p. 125-129 °C. $^1H$ NMR (DMSO-$d_6$, 300 MHz) δ 7.77-7.74 (m, 2H), 7.61-7.58 (m, 2H), 7.34-7.21 (m, 7H), 6.88(d, 2H, *J* = 7.92 Hz), 3.71 (s, 3H). MS *m*/*z* 351 (M+Na)$^+$. |
| **4o**<br>*N*-Biphenyl-4-yl-*N*-(4-hydroxyphanyl)-4-(2-piperidin-1-ylethoxy)-benzamide | pale-yellow solid. 40% yield. M.p. 132-135 °C. $^1H$ NMR (DMSO-$d_6$, 300 MHz) δ 7.65-7.61 (m, 4H), 7.47-7.45 (m, 2H), 7.36-7.33 (m, 1H), 7.28-7.26 (m, 2H), 7.21-7.19 (m, 2H), 7.01-6.98 (m, 2H), 6.70-6.67 (m, 2H), 6.62-6.61 (m, 2H), 4.05 (bs, 2H), 2.66 (bs, 2H), 2.50-2.45 (m,4H), 1.49-1.38 (m, 6H). MS *m*/*z* 493 (M+H)$^+$. |
| **3x**<br>3-Hydroxy-*N*-(4-hydroxyphenyl)-*N*-phenyl-benzamide | tan solid. 78% yield. M.p. 221-222 °C $^1H$ NMR ((DMSO-$d_6$, 300 MHz) δ 9.51 (bs, 2H), 7.33-7.29 (m, 2H), 7.19-7.15 (m, 3H), 7.04-6.98 (m, 3H), 6.82-6.66 (m, 5H). MS *m*/*z* 304 (M-H)$^-$. |
| **10a**<br>4-cyano-*N*-(4-hydroxyphenyl)-*N*-phenylbenzamide | yellow solid, 74% yield, M.p. 243-244 °C. $^1H$ NMR (DMSO-$d_6$, 300 MHz) δ 7.76-7.74 (m, 1H), 7.58-7.56 (m, 2H), 7.33-7.21 (m, 5H), 7.09-7.08 (m, 2H), 6.67 (s, 2H). MS *m*/*z* 313 (M-H)$^-$. |
| **4p**<br>4-Methoxy-*N*-phenyl-*N*-[4-(2-piperidin-1-ylethoxy) phenyl]-benzamide | yellow solid. 84% yield. M.p. 139-141 °C. $^1H$ NMR (DMSO-$d_6$, 300 MHz) δ 7.35-7.33 (m, 4H), 7.26-7.22 (m, 5H), 7.15-7-14 (m, 2H), 7.12-7.11 (m, 2H), 6.91-6.87 (m, 2H), 6.83-6.80 (m, 2H), 4.02 (t, 2H, *J* = 5.79 Hz), 3.72 (s, 3H), 2.63 (t, 2H, *J* = 5.79 Hz), 2.41 (bs, 4H), 1.48-1.46 (m, 4H), 1.38-1.36 (m, 2H). MS *m*/*z* 507 (M+H)$^+$. |
| **2y**<br>4-Cyano-*N*-(3-methoxyphenyl)-*N*-phenylbenzamide | brown oil. 85 % yield. $^1H$ NMR (DMSO-$d_6$, 300 MHz) δ 7.77-7.75 (m, 2H). 7.63-7.61 (m, 2H), 7,35-7.30 (m, 4H), 7.25-7.22 (m, 2H), 6.91 (s, 1H), 6.83-6.80 (m, 2H), 3.67 (s, 3H). MS *m*/*z* 351 (M+Na)$^+$. |
| **2x**<br>4-Cyano-*N,N*-diphenylbenzamide | tan solid. 85% yield. M.p.145-147 °C. $^1H$ NMR (DMSO-$d_6$, 300 MHz) δ7.76-7.74 (m, 2H), 7.61-7.59 (m, 2H), 7.34-7.22 (m, 10H). MS *mlz* 321 (M+Na)$^+$. |
| **4q**<br>*N*-(4-Hydroxyphenyl)-*N*-phenyl-3-(2-piperidin-1-ylethoxy)-benzamide | tan solid. 64% yield. M.p. 93-95 °C. $^1H$ NMR (DMSO-$d_6$) δ 9.56 (d, 1H, *J* = 6.00 Hz), 7.35-7.30 (m, 2H), 7.23-7.14 (m, 4H), 7.05-6.76 (m, 5H), 6.69-6.67 (m, 2H), 4.20 (bs, 2H), 2.81-2.73 (m, 6H), 1.48 (bs, 4H), 1.46 (bs, 2H). MS *m*/*z* 417 (M+H)$^+$. |

(continued)

| Compound # and IUPAC NAME | PHYSICAL CHARACTERIZATION |
|---|---|
| **3y** *N*-Biphenyl-4-yl-4-hydroxy-*N*-[4-(2-piperidin-1-ylethoxy) phenyl]-benzamide | pale-yellow solid. 46% yield. M.p.109-112 °C $^1$H NMR ((DMSO-$d_6$, 300 MHz) δ 9.89 (s, 1H), 7.65-7.61 (m, 4H), 7.46-7.43 (m, 2H), 7.37-7.33 (m, 1H), 7.31-7.28 (m, 2H), 7.22-7.19 (m, 2H), 7.12-7.09 (m, 2H), 6.91-6.89 (m, 2H), 6.63-6.60 (m, 2H), 4,04 (bs, 2H), 2.67-2.64 (m, 2H), 2,46 (bs, 2.46 4H), 1.50-1.49 (m, 4H), 1.37 (bs, 2H). MS *m*/*z* 493 $(M+H)^+$. |
| **10b** *N*-(biphenyl-4-yl)-4-cyano-*N*-(4-methoxyphenyl)-benzamide | yellow solid, 70% yield, M.p. 209-211 °C. $^1$H NMR (DMSO-$d_6$, 300 MHz) δ 7.78-7.77 (m, 2H), 7.65-7.63 (m, 6H), 7.47-7.44 (m, 2H), 7.37-7.34 (m, 3H), 7.27-7.25 (m, 2H). 6.89 (bs, 2H), 3,72 (s, 3H). MS *m*/*z* 405 $(M+H)^+$. |
| **10c** *N,N*-bis(4-hydroxyphenyl)biphenyl-4-carboxamide | tan solid, 72% yield, M.p. > 250 °C.$^1$H NMR (DMSO-$d_6$, 300 MHz) δ 9.49 (s, 2H), 7.66-7.64 (m, 2H), 7.58-7.56 (m, 6H), 7.46-7.42 (m, 4H), 7.38-7.35 (m, 1H), 7.05-7.03 (m, 4H). 6.69 (bs, 4H). MS *m*/*z* 382 $(M+H)^+$. |
| **10d** *N,N*-bis(4-hydroxyphenyl)-3,4-dimethylbenzamide | tan solid, 68% yield, M.p. > 250 °C. $^1$H NMR (*DMSO-$d_6$*, 300 MHz) δ 9.46 (s, 3H), 7.20 (s, 1H), 7.03-6.95 (m, 6H), 6.68- ' 6.65 (m, 4H), 2.14 (s, 3H), 2.12 (s, 4H). MS *m*/*z* 334 $(M+H)^+$. |
| **10e** *N*-(biphenyl-4-yl)-4-cyano-*N*-(4-hydroxyphenyl)-benzemide | yellow solid, 58% yield, M.p. 223-224 °C. $^1$H NMR (DMSO-$d_6$, 300 MHz) δ 9.63 (s, 3H), 7.78-7.76 (m, 2H), 7.66-7.60 (m, 6H), 7.48-7.43 (m, 2H), 7.38-7.35 (m, 3H), 7.12 (d, 2H, *J* = 8.27 Hz), 6.69 (d, 2H, *J* = 8.27 Hz). MS *m*/*z* 334 $(M+H)^+$. |
| **10f** 3-fluoro-4-hydroxy-*N*-(4-hydroxyphenyl)-*N*-phenylbenzamide | white solid, 66% yield, M.p. 223-225 °C. $^1$H NMR (DMSO-$d_6$, 300 MHz) δ 10.11 (bs, 1H), 9.57 (bs, 1H), 7.34-7.29 (m, 2H), 7.20-7.10 (m, 4H), 7.06-6.97 (m, 3H), 6.81-6.75 (m, 1H), 6.70-6.67 (m, 2H). MS *m*/*z* 324 $(M+H)^+$. |
| **10g** 4-fluoro-3-hydroxy-*N,N*-bis(4-hydroxyphenyl)-benzamide | tan solid, 71% yield, M.p. >250 °C. $^1$H NMR (DMSO-$d_6$, 300 MHz) δ 9.95 (bs, 1H), 9.47 (bs, 2H), 7.02-6.95 (m, 6H), 6.75-6.72 (m, 1H), 6.68-6.66 (m, 4H). MS *m*/*z* 340 $(M+H)^+$. |
| **10i** 4-hydroxy-*N,N*-bis(4-hydroxyphenyl)-3,5-dimethylbenzamide | tan solid, 91% yield, M.p. > 250 °C. $^1$H NMR (DMSO-$d_6$, 300 MHz) δ 9.41 (bs, 2H), 6.96-6.92 (m, 6H), 6.66 (d, 4H, *J* = 8.79 Hz), 2.02 (s, 6H). MS *m*/*z* 350 $(M+H)^+$. |
| **10j** *N,N*-bis(4-hydroxyphenyl)-2,3-dimethylbenzamide | peach-white solid, 68% yield, M.p. >250 °C. $^1$H NMR (DMSO-$d_6$, 300 MHz) δ 9.47 (bs, 2H), 7.18 (d, 2H, *J* = 8.30 Hz), 7.06 (d, 1H, *J* = 7.08 Hz), 7.00-6.92 (m, 4H), 6.78 (d, 2H, *J* = 8.30 Hz), 6.51 (d, 2H, *J* = 8.06 Hz), 2.22 (s, 3H), 2.15 (s, 3H). MS *m*/*z* 334 $(M+H)^+$. |
| **10k** 3-fluoro-4-hydroxy-*N,N*-bis(4-hydroxyphenyl)-benzamide | tan solid, 71% yield, M.p. >250 °C. $^1$H NMR (DMSO-$d_6$, 300 MHz) δ 10-25 (bs, 1H), 9.48 (bs, 2H), 7.12-6.95 (m, 6H), 6.80-6.65 (m, 5H). MS *m*/*z* 338 $(M-H)^-$. |
| **10l** *N,N*-bis(4,hydroxyphenyl)-4-propylbenzamide | tan solid, 77% yield, M.p. 224-225 °C. $^1$H NMR (DMSO-$d_6$, 300 MHz) δ 9.46 (s, 2H), 7.27-7.26 (m 2H), 7.06-7.04 (m, 2H), 6.99-6.97 (m, 4H), 6.66-6.65 (m, 4H), 2.50 (s, 2H), 1.53-1.52 (m, 2H), 0.82 (t, 3H, *J* = 7.33 Hz). MS *m*/*z* 346 $(M-H)^-$. |

(continued)

| Compound # and IUPAC NAME | PHYSICAL CHARACTERIZATION |
|---|---|
| **10m**<br>3,4-dihydroxy-*N,N*-bis(4-hydroxyphenyl)-benzamide hydroxyphenyl)-benzamide | light-pink solid, 66% yield, M.p. >250 °C. $^1$H NMR (DMSO-$d_6$, 300 MHz) δ 9.39 (bs, 4H), 6.91 (d, 2H, *J* = 8.79 Hz), 6.84 (d, 1H, *J* = 1.95 Hz), 6.66 (d, 4H, *J* = 8.55 Hz), 6.62-6.60 (m, 1H), 6.51 (d, 1H, *J* = 8.30 Hz). MS *m/z* 336 $(M-H)^-$. |
| **10n**<br>4-hydroxy-*N,N* bis(4-hydroxyphenyl)-3-methylbenzamide | tan solid, 78% yield, M.p. > 250 °C. $^1$H NMR (DMSO-$d_6$, 300 MHz) δ 9.69 (bs, 1H), 9.44 (bs, 2H), 7.15 (d, 1H, *J* = 1.71 Hz), 6.97 (dd, 2H, *J* = 1.95, 8.30 Hz), 6.93 (d, 4H, *J* = 8.55 Hz), 6.66 (d, 4H, *J* = 8.80 Hz), 6.55 (d, 1H, *J* = 8.55 Hz), 2.50 (s, 3H). MS *m/z* 334 $(M-H)^-$. |
| **10o**<br>N-(4-hydroxyphenyl)-*N*-(4-(2-(piperidin-1-yl)ethoxy) phenyl)-4-propylbenzamide | yellow solid, 39% yield, M.p. 168-171 °C. $^1$H NMR (DMSO-$d_6$, 300 MHz) δ 9.49 (s, 1H), 7.28 (d, 2H, *J* = 7.28 Hz), 7.09 (d, 2H, *J* = 8.06 Hz), 7.06 (d, 2H, *J* = 8.30 Hz), 6.99 (d, 2H, *J* = 8.06 Hz), 6.86 (d, 2H, *J* = 7.82 Hz), 6.66 (d, 2H, *J* = 7.57 $H_2$), 4.00 (bs, 2H), 2.62 (bs, 2H), 2.51-2.50 (m, 2H), 2.40 (bs, 4H), 1.54-1.46 (m, 6H), 1.37-1.36 (m, 2H), 0.82 (t, 3H, *J*=7.33 Hz). MS *m/z* 459 $(M+H)^+$. |
| **10p**<br>*N*-(4-hydroxyphenyl)-2,3-dimethyl-*N*-(4-(2-(piperidin-1-yl)ethoxy)phenyl)-benzamide | tan foam, 32% yield, M.p. 93-96 °C. $^1$H NMR (DMSO-$d_6$, 300 MHz) δ 9.46 (s, 1H), 7.28 (d, 1H, *J* = 7.82 Hz), 7.18 (d, 1H, *J* = 7.08 Hz), 7.08 (d, 2H, *J* = 7.08 Hz), 6.99-6.91 (m, 4H), 6.77 (d, 1H, *J* = 7.33 Hz), 6.70 (d, 1H, *J* = 7.33 Hz), 6.51 (d, 1H, *J* = 7.57 Hz), 4.07 (bs, 1H), 3.91 (bs, 1H), 2.68-2.64 (m, 2H), 2.50-2.35 (m, 4H), 2.22 (s, 3H), 2.14 (s, 3H), 1.50-1.37 (m, 6H). MS *m/z* 445 $(M+H)^+$. |
| **10q**<br>*N,N*-bis(4-hydroxyphenyl)-2,4-dimethylbenzamide | yellow solid, 80% yield, M.p. 227-228 °C. $^1$H NMR (DMSO-$d_6$, 300 MHz) δ 9.45 (bs, 2H), 7.10-7.08 (m, 4H), 6.99-6.83 (m, 3H), 6.81-6.54 (m, 4H), 2.28 (s, 3H), 2.17 (s, 3H). MS *m/z* 334 $(M+H)^+$. |
| **10r**<br>*N,N*-bis(4-hydroxyphenyl)-3,5-dimethylbenzamide | white solid, 61% yield, M.p. > 250 °C. $^1$H NMR (DMSO-$d_6$, 300 MHz) δ 9.45 (bs, 2H), 7.10-7.08 (m, 4H), 6.98-6.83 (m, 3H), 6.81-6.54 (m, 4H), 2.28 (s, 3H), 2.17 (s, 3H). MS *m/z* 334 $(M+H)^+$. |
| **10s**<br>*N,N*-bis(4-hydroxyphenyl)-4-methylbenzamide | tan solid, 32% yield, M.p. 223-225 °C. $^1$H NMR (DMSO-$d_6$, 300 MHz) δ 9.47 (bs, 2H), 7.25 (d, 2H, *J* = 8.04 Hz), 7.05-6.97 (m, 6H), 6.66 (d, 4H, *J* = 8.33 Hz), 2.23 (s, 3H). MS *m/z* 320 $(M+H)^+$. |
| **10t**<br>4,4'-(2,3-dimethyl-benzylazanediyl)diphenol | tan foam, 41% yield, M.p. 147-150 °C. $^1$H NMR (DMSO-$d_6$, 300 MHz) δ 8.92 (s, 2H), 7.07 (d, 1H, *J* = 7.33 Hz), 1), 7.00-6.94 (m, 2H), 6.76-6.72 (m, 4H), 6.63-6.59 (m, 4H), 4.72 (s. 2H), 2.23 (s, 3H), 2.16 (s, 3H). MS *m/z* 320 $(M+H)^+$. |
| **10u**<br>4-formyl-*N,N*-bis(4-hydroxyphenyl)-benzamide | yellow foam, 50% yield, M.p. 117-122 °C. $^1$H NMR (DMSO-$d_6$, 300 MHz) δ 9.98 (s, 1H), 9.52 (s, 2H), 7.78 (d, 2H, *J* = 8.13 Hz), 7.61 (d, 2H, *J* = 8.13 Hz), 7.06 (bs, 4H), 6.67 (bs, 4H). MS *m/z* 332 $(M-H)^-$. |
| **11l**<br>4-((hydroxyimino)methyl)-*N,N*-bis(4-hydroxyphenyl) benzamide | yellow solid, 67% yield, M.p. 146-148 °C. $^1$H NMR (DMSO-$d_6$, 300 MHz) δ 11.36 (s, 1H), 9.49 (s, 2H), 8.07 (s, 1H), 7.45 (d, 2H, J = 8.28 Hz), 7.37 (d, 1H, J = 8.28 Hz), 7.01 (d, 4H, J = 7.52 Hz), 6.67 (d, 4H, J = 6.45 Hz). MS *m/z* 349 $(M+H)^+$. |

(continued)

| Compound # and IUPAC NAME | PHYSICAL CHARACTERIZATION |
|---|---|
| **11m**<br>*N*-(4-hydroxyphenyl)-2,4-dimethyl-*N*-(4-(2-(piperidin-1-yl)ethoxy)phenyl)benzamide | pale-yellow foam, 26% yield, M.p. 92-95 °C. $^1H$ NMR (DMSO-$d_6$, 300 MHz) δ 9.48 (s, 1H), 7.13-6.81 (m, 11H), 3.33 (bs, 2H), 2.65 (bs, 2H), 2.51-2.45 (m, 4H), 2.29 (s, 3H), 2.17 (s, 3H), 1.49 (bs, 4H), 1.38 (bs, 2H). MS *m*/*z* 446 $(M+H)^+$. |
| **11n**<br>*N*-(4-hydroxyphenyl)-3,5-dimethyl-*N*-(4-(2-(piperidin-1-yl)ethoxy)phenyl)benzamide | pale-yellow foam, 26% yield, M.p. 94-100 °C. $^1H$ NMR (DMSO-$d_6$, 300 MHz) δ 9.50 (s, 1H), 7.11 (d, 2H, *J* = 8.67 δ 9.50 (s, 1H), 7.11 Hz), 7.02-6.98 (m, 3H), 6.91-6.86 (m, 3H), 6.67 (d, 2H, *J* = 8.46Hz), 4.03 (t, 2H, *J* = 5.10 Hz), 2.67 (bs, 2H), 2.51-2.46 8.46 Hz), 4.03 (t, 2H, *J* = 5.10 Hz), 2.67 (bs, 2H), 2.51-2.46 (m, 4H), 2.15 (s, 6H), 1.50-1.49 (m, 4H), 1.39-1.37 (m, 2H). MS *m*/*z* 445. |
| **11o**<br>4-((2,3-dimethylbenzyl)(4-(2-(piperidin-1-yl)ethoxy)phenyl)amino)phenol | purple foam, 38% yield, M.p. 65-70 °C. $^1H$ NMR (DMSO-$d_6$, 300 MHz) δ 9.08 (s, 1H), 7.08-6.86 (m, 5H), 6.78-6.65 (m, 6H), 4.75 (s, 2H), 3.96 (t, 2H, *J* = 5.83 Hz), 3.34 (bs, 2H), 2.65 (bs, 2H), 2.51 (bs, 2H), 2.27 (s, 3H), 2.16 (s, 3H), 1.51-1.48 (m, 4H), 1.38-1.37 (m, 2H). MS *m*/*z* 432. |
| **11p**<br>*N*,*N*-bis(4-hydroxyphenyl)-4-pentylbenzamide | white solid, 68% yield, M.p. 201-202 °C. $^1H$ NMR (DMSO-$d_6$, 300 MHz) δ 9.46 (s, 2H), 7.26 (d, 2H, J = 8.10 Hz), 7.05 (d, 2H, J = 8.10Hz), 6.98 (d, 4H, J = 8.42 Hz), 6.66 (d, 4H, J = 8.28 Hz), 2.52-2.47 (m, 2H), 1.52-1.48 (m, 2H), 1.28-1.21 (m, 4H), 0.83 (t, 3H, J = 7.00 Hz). MS *m*/*z* 376. |
| **11q**<br>*N*-(4-hydroxyphenyl)-4-pentyl-*N*-(4-(2-(piperidin-1-yl)ethoxy)phenyl)benzamide | tan solid, 31% yield, M.p. 172-174 °C- $^1H$ NMR (DMSO-$d_6$, 300 MHz) δ 9.49 (s, 1H), 7.27 (d, 2H, J = 8.06 Hz), 7.09 (d, 2H, J = 7.82 Hz), 7.05 (d, 2H, J = 8.30 Hz), 6.99 (d, 2H, J = 7.82 Hz), 6.86 (d, 2H, J = 7.33 Hz), 6.66 (d, 2H, J = 7.33 Hz), 4.01 (bs, 2H), 3.33 (bs, 4H), 2.64-2.63 (m, 2H), 2.51-2.36 (m, 2H), 1.53-1.51 (m, 6H), 1.37 (bs, 2H), 1.27-1.23 (m, 4H), 0.83 (t, 3H, J = 7.20 Hz). MS *m*/*z* 488 $(M+H)^+$. |
| **11r**<br>4-*terl*-butyl-*N*,*N*-bis(4-hydroxyphenyl)benzamide | tan solid, 80% yield, M.p. > 250 °C. $^1H$ NMR (DMSO-$d_6$, 300 MHz) δ 9.48 (bs, 2H), 7.32-7.24 (m, 4H), 6.99 (d, 4H, J = 8.56 Hz), 6.67 (d, 4H, J = 8.44 Hz), 1.21 (s, 9H). MS *m*/*z* 363. |
| **11s**<br>4-*tert*-butyl-*N*-(4-hydroxyphenyl)-*N*-(4-(2-(piperidin-1-yl)ethoxy)phenyl)benzamide | tan solid, 39% yield, M.p. 208-210 °C. $^1H$ NMR (DMSO-$d_6$, 300 MHz) δ 9.50 (s, 1H), 7.34-7.25 (m, 4H), 7.10 (d, 2H, J = 8.71 Hz), 6.99 (d, 2H, J = 8.61 Hz), 6.87 (d, 2H, J = 8.71 Hz), 6.67 (d, 2H, J = 8.61 Hz), 4.02 (t, 2H, J = 5.70 Hz), 3.33 (bs, 2H), 2.64 (bs, 2H), 2.43 (bs 2H), 1.50-1.47 (m, 4H), 1.38-1.36 (m, 2H), 1.21 (s, 9H). MS *m*/*z* 473 $(M+H)^+$. |
| **11t**<br>3-{4-[*Bis*-(4-hydroxy-phenyl)-carbamoyl]-phenyl)-acrylic acid | yellow foam, M.p. 129-131 °C . $^1H$ NMR ((DMSO-$d_6$, 300 MHz) δ 9.49 (s, 2H), 7.61-7.56 (m, 3H), 7.39 (d, *J* = 8.26 Hz, 2H), 7.04-7.02 (m, 4H), 6.66-6.61 (m, 5H), 3.71 (s, 3H), 1.53 -1.52 (m, 2H, $CH_2$), 0.82, t*J* = 7.33 Hz, 3H, $CH_3$). MS *m*/*z* 388.1 $(M-H)^-$. |

(continued)

| Compound # and IUPAC NAME | PHYSICAL CHARACTERIZATION |
|---|---|
| **11u**<br>3-{4-[Bis-(4-hydroxy-phenyl)-carbamoyl]-phenyl}-propionic acid | pale-yellow foam, M.p. 122-124 °C. $^1$H NMR (DMSO-$d_6$, 300 MHz) δ9.51 (bs, 1H), 7.29 (d, *J* = 8.23 Hz, 2H), 7.13-7.08 (m, 4H), 6.99 (d, *J* = 8.60 Hz, 2H), 6.85 (d, *J* = 8.60 Hz, 2H), 6.65 (d, *J* = 8.23 Hz, 2H), 3.99 (q, *J* = 7.10, 14.21 Hz, 2H), 3.71 (s, 3H), 2.78 (t, *J* = 7:44 Hz, 2H), 2.56 (t, *J* = 7.44 Hz, 2H), 1.11 t, *J* = 7.06 Hz, 3H, $CH_3$). MS *m/z* 418.1 $(M-H)^-$. |
| **11v**<br>*N*,*N*-Bis-(4-hydroxy-phenyl)-4-(3-hydroxy-propyl)-benzamide | pale-yellow foam, M.p. 122-124 °C. $^1$H NMR ((DMSO-$d_6$, 300 MHz) δ 9.51 (bs, 1H), 7.29 (d, *J* = 8.23 Hz, 2H), 7.13-7.08 (m, 4H), 6.99 (d, *J* = 8.60 Hz, 2H), 6.85 (d, *J* = 8.60 Hz, 2H), 6.65 (d, *J* = 8.23 Hz, 2H), 3.99 (q, *J* = 7.10, 14.21 Hz, 2H), 3.71 (s, 3H), 2.78 (t, *J* = 7.44 Hz, 2H), 2.56 (t, *J* = 7.44 Hz, 2H), 1.11 t *J* = 7.06 Hz, 3H $CH_3$). MS *m/z* 418.1 $(M-H)^-$. |
| **6a**<br>2-(*N*-(4-methoxyphenyl)-4-methylphenylsulfonamido) ethyl 4-methylbenzenesulfonate | white solid. 49% yield. $^1$H NMR (DMSO-$d_6$, 500 MHz) δ 7.69-7.68 (m, 2H), 7.46-7.45 (m, 2H), 7.41-7.40 (m, 2H), 7.37-7.35 (m, 2H), 6.79-6.77 (m, 2H), 6.72-6.70 (m, 2H), 3.91 (t, *J* = 5.0Hz, 2H), 3.77 (t, *J* = 5.0Hz, 2H), 3.72 (s, 3H), 2.43 (s, 3H), 2.39 (s, 3H). MS *m/z* 498 $(M+Na)^+$. |
| **6b**<br>(*R*)-3-bromo-2-hydroxy-*N*-(4-methoxyphenyl)-2-methylpropanamide | white solid, 63% yield. M.p. 79.0-81.0 °C. $^1$H NMR (DMSO-$d_6$, 500 MHz) δ 9.47 (s, 1H), 7.65 (d, 2H, *J* = 9.0 Hz), 6.89 (d, 2H, *J* = 9.0Hz), 6.12 (s, 1H), 3.81 (d, 1H, *J* = 10.2 Hz), 3.71 (s, 3H), 3.56 (d, 1H, *J* = 10.2 Hz), 1.45 (s, 3H). MS *m/z* 288 $(M+H)^+$. |
| | |
| **6c**<br>(*S*)-2-hydroxy-N,3-bis(4-methoxyphenyl)-2-methylpropanamide | colorless oil, 39% yield. $^1$H NMR ($CDCl_3$, 300 MHz) δ 8.34 (s, 1H), 7.40 (d, 2H, *J* = 9.0 Hz), 7.16 (d, 2H, *J* = 8.4 Hz), 6.86-6.83 (m, 4H), 3.79 (s, 3H), 3.78 (s, 3H), 3.38 (d, 1H, *J* = 13.6Hz), 2.80 (d, 1H, *J* = 13.9 Hz), 1.53 (s, 3H). MS *m/z* 338 $(M+Na)^+$. |
| **6d**<br>(*S*)-2-hydroxy-3-(4-methoxyphenoxy)-*N*-(4-methoxyphenyl)-2-methylpropanamide | white solid, 99% yield. M.p. 101.0-102.0 °C. $^1$H NMR ($CDCl_3$, 300 MHz) δ 8.64 (s, 1H), 7.49 (d, 2H, *J* = 8.9 Hz), 6.89-6.79 (m, 6H), 4.35 (d, 1H, *J* = 8.9 Hz), 3.93 (d, 1H, *J* = . 8.9 Hz), 3.79 (s, 3H), 3.76 (s, 3H), 1.56 (s, 3H). MS *m/z* 254 $(M+Na)^+$. |
| **6e**<br>(*R*)-3-bromo-2-hydroxy-*N*-(4-hydroxyphenyl)-2-methylpropanamide | colorless oil, 98% yield. $^1$H NMR (DMSO-$d_6$, 500 MHz) δ 9.33(s, 1H), 9.21(s, 1H), 7.47(d, 2H, *J* = 8.9 Hz), 6.68(d, 2H, *J* = 9.0 Hz), 6.08(s, 1H), 3.80(d, 1H, *J* = 10.3 Hz), 3.55(d, 1H, *J* = 10.5 Hz), 1.44(s, 3H). MS *m/z* 297 $(M+Na)^+$. |
| **6f**<br>(*S*)-2-hydroxy-3-(4-hydroxyphenoxy)-*N*-(4-hydroxyphenyl)-2-methylpropanamide | colorless oil, 67% yield. $^1$H NMR (DMSO-$d_6$, 500 MHz) δ 9.35 (s, 1H), 9.19 (s, 1H), 8.90 (s, 1H), 7.48 (d, 2H, *J* = 8.8 Hz), 6.73 (d, 2H, *J* = 9.0 Hz), 6.68 (d, 2H, *J* = 9.0 Hz), 6.63 (d, 2H, *J* = 9.0Hz), 5.89 (s, 1H), 4.06 (d, 1H, *J* = 9.5 Hz), 3.81 (d, 1H, *J* = 9.3 Hz), 1.36 (s, 3H). MS *m/z* 326 $(M+Na)^+$. |

(continued)

| Compound # and IUPAC NAME | PHYSICAL CHARACTERIZATION |
|---|---|
| **6g**<br>(*S*)-2-hydroxy-*N*,3-bis(4-hydroxyphenyl)-2-methylpropanamide | colorless oil, 65% yield. $^1$H NMR (DMSO-$d_6$, 300 MHz) δ 9.15 (s, 1H), 9.08 (s, 1H), 9.03 (s, 1H), 7.34 (d, 2H, *J* = 9.0 Hz), 6.97 (d, 2H, *J* = 8.4 Hz), 6.64 (d, 2H, J= 8.9 Hz), 6.58 (d, 2H, *J* = 8.4 Hz), 5.50 (s, 1H), 2.90 (d, 1H, *J* = 13.5 Hz), 2.68 (d, 1H, *J* = 13.5 Hz), 1.29 (s, 3H). MS *m*/*z* 310 (M+Na)$^+$. |
| **2a**<br>4-Methoxy-*N*,*N*-bis-(4-methoxyphenyl)-benzamide | white solid, 98% yield. M.p. 119.5-120 °C. $^1$H NMR ($CDCl_3$, 300 MHz) δ 7.42 (d, 2H, *J* = 8.9Hz), 7.05 (d, 4H, *J* 8.8 Hz), 6.81 (d, 4H, *J* = 8.9 Hz), 6.71 (d, 2H, *J* = 8.9Hz), 3.77 (s, 9H). MS *m*/*z* 364(M+H). |
| **3a**<br>4-Hydroxy-*N*,*N*-bis-(4-hydroxyphenyl)-benzamide | white solid, 79% yield. M.p. 275-276 °C (decomposed). $^1$H NMR (DMSO-$d_6$, 300 MHz) δ 9.79 (s, 1H), 9.44 (s, 2H), 7.21 (d, 2H, *J* = 9.0 Hz), 6.93(d, 4H, *J* = 8.7Hz), 6.66 (d, 4H, *J* = 8.7Hz), 6.58 (d, 2H, *J* = 9.0Hz). MS *m*/*z* 344 (M+Na)$^+$. |
| **2d**<br>*N*,*N*-Bis-(4-methoxyphenyl)-benzamide | white solid, 98% yield. M.p. 77-77.5 °C. $^1$H NMR ($CDCl_3$, 300 MHz) δ 7.46-7.42 (m, 2H), 7.29-7.17 (m, 3H), 7.09-7.06 (m, 4H), 6.81-6.78 (m, 4H), 3.76 (s, 6H). MS *m*/*z* 356 (M+Na)$^+$. |
| **3d**<br>*N*,*N*-Bis-(4-hydroxyphenyl)-benzamide | white solid, 98% yield. M.p. >265 °C (decomposed). $^1$H NMR (DMSO-$d_6$, 300 MHz) δ 9.48 (s, 2H), 7.37-7.20 (m, 5H), 7.01 (d, 4H, *J* = 8.9Hz), 6.66 (d, 4H, *J* = 7.9Hz), 6.58 (d, 2H, *J* = 7.3Hz). MS *m*/*e* 304 (M-H)$^-$. |
| **2g**<br>*N*,*N*-Diphenyl-benzamide | white solid, 89% yield. M.p. 178.4-179.3 °C. $^1$H NMR ($CDCl_3$, 300 MHz) δ 7.46-7.44 (m, 2H), 7.28-7.23 (m, 5H), 7.21-7.14 (m, 8H). MS *m*/*z* 296 (M+Na)$^+$. |
| **3e**<br>4-Hydroxy-*N*,*N*-diphenyl-benzamide | white solid, 57% yield. M.p. 193.7.0-194.3 °C. $^1$H NMR (DMSO-$d_6$, 300 MHz) δ 9.89 (s, 1H), 7.35-7.13 (m, 12H), 6.59 (d, 2H, *J* = 8,6Hz). MS m/z 312 (M+Na)$^+$. |
| **2i**<br>*N*-(3-methoxyphenyl)-*N*-phenyl-benzamide | white solid, 93% yield. M. p. 103-105.9°C. $^1$H NMR ($CDCl_3$, 300 MHz) δ 7.49-7.45 (m, 2H), 7.31-7.15 (m, 9H), 6.75-6.70 (m, 3H), 3.76 (s, 3H). MS m/z 326 (M+Na)$^+$. |
| **3h**<br>*N*-(3-Hydroxyphenyl)-*N*-phenyl-benzamide | white solid, 56% yield. M.p. 199.0-202.0 °C. $^1$H NMR (DMSO-d6, 300 MHz) δ 9.55 (s, 1H), 7.44-7.06 (m, 12H), 6.62-6.59 (m, 2H). MS *m*/*z* 312 (M+Na)$^+$. |
| **2j**<br>4-Methoxy-*N*-(4-methoxypheny)-*N*-phenyl-benzamide | white solid, 78% yield. $^1$H NMR ($CDCl_3$, 300 MHz) δ 7.44-7.41 (m, 2H), 7.29-7.26 (m, 2H), 7.15-7.05 (m, 5H), 6.83-6.80 (m, 2H), 6.72-6.7.0 (m, 2H), 3.77 (s, 6H). MS m/z 356 (M+Na)$^+$. |
| **3i**<br>4-Hydroxy-*N*(4-hydroxyphenyl)-*N*-phenyl-benzamide | white solid, 55% yield. M.p. 240.0-243.0 °C (decomposed). $^1$H NMR (DMSO-$d_6$, 300 MHz) δ 9.84 (s, 1H), 9.51 (s, 1H), 7.33-7.11 (m, 7H), 6.97-6.94 (m, 2H), 6.69-6.67 (m, 2H), 7.33-7.11 6.61-6.58 (m,2H). MS *m*/*e* 304 (M-H)$^-$. (m, 2H). MS *m*/*e* 304 (M-H)$^-$. |
| **2h**<br>*N*-(4-methoxyphenyl)-*N*-phenyl-benzamide | white solid, 95% yield. M. p. 153-154.2°C. $^1$H NMR ($CDCl_3$, 300 MHz) δ 7.47-7.43 (m, 2H), 7.30-7.02 (m, 8H), 6.83-6.78 (m, 2H), 3.76 (s, 3H). MS m/z 326 (M+Na)$^+$. |

(continued)

| Compound # and IUPAC NAME | PHYSICAL CHARACTERIZATION |
|---|---|
| **2k**<br>4-Methoxy-*N*-(3-methoxyphenyl)-*N*-phenyl-benzamide | white solid, 84% yield. M.p. 119.0-119.5 °C. $^1$H NMR. ($CDCl_3$, 300 MHz) δ 7.47-7.43 (m, 2H), 7.31-7.13 (m, 7H), 6.75-6.68 (m, 4H), 3.77 (s, 3H), 3.71 (s, 3H). MS m/z 356 $(M+Na)^+$. |
| **3g**<br>*N*-(4-Hydroxyphenyl)-*N*-phenyl-benzamide; | white solid, 70% yield. M.p. 255.0-256.0 °C. $^1$H NMR (DMSO-$d_6$, 300 MHz) δ 9.53 (*s*, 1H), 7.40-7.15 (m, 10H), 7.02(d, 2H, *J* = 8.7Hz), 6.67 (d, 2H, *J* = 8.7Hz). MS *m/z* 312 $(M+Na)^+$. |
| **3j**<br>4-Hydroxy-*N*-(3-hydroxyphenyl)-*N*-phenyl-benzamide; | white solid, 73% yield. M.p. 245.-247.5°C (decomposed). $^1$H NMR (DMSO-$d_6$, 300 MHz) δ 9.90 (s, 1H), 9.53 (s, 1H), 7.35-7.06 (m, 8H), 6.63-6.52 (m, 5H). MS *m*/*e* 304 $(M-H)^-$. |
| **4a**<br>*N*-(4-Hydroxyphenyl)-*N*-[4-(2-piperidin-1-ylethoxy)-phenyl]-benzamide | yellow solid. 45% yield. M.p. 164.5-165.0 °C. $^1$H NMR (DMSO-$d_6$, 300 MHz) δ 9.49 (s, $^1$H), 7.38-7.36 (m, 2H), 7.26-7.24 (m, 3H), 7,12 (d, 2H, *J* = 8.5Hz), 7.01 (d, 2H, *J* = 8.5Hz), 6.87 (d, 2H, *J* = 8.3Hz), 6.65 (d, 2H, *J* = 83Hz), 4.01 (t, 2H, *J* = 5.1Hz), 2.63 (br, 2H), 2.50-2.43 (m, 4H), 1.48 (br, 4H), 1.38-1.36 (m, 2H). MS *m/z* 417 $(M+H)^+$. |
| **3b**<br>3-Hydroxy-*N*-bis-(4-hydroxyphenyl)-benzamide | white solid, 92% yield. M.p. 257.0-259-0 °C. $^1$H NMR (DMSO-$d_6$, 300 MHz) δ 9.49 (s, 1H), 9.47 (s, 2H), 7.02-6.98 (m, 5H), 6.80-6.65 (m, 7H). MS *m*/*e* 320 $(M-H)^-$. |
| **3k**<br>*N*,*N*-Bis(4-hydroxyphenyl)-4-fluoro-benzamide | off-white solid, 87% yield. M.p. 270.0-271.0 °C. $^1$H NMR (DMSO-$d_6$, 300 MHz) δ 9.90 (s, 1H), 9.53 (s, 1H), 7.35-7.06 (m, 8H), 6.63-6.52 (m, 5H). MS *m*/*e* 304 $(M-H)^-$. |
| **3f**<br>3-Hydroxy-*N*,*N*-diphenyl-benzamide | white solid, 85% yield. M.p. 152.5-153.2 °C. $^1$H NMR (DMSO-$d_6$, 300 MHz) δ 9.89 (s, 1H), 7.35.7.13 (m, 12H), 6.59 (d, 2H, *J* = 8.6Hz). MS *m/z* 312 $(M+Na)^+$. |
| **3c**<br>4-Hydroxy-*N*-(4-hydroxyphenyl)-*N*-(3-hydroxyphenyl)-benzamide | white solid, 92% yield. M.p. 249.1 °C (decomposed). $^1$H NMR (DMSO-$d_6$, 300 MHz) δ 9.79 (s, 1H), 9.44 (s, 2H), 7.21 (d, 2H, *J* = 9.0 Hz), 6.93 (d, 4H, *J* = 8.7Hz), 6.66 (d, 4H, *J* = 8.7Hz), 6.58 (d, 2H, *J* = 9.0Hz). MS m/z 344 (M+Na). |
| **4c**<br>*N*,*N*-diphenyl-[3-(2-piperidinyl-ethoxy)]-benzamide hydrochloride | yellow solid, 57% yield. M.p. 149.5 - 150.0 °C. $^1$H NMR (DMSO-$d_6$, 300 MHz), δ 10-76 (*s*, 1H), 7.33-6.92 (m, 14H), 4.32 (s, 2H), 3.42-3.40 (m, 4H), 2.44-2.92 (m, 2H), 1.78-1.67 (m, 5H), 1.38 (br, 1H). MS m/z 401 $(M+H)^+$. |
| **4d**<br>*N*,*N*-diphenyl-[3-(2-piperidinyl-ethoxy)]-benzamide hydrochloride | yellow solid, 50% yield. M.p. 148.5-149.5 °C. $^1$H NMR (DMSO-$d_6$, 300 MHz) δ 10.67 (s, 1H), 7.40-6.85 (m, 14H), 4.38 (s, 2H), 3.48-3.41 (m, 4H), 2.96-2.94 (m, 2H), 1.77-1,66 (in, 5H), 1.34 (br, 1H). MS *m/z* 401 $(M+H)^+$. |
| **3l**<br>4-Hydroxy-*N*,*N*-diphenyl-phenyl-sulfonamide | white solid, 86% yield. M.p. 158.0-158.8 °C. $^1$H NMR ($CDCl_3$, 300 MHz) δ 10.61 (s, 1H), 7.52-7.47 (m, 2H), 7.39-7.25 (m, 10H), 6.93-6.89 (m, 2H). MS *m/z* 324 $(M-H)^-$. |
| **4e**<br>*N*-(4-Hydroxyphenyl)-*N*-phenyl-[4-(2-piperidin-1-ylethoxy)]-benzamide hydrochloride | yellow solid, 38% yield. M.p. 183.7-185.0 °C (decomposed). $^1$H NMR (DMSO-$d_6$, 300 MHz) δ 10.76 (s, 1H), 9.66 (s, 1H), 7.35-6.61 (m, 13H), 4.37 (m, 2H), 3.40 (m, 4H), 2.94 (m, 2H), 1.76-1.65 (m, 5H), 1.34 (m, 1H). MS m/z 417 $(M+H)^+$. |

(continued)

| Compound # and IUPAC NAME | PHYSICAL CHARACTERIZATION |
|---|---|
| **4u**<br>*N*-[4-(2-piperidin-1-ylethoxy)-phenyl]-*N*-phenyl-[4-(2-piperidin-1-ylethoxy)]-benzamide dihydrochloride | yellow solid, 27% yield. M.p. 210.9-212.0 °C (decomposed). $^1H$ NMR (DMSO-$d_6$, 300 MHz) δ 11.07 (s, 2H), 7.35-6.84 (m, 13H), 4.38 (m, 4H), 3.40 (br, 8H), 2.95 (m, 4H), 2.05-1.65 (m, 10H), 1.34 (m, 2H). MS m/z 528 $(M+H)^+$. |
| **4b**<br>*N*-(phenyl)-*N*-[4-(2-piperidin-1-ylethoxy)-phenyl]-benzamide hydrochloride | yellow solid, 89% yield. M.p. 138.3-139.5 °C. $^1H$ NMR (DMSO-$d_6$ 300 MHz) δ 9.52 (s, 1H), 7.42-7.39 (m, 2H), 7.33-7.16 (m, 10H), 6.89-6.91 (m, 2H), 4.17 (s, 2H), 2.76 (s, 2H), 2.51-2.49 (m, 4H), 1.61 (br, 4H), 1.43 (br, 2H). MS *m*/*z* 401 $(M+H)^+$. |
| **3m**<br>4-Hydroxy-*N*-(4-hydroxyphenyl)-*N*-(fluorophenyl)-benzamide | white solid, 90% yield. M.p. 246.3,247.0 °C. $^1H$ NMR (DMSO-$d_6$, 300 MHz) δ 9.84 (s, 1H), 9.53 (s, 1H), 7.24-7.13 (m, 6H), 6.98-6,95 (m, 2H), 6.69-6.67 (m, 2H), 6.69-6.66 (m, 2H), 6.61-6.58 (m, 2H). MS m/z 324 $(M+H)^+$. |
| **4f**<br>*N*,*N*-diphenyl-bis[4-(2-piperidin-1-ylethoxy)-phenyl]-sulfonamide hydrochloride | pale-yellow solid. 79% yield. M.p. 211.6-212.5 °C. $^1H$ NMR (DMSO-$d_6$, 300 MHz) δ 10.78 (s, 1H), 7.65-7.17 (m, 14H), 4.52 (m, 2H), 3.36-3.4,7 (m, 4H), 3.00 (br, 2H), 1.67-2.50 (m, 5H), 1.38 (m, 1H). MS *m*/*z* 437 $(M+H)^+$. |
| **4g**<br>*N*-(4-Fluorophenyl)-*N*-[4-hydroxyphonyl]-[4-(2-piperidin-1-ylethoxy)]-benzamide | pale-yellow solid, 45% yield. M.p. 168.3-169.0 °C. $^1H$ NMR (DMSO-$d_6$, 500 MHz) δ 10.61 (s, 1H), 9.65 (s, 1H), 7.38-6.69 (m, 12H), 4.38 (m, 2H), 3.46-3.36 (m, 4H), 2.96 (m, 2H), 2.04-1.66 (m, 5H), 1.35 (br, 1H). MS m/z 435 $(M+H)^+$. |
| **4r**<br>*N*-(4-Fluorophenyl)-*N*-[4-(2-piperidin-1-ylethoxy)-phenyl]-[4-(2-piperidin-1-yl-ethoxy)]-benzamide dihydrochloride | yellow solid, 95% yield. M.p. 253.5-254.0 °C (decomposed). $^1H$ NMR (DMSO-$d_6$, 300 MHz) δ 11.03 (s, 2H), 7.42-7.39 (m, 2H), 7.28-7.26 (m, 3H), 7.20-7.17 (m, 4H), 6.94-6.92 (m, 4H), 4.39 (br, 4H), 3.46-3.42 (m, 8H), 3.01-2.94 (m, 4H), 1.85-1.65 (m, 10H), 1.38-1.34 (m, 2H). MS *m*/*z* 528 $(M+H)^+$. |
| **4h**<br>*N*-(4-Hydroxyphenyl)-*N*-[4-(2-piperidin-1-ylethoxy)-phenyl]-4-fluoro-benzamide hydrochloride | yellow solid, 42% yield. M.p. 234.0-235.8 °C (decomposed). $^1H$ NMR (DMSO-$d_6$, 300 MHz) δ 10.85 (s, 1H), 9.65 (s, 1H), 7.42-6.68 (m, 12H), 4.38 (m, 2H), 3.40 (m, 4H), 2.95 (m, 2H), 2.06-1.77 (m, 5H), 1.35 (m, 1H). MS m/z 435 $(M+H)^+$. |
| **4s**<br>*N*,*N*-Bis-[4-(2-piperidin-1-ylethoxy)-phenyl]-4-fuoro-benzamide dihydrochloride; | yellow solid, 20% yield. M.p. 204.8-205.5 °C (decomposed). $^1H$ NMR (DMSO-$d_6$, 300 MHz) δ 11.01 (s, 2H), 7.45-6.92 (m, 12H), 4.38 (m, 4H), 3.38 (m, 8H), 2.95 (m, 4H), 2.06-1.67 (m, 10H), 1.35 (m, 2H). MS m/z 546 $(M+H)^+$. |
| **3n**<br>*N*,*N*-Bis(4-hydroxyphenyl)-1-naphthylamide; (3n) | white solid, 86% yield. M.p. 215.7 °C (decomposed). $^1H$ NMR (DMSO-$d_6$, 500 MHz) δ 9.54 (s, IH), 9.35 (s, IH), 8.11 (d, 1H, *J* = 9.0Hz), 8.87(d, 1H, *J* = 8.0Hz), 7.79 (d, 1H, *J* = 8.5Hz), 7.61-7.58 (m, 1H), 7.53-7.48 (m, 2H), 7.37-7.34 (m, 1H), 7.30 (s, 2H), 7.00 (s, 2H), 6.83 (s, 2H), 6.38 (s, 2H). MS m/z 356 $(M+H)^+$. |
| **4t**<br>*N*,*N*-Bis[4-(2-piperidin-1-ylethoxy)-phenyl-benzamide dihydrochloride: (4t) | yellow solid, 28% yield. M.p. 218.6-219.5 °C (decomposed). $^1H$ NMR (DMSO-$d_6$, 300 MHz) δ 11.09 (s, 2H), 7.38-6.86 (m, 12H), 4.40 (m, 4H), 3.39 (m, 8H), 2.96 (m, 4H), 2.07-1.66 (m, 10H), 1.35 (m, 2H). MS m/z 546 $(M+H)^+$. |

(continued)

| Compound # and IUPAC NAME | PHYSICAL CHARACTERIZATION |
|---|---|
| **3o**<br>4-Hydroxy-*N*-(1-Naphthyl)-*N*-(4-hydroxyphenyl)-benzamide | white solid, 84% yield. M.p. >300 °C (decomposed). $^1H$ NMR (DMSO-$d_6$, 300 MHz) δ 9.84 (s,1H), 9.47 (s, 1H), 8.07 (d, 1H, *J* = 7.8Hz), 7.97 (d, 1H, *J* = 7.8Hz), 7.86 (d, 1H, *J* = 8.1Hz), 7.58-7.45 (m, 3H), 7.39-7.30 (m, 3H), 7.02 (d, 2H, *J* = 8.1 Hz), 6.66-6.56 (m, 4H). MS m/e 354 $(M-H)^-$. |
| **5a**<br>4-Chloro-*N*-[4-hydroxyphenyl]-*N*-(4-methoxyphenyl)-benzamide | white solid. 93% yield. M.p. 237.5-238.9 °C. $^1H$ NMR (DMSO-$d_6$, 300 MHz) δ 9.53 (s, 1H), 7.41-7.38 (m, 2H), 7.34-7.31 (m, 2H), 7.17-7.14 (m, 2H), 7.05-7.02 (m, 2H), 6.88-6.86 (m, 2H), 6.70-6.66 (m, 2H), 3.71 (s, 3H). MS *m*/*z* 354 $(M+H)^+$. |
| **5b**<br>4-Cyano-*N*-[4-hydroxyphenyl]-*N*-(4-methoxyphenyl)-benzamide | white solid. 71 % yield. M.p. 182-182.9 °C. $^1H$ NMR (DMSO-$d_6$, 300 MHz) δ 9.56 (s, 1H), 7.76-7.71 (m, 2H), 7.56-7.55 (m, 2H), 7.20 (br, 2H), 7.07 (br, 2H), 6.87 (br, 2H), 6.67 (br, 2H), 3.71 (s, 3H). MS *m*/*z* 345 $(M+H)^+$. |
| **4j**<br>4-Cyano-*N*-[4-(2-piperidin-1-ylethoxy)-phenyl]-*N*-(4-methoxyphenyl)-benzamide | colorless oil, 61% yield. M.p. C. $^1H$ NMR (DMSO-$d_6$, 300 MHz) δ 7.42-7.40 (m, 2H), 7.34-7.31 (m, 2H), 7.18-7.13 (m, 4H), 6.88-6.86 (m, 4H), 4.00 (tr, 2H, *J* = 5.7 Hz), 3.71 (s, 3H). 2.60 (tr, 2H, *J* = 5.7 Hz), 2.40 (br, 4H), 1.47-1.45 (m, 4H), 1.37-1.36 (m, 2H). MS *m*/*z* 465 $(M+H)^+$. |
| **5c**<br>3-Chloro-*N*-[4-hydroxyphenyl]-*N*-(4-methoxyphenyl)-benzamide | white solid. 74% yield. M.p. C. $^1H$ NMR (DMSO-$d_6$, 300 MHz) δ 9.53 (s, 1H), 7.45-7.44 (m, 1H), 7.36-7.24 (m, 3H), 7.20-7.18 (m, 2H), 7.08-7.05 (m, 2H), 6.89 (br, 2H), 6.69 (bar, 2H), 3.71 (s, 3H). Ms *m*/*z* 354 $(M+H)^+$. |
| **4i**<br>3-(2-piperidin-1-ylethoxy)-*N*,*N*-bis(4-hydroxyphenyl)-benzamide | yellow solid. 47% yield. M.p. 293.9-295.0 °C (decomposed). $^1H$ NMR (DMSO-$d_6$, 300 MHz) δ 10.51 (s, 2H), 8.18-7.68 (m, 12H), 4.00 (tr, 2H, *J* = 5.5 Hz), 2.62 (m, 2H), 2.41 (m, 4H), 1.50-1.46 (m, 4H), 1.37-1.35 (m, 2H). MS *m*/*e* 431 $(M-H)^-$. |
| **4k**<br>4-Chloro-*N*-[4-(2-piperidin-1-ylethoxy)-phenyl]-*N*-(4-methoxyphenyl)-benzamide | white solid, 45% yield. M.p. 285.0-287.0 °C (decomposed). $^1H$ NMR (DMSO-$d_6$, 300 MHz), δ 9.55 (s, 1H),7.40-7.39 (m, 2H), 7.34-7.32 (m, 2H), 7.20 (br, 2H), 7.02 (br, 2H), 6.95 (br, 2H), 6.68-6.67 (m, 2H), 4.28 (m, 2H), 3.34 (br, 4H), 2.99 (m, 2H), 1.72 (br, 4H), 1.46 (br, 2H). MS *m*/*z* 451 $(M+H)^+$. |
| **4l**<br>4-Cyano-*N*-[4-(2-piperidin-1-ylethoxy)-phenyl]-*N*-(4-methoxyphenyl)-benzamide | yellow solid, 75% yield. M.p. 118.1-118.5 °C. $^1H$ NMR (DMSO-$d_6$, 300 MHz) δ 7.51 (br, 4H), 7.05 (br, 4H), 6.82 (br, 4H), 4.10 (m, 2H), 3.78 (s, 3H), 2.81 (m, 2H), 2.56 (m, 4H), 1,64-1.62 (m, 4H), 1.48-1.46 (m, 2H). MS *m*/*z* 456 $(M+H)^+$. |
| **4m**<br>3-Chloro-*N*-[4-(2-piperidin-1-ylethoxy)-phenyl]-*N*-(4-methoxyphenyl)-benzamide | yellow solid, 82% yield. M.p. 114.9-115.5 °C. $^1H$ NMR (DMSO-$d_6$, 300 MHz) δ 7.47-7.46 (m, 1H), 7.26-7.23 (m, 2H), 7.14-7.05 (m, 5H), 6.83-6.80 (m, 4H), 4.08 (tr, 2H, *J* = 6.0 Hz), 3.77 (s, 3H), 2.76 (tr, 2H, *J* = 6.0 Hz), 2.53-2.49 (m, 4H), 1.65-1.58 (m, 4H), 1.48-1.43 (m, 2H). MS *m*/*z* 465 $(M+H)^+$. |
| **7a**<br>5-[4-methoxy-phenyl]-5H-phenanthridin-6-one | yellow solid. 65% yield. M.p. 217.0-218.5 °C (decomposed). $^1H$ NMR (DMSO-$d_6$, 500 MHz) δ 8.61-8.59 (m, 1H), 8.54-8.51 (m, 1H), 8.36-8.34 (m, 1H), 7.94-7.89 (m, 1H), 7.71-7.66 (m, 1H), 7.43-7.28 (m, 4H), 7.19-7.16 (m, 2H), 6.63-6.60 (m, 1H). MS *m*/*z* 302 $(M+H)^+$. |

(continued)

| Compound # and IUPAC NAME | PHYSICAL CHARACTERIZATION |
|---|---|
| **3p**<br>4-Cyano-*N,N*-Bis(4-hydroxyphenyl)-benzamide | white solid, 84% yield. M.p. 272.0-273.5 °C (decomposed). $^1H$ NMR (DMSO-$d_6$, 300 MHz) δ 9.53 (s, 2H), 7.74-7.73 (m, 2H), 7.55-7.53 (m, 2H), 7.12-7.02 (m, 4H), 6.74-6.57 (m, 4H). MS *m/e* 329 $(M-H)^-$. |
| **7b**<br>5-[4-hydroxy-phenyl]-5H-phenanthridin-6-one | yellow solid. 78% yield. M.p. 325.7- 327.0 °C (decomposed). $^1H$ NMR (DMSO-$d_6$, 500 MHz) δ 9.82 (s, 1H), 8.60-8.58 (m, 1H), 8.52-8.51 (m, 1H), 8.35-8.33 (m, 1H), 7.92-7.89 (m, 1H), 7.69-7.66 (m , 1H), 7.41-7.38 (m, 1H), 7.32-7.29 (m, 1H), 7.15-7.13 (m, 2H), 6.99-6.97 (m, 2H), 6.65-6.63 (m, 1H). MS m/z 310 $(M+Na)^+$. |
| **3q**<br>3-Cyano-*N,N*-Bis(4-hydroxyphenyl)-benzamide | white solid, 84% yield. M.p. 237.5-238.0 °C. $^1H$ NMR (DMSO-$d_6$, 300 MHz) δ 9.53 (s, 2H), 7.81-7.80 (m, 1H), 7.75-7.74 (m, 1H), 7.73-7.72 (m, 1H), 7.69-7.68 (m, 1H), 7.67-7.7.66 (m, 1H), 7.48-7.44 (m, 1H), 7.07 (br,4H), 6.65 (br, 4H). MS *m/z* 353 $(M+Na)^+$. |
| **7c**<br>5-[4-(2-piperidin-1-ylethoxy)-phenyl]-*5H*-phenanthridin-6-one | yellow solid. 79% yield. M.p. 220.0- 221.5 °C (decomposed). $^1H$ NMR (DMSO-$d_6$, 300 MHz) δ 8.56-8.53 (m, 1H), 8.35-8.29 (m, 2H), 7.84-7.79 (m, 1H), 7.64-7.59 (m, 1H), 7.36-7.24 (m, 4H), 7.23-7.10 (m, 2H), 6.76-6.73 (m, 1H), 4.45 (tr, 2H, *J* = 5.1Hz), 3.16 (fr, 2H, *J* = 5.1Hz), 2.94 (br, 4H), 1.90-1.85 (m, 4H), 1.61-1.59 (m, 2H). MS m/z 399 $(M+H)^+$. |
| **8b**<br>Cyclohexane-carboxylic acid bis(4-hydroxyphenyl)-amide; | white solid, 86% yield. M.p. 265.1-266.2 °C (decomposed). $^1H$ NMR (DMSO-$d_6$, 500 MHz) δ 9.65 (s, 1H), 9.37 (s, 1H), 7.17-6.70 (m, 4H), 6.78-6.67 (m, 4H), 2.29-2.23 (m, 1H), 1.71-1.62(m, 4H), 1.54-1.51 (m, 1H), 1.41-1.32 (m, 2H), 1.21-1.07(m, 1H), 0.97-0.90 (m, 2H). MS *m/z* 334 $(M+Na)^+$. |
| **3r**<br>*N,N*-Bis(4-hydroxyphenyl)-2-naphthylamide | white solid, 86% yield. M.p. 264.3-265.2 °C (decomposed). $^1H$ NMR (DMSO-$d_6$, 500 MHz) δ 9.46 (s, 2H), 7.98 (s, 1M), 7.85-7.75 (m, 2H), 7.75-7.73 (m, 2H), 7.54-7.48 (m, 2H), 7.45-7.43 (m, 1H), 7.05 (s, 4H), 6.66 (s, 4H). MS *m/z* 356 $(M+H)^+$. |
| **3s**<br>4-Cyano-*N*-[4-(2-piperidin-1-ylethoxy)-phenyl]-N-(4-hydroxyphenyl)-benzamide | white solid, 50% yield. M.p. 266.7-268.0 (decomposed). $^1H$ NMR (DMSO-$d_6$, 300 MHz) δ 9.57 (s, 1H), 7.76-7.74 (m, 2H), 7.58-7.55 (m, 2H), 7.24-6.96 (m, 6H), 6.66 (s, 2H), 4.26-4.21 (m, 2H), 3.33 (br, 4H). 2.98 (br, 2H), 1.70 (br, 4H), 1.50-1.44 (m, 2H). MS *m/z* 442 $(M+H)^+$. |
| **3t**<br>3-Chloro-*N*-[4-(2-piperidin-1-ylethoxy)-phenyl]-*N*-(4-hydroxyphenyl)-benzamide | white solid, 38% yield. M.p. 208.9-209.5 °C (decomposed). $^1H$ NMR (DMSO-$d_6$, 300 MHz) δ 9.54 (s, 1H), 7.44 (s, 1H), 7.37-7.21 (m, 5H), 7.08-7.05 (m, 2H), 6.96 (s, 2H), 6.69-6.67 (s, 2H), 4.27 (s, 2H), 3.33 (br, 4H). 3.02 (br, 2H), 1.71 (br, 4H), 1.50 (br, 2H). MS *m/z* 451 $(M+H)^+$. |

(continued)

| Compound # and IUPAC NAME | PHYSICAL CHARACTERIZATION |
|---|---|
| **10w**<br>*N*-cyclohexyl-4-hydroxy-*N*-(4-hydroxyphenyl)-benzamide | white solid. 81% yield. M.p. 267.3-268.5 °C. $^1$H NMR (DMSO-*d6*, 500 MHz) δ 9.56 (s, 2H), 7.03 (d, 2H, *J* = 8.7 Hz), 6.83 (d, 2H, *J* = 8.8 Hz), 6.60 (d, 2H, *J* = 8.5 Hz), 6.50 (d, 2H, *J* = 8,3 Hz), 4.43 (m, 1H), 1.83-1.81 (m, 2H), 1.72-1.69 (m, 2H), 1.54-1.52 (m, 1H), 1.35-1.28 (m, 2H), 1.11-1.03 (m, 2H), 0.93-0.89 (m, 1H). MS *m*/*z* 312 $(M+H)^+$. |
| **10x**<br>4-((4-Fluorophenyl)(4-hydroxybenzyl)-amino)phenol | yellow oil. 92% yield. $^1$H NMR (DMSO-$d_6$, 500 MHz) δ 9.29 (s, 1H), 9.24 (s, 1H), 7.09 (d, 2H, *J* = 8.3 Hz), 6.98 (d, 2H, *J* = 9.0 Hz), 6.94-6.91 (m, 2H), 6.73 (d, 2H, *J* = 9.0 Hz), 6.68-6.64 (m, 4H), 4.70 (s, 2H). MS *m*/*z* 308 $(M-H)^-$. |
| **10y**<br>*N*-(4-(2-(dimethylamino)ethoxy)phenyl)-*N*-(4-hydroxy-phenyl)-benzamide | white solid. 57% yield. M.p. 170.0-172.0°C. $^1$H NMR (DMSO-$d_6$, 500 MHz) δ 9.50 (s, 1H), 7.37 (d, 2H, *J* = 8.0 Hz), 7.29-7.24 (m, 3H), 7.12(d, 2H, *J* = 6.5 Hz), 7.01 (d, 2H, *J* = 6.5 Hz), 6.87 (s, 2H), 6.66 (s, 2H), 3.99 (s, 2H), 2.61 (t, 2H, *J* = 5.5 Hz), 2.21 (s, 6H). MS *m*/*z* 375 $(M-H)^-$. |
| **10z**<br>3-Cyano-*N*-(4-hydroxyphenyl)-*N*-(4-(2-(piperidin-1-yl) ethoxy)phenyl)-benzamide; | pale-yellow solid. 63% yield. M.p. 160.7-162.3°C. $^1$H NMR (DMSO-$d_6$, 500 MHz) δ 9.56 (s, 1H), 7.83 (s, 1H), 7.76-7.74 (m, 1H), 7.71-7.68 (m, 1H), 7.47 (t, 1H, *J* = 7.5 Hz), 7.19 (br, 2H), 7.08 (br, 2H), 6.90 (br, 2H), 6.66 (br, 2H), 4.02 (br, 2H), 2.63 (br, 2H), 2.42 (br, 4H), 1.48 (br, 4H), 1.36 (br, 2H). MS *m*/*z* 442 $(M+H)^+$. |
| **11a**<br>*N*-(4-Hydroxyphenyl)-*N*-(4-(2-(pyrrolidin-1-yl)ethoxy) phenyl)-benzamide; | white solid. 64% yield, M.p. 163.9-165.1°C. $^1$H NMR (DMSO-$d_6$, 500 MHz) δ 9.63 (s, 1H), 7.39-7.38 (m, 2H), 7.31-7.23 (m, 3H), 7.13 (d, 2H, *J* = 6.0 Hz), 7.02 (d, 2H, *J* = 7.0 Hz), 6.8 (br, 2H), 4.03 (br, 2H), 2.82 (br, 2H), 2.56 (br, 4H), 1.69-1.68 (m,4H). MS *m*/*z* 401 $(M-H)^-$. |
| **11b**<br>*N,N*-Bis(4-hydroxyphenyl)-4-(trifluoromethyl)-benzamide; | white solid. 96% yield. M.p. 255.9-256.5 °C. $^1$H NMR (DMSO-$d_6$, 500 MHz) δ 9.52 (s, 2H), 7.64-7.56 (m, 4H), 7.06 (br, 4H), 6.64 (br, 4H). MS *m*/*z* 374 $(M+H)^+$. |
| **11c**<br>*N*-(4-Hydroxyphenyl)-*N*-(4-(2-(piperidin-1-yl)ethoxy) phenyl)-4-(trifluoromethyl)-benzamide; | white solid. 41% yield. M.p. 158.1-158.7 °C. $^1$H NMR (DMSO-$d_6$, 500 MHz) δ 9.55 (s, 1H), 7.65-7.58 (m, 4H), 7.18-6.65 (m, 8H), 4.01 (br, 1H), 2.63-2.61 (m, 2H), 2.40-2.36 (m, 4H), 1.47 (br, 4H), 1.36 (br, 2H). MS *m*/*z* 485 $(M+H)^+$. |
| **11d**<br>*N,N*-Bis(4-hydroxyphenyl)-4-nitro-benzamide | white solid. 92% yield. M.p. 216.0-217,0 °C (decomposed), $^1$H NMR (DMSO-$d_6$, 500 MHz) δ 9.55 (s, 2H), 8.11-8.09 (m, 2H), 7.65-7.62 (m, 2H), 7.15-7.03 (m, 4H), 6.77-6.58 (m, 4H). MS *m*/*z* 349 $(M-H)^-$. |
| **11e**<br>3-Fluoro-*N,N*-bis(4-hydroxyphenyl)-benzamide | white solid. 87% yield. M.p. 254.1.1-254.6 °C. $^1$H NMR (DMSO-$d_6$, 500 MHz) δ 9.51 (s, 2H), 7.31-7.26 (m, 1H), 7.19-7.18 (m, 2H), 7.13-7.09 (m, 1H), 7.05 (br, 4H), 6.68 (br, 4H). MS *m*/*z* 322 $(M-H)^-$. |
| **11f**<br>*N*-(4-Hydroxyphenyl)-*N*-(4-(2-(piperidin-1-yl)ethoxy) phenyl)-1-naphthamide | white solid. 71 % yield. M.p. 198.5-199.1 °C. $^1$H NMR (DMSO-$d_6$, 500 MHz) δ 9.56 (s, 0.5H), 9.40 (s, 0.5H), 8.13-8.10 (m, 1H), 7.89-7.78 (m, 2H), 7.63-7.58 (m, 1H), 7.54-7.49 (m, 2H), 7.38-7.30 (m, 3H), 7.00 (br, 3H), 6.83 (br, 1H), 6.61 (br, 1H), 6.40 (br, 1H), 4.17-3.92 (m, 2H), 2.73-2.44 (m, 6H), 1.55-1.41 (m, 6H). MS *m*/*z* 467 $(M+H)^+$. |

(continued)

| Compound # and IUPAC NAME | PHYSICAL CHARACTERIZATION |
|---|---|
| **11g**<br>3-Fluoro-*N*-(4-hydroxyphenyl)-*N*-(4-(2-(piperidin-1-yl) ethoxy)phenyl)benzamide | white solid. 53% yield. M.p. 227.3-228.0 °C. $^1$H NMR (DMSO-$d_6$, 500 MHz) δ 9.56 (s, 1H), 7.33-7.26 (m, 1H), 7.21-7.12 (m, 5H), 7.09-7.04 (m, 2H), 6.89 (br, 2H), 4.05 (br, 2H), 2.71 (br, 2H), 2.50 (br, 4H), 1.93 (br, 2H), 1.51-1.49 (m, 4H), 1.39-1.37 (br, 2H). MS *m/z* 435 (M+H)*. |
| **11h**<br>*N*-(4-Hydroxypheny)-4-nitro-*N*-(4-(2-(piperidin-1-yl) ethoxy)phenyl)benzamide | yellow solid. 49% yield. M.p. 181.7-182.3 °C. $^1$H NMR (DMSO-$d_6$, 300 MHz) δ 9.55 (s, 1H), 8.07 (d, 2H, *J* = 8.7 Hz), 7.62 (d, 2H, *J* = 8.7 Hz), 7.18-6.90 (m, 4H), 6.77-6.61 (m, 4H), 4.01 (br, 2H), 2.64-2.43 (m, 6H), 1.46-1.35 (m, 6H). MS *m/z* 4 62 $(M+H)^+$. |
| **11i**<br>*N,N*-Bis(4-hydroxyphenyl)-4-methoxy-1-naphthamide | white solid. 48% yield. M.p. 305.4 °C (decomposed). $^1$H NMR (DMSO-$d_6$, 300 MHz) δ 9.45 (s, 2H), 8.14-8.10 (m, 2H), 7.65-7.60 (m, 1H), 7.54-7.49 (m, 1H), 7.44-7.41 (m, 1H), 7.11 (br, 4H), 6.84-6.81 (m, 1H), 6.70-6.65 (m, 4H), 3.93 (s, 3H). MS *m/z* 386 $(M+H)^+$. |
| **11j**<br>*N*-(4-Hydroxyphenyl)-*N*-(4-(2-(piperidin-1-yl)ethoxy) phenyl)-2-naphthamide | white solid. 45.0% yield. M.p. 195.8-196.3 °C. $^1$H NMR (DMSO-$d_6$, 300 MHz) δ 9.49 (s,1H), 8.01 (s,1H), 7.87-7.84 (m, 2H), 7.77-7.74 (m, 1H), 7.56-7.43 (m, 3H), 7.19-7.16 (m, 2H), 7.08-7.06 (m, 2H), 6.88-6.85 (m, 2H), 6.67-6.65 (m, 2H), 3.99 (s, 2H), 2.61-2.57 (m, 2H), 2.39 (s, 4H), 1.46-1.45 (m, 4H), 1.37-1.35 (m, 2H). MS *m/z* 467 $(M+H)^+$. |
| **11k**<br>2-Hydroxy-*N,N*,2-tris(4-hydroxyphenyl)-propanamide | white solid. 78% yield. M.p. 292.0-294.0 °C. $^1$H NMR (DMSO-$d_6$, 300 MHz) δ 9.74 (s, 1H), 9.39 (s, 1H), 9.15 (s, 1H), 7.18 (d, 2H, *J* = 8.7 Hz), 7.09 (d, 2H, *J* = 8.7 Hz), 6.80 (d, 2H, *J* = 8.7 Hz), 6.72 (d, 2H, *J* = 8.7 Hz), 6.60 (d, 2H, *J* = 9.1 Hz), 6.53 (d, 2H, *J* = 9.1 Hz), 1.68 (s, 3H). MS *m/z* 348 $(M-H_2O)^+$. |
| **11W**<br>*N*-(4-hydroxyphenyl)-4-(3-hydroxypropyl)-*N*-(4-methoxyphenyl)-benzamide | pale-yellow solid, M.p. 145-147 °C. $^1$H NMR ((DMSO-$d_6$, 300 MHz) δ 9.50 bs, 1H), 7.28 (0 (d, *J* = 8.10 Hz, 2H), 7.15-7.05 (m, 4H), 6.99 (d, *J* = 8.62 Hz, 2H), 6.86 (d, *J* = 8.74 Hz, 2H), 6.66 (d, *J* = 8.56 Hz, 2H), 4.44 (bs, 1H), 3.71 (s, 3H), 3.35-3.33 (m, 2H), 2.57-2.49 (m, 2H), 1.69-1.60 (m, 2H). MS *m/z* 376.0 $(M-H)^-$. |
| **11x**<br>4-fluoro-*N,N*-bis(4-hydroxyphenyl)-2-(trifluoromethyl)-benzamide | colorless oil. $^1$H NMR ((DMSO-$d_6$, 300 MHz) δ7.29 (d, *J* = 8.13 Hz, 2H), 7.13-7.09 (m, 4H), 7.00 (d, *J* = 8.61 Hz, 2H), 6.86 (d, *J* = 8.70 Hz, 2H), 6.66 (d, *J* = 8.49 Hz, 2H), 3.71 (s, 3H), 2.76 (t, *J* = 7.43 Hz, 2H), 2.51 - 2.45 (m, 2H). MS *mlz* 390.0 $(M-H)^-$. |
| **11y**<br>3-fluoro-*N*-(4-fluorophenyl)-4-hydroxy-*N*-(4-hydroxyphenyl)benzamide | M.p. 110-112°C. MS *m/z* 364.1 $(M+Na)^+$. $^1$H NMR ((DMSO-$d_6$, 300 MHz) δ 10.14 (bs, 1H), 9.71 (bs, 1H), 7.25-7.11 (m, 5H), 7.05-6.99 (m, 3H), 6.78 (t, *J* = 8.61 Hz, 2H), 6.68 (d, *J* = 8.68 Hz, 2H). |
| **11z**<br>*N*-(4-hydroxyphenyl)-4-methyl-*N*-(4-(2-(piperidin-1-yl) ethoxy)phenyl)bonzamide | M.p.135-137 °C.MS *m/z* 431.3 $(M+H)^+$. $^1$H NMR ((DMSO-$d_6$, 300 MHz) δ 9.49 (bs, 1H), 7.26 (d, *J* = 8.06 Hz, 2H), 7.11-6.98 (m, 6H), 6.85 (d, *J* = 8.72 Hz, 2H), 6.66 (d, *J* = 8.53 Hz, 2H), 4.00 (t, *J* = 5.85 Hz, 2H), 2.60 (t, *J* = 5.85 Hz, 2H), 2.41-2.38 (m, 4H), 1.51-1.44 (m, 4H), 1.37-1.36 (m, 2H). |

(continued)

| Compound # and IUPAC NAME | PHYSICAL CHARACTERIZATION |
|---|---|
| **11aa**<br>*N,N*-bis(4-hydroxyphenyl)-isonicotin-amide | M.p. > 240 °C.MS *m/z* 304.9 $(M-H)^-$. $^1H$ NMR (($DMSO-d_6$, 300 MHz) δ 9.54 (bs, 2H), 8.52-8.43 (m, 2H), 7.76-7.72 (m, 1H), 7.31-7.27 (m, 1H), 7.07 (bs, 4H), 6.68 (bs, 4H). |
| **11ab**<br>*N*-(4-hydroxyphenyl)-*N*-(4-(2-(piperidin-1-yl) ethoxy) phenyl)-isonicotinamide | MS *m/z* 418.4 $(M+H)^+$. $^1H$ NMR (($DMSO-d_6$, 300 MHz) δ (s, 1H), 8.53 (d, 9.56 *J* = 1.65 Hz, 1H), 8.44 (dd, *J* = 1.61,4.83 Hz, 1H), 7.78-7.74 (m, 1H), 7.31-7.27 (m, 1H), 6.89 (bs, 2H), 6.68 (bs, 2H), 4.02 (t, *J* = 7.03 Hz, 2H), 2.64 (bs, 2H), 2.43 (bs, 4H), 1.49-1.47 (m, 4H), 1.38-1.36 (m, 2H). |

## EXAMPLE 2

### Effects of SERMs on ER-α, ER-β and AR transactivation

### Materials and Methods:

COS or 293 cells were plated in DME without phenol red + 10% cs FBS at 90,000 cells per well in 24 well plates, and were transfected with 0.25 μg of the vector "ERE-LUC", where a firefly luciferase gene is driven by two estrogen responsive elements and 0.02 μg of the control CMV-LUC, Renilla where a luciferase gene is driven by a CMV promoter. Also 25 ng of ER-α (Figures 1, 5, and 9), 50 ng of ER-β (Figures 2, 6 and 10) or 12.5 ng of AR (Figure 3) were introduced by lipofectamine. All the receptors were cloned from rat tissue into the PCR3.1 vector backbone. Twenty four hours post transfection, cells were treated with 4a, toremifene, 4h, estrogen, DHT, and other SERMs or combinations thereof, as indicated in figures. Cells were harvested 48 hrs after transfection, and assayed for firefly and Renilla luciferas e activity.

### Results

Treating cells with either 4a or toremifene alone had no effect on ER- α activity, under the tested conditions. However, both the compounds inhibited the estradiol ($E_2$) induced ER- α activity to basal levels, suggesting that 4a can regulate ER activity, or function as a SERM, and in this assay functions as an ER- α antagonist.

In order to determine whether the compounds can function as agonists of the ER, COS or 293 cells expressing constructs with luciferase expression under the control of an ERE were incubated with estrogen, toremifene or 4a. While estrogen addition resulted in dose-dependent luciferase expression, neither SERM alone showed any such effect. Similarly, 4h was evaluated for expression of luciferase (Figure 5 a, b and c).

Both 4a and 4h inhibited estrogen stimulated luciferase expression, indicating these compounds may function as SERMs in these circumstances, for example, as estrogen receptor α -antagonists. Compound 10o also inhibited estrogen stimulated luciferase expression, in 293 cells expressing ER-α similarly evaluated (Figure 5 d,e).

COS or 293 cells expressing ER-β (Figures 2 and 6) were similarly evaluated. Under these experimental conditions, neither 4a nor 4h stimulated LUC expression, and each inhibited $E_2$-stimulated LUC expression, indicating their activity as antagonists for ER-β as well.

Under the tested conditions, compound 4a was specific for the ER, since the compound had no effect on LUC expression in COS cells expressing an androgen receptor (AR), nor did it inhibit DHT-induced AR activation (Figure 3).

Additional SERMs were similarly tested for their ability to mediate estrogen receptor signaling in the indicated conditions (Figure 9). Of the SERMs tested, compounds 3e and 3i were the most potent in stimulating ER-α, and compounds 3a, 3e, 3i and 3g were most potent in stimulating ER-β (Figure 10).

## EXAMPLE 3

### Agonist activity of some embodiments of the compounds

### Materials and Methods:

MCF-7 cells were plated at 500,000 cells per well of a 6 well plate. The cells were serum starved for 3 days and then were treated as above for 16 hrs. RNA was isolated and gene expression levels assessed by realtime RT-PCR, following normalization to 18S ribosomal RNA.

**Results:**

While estrogen increased pS2 (gene encoding the trefoil peptides) expression in MCF-7 cells under the conditions tested, 4a only minimally did so, moreover, it inhibited estrogen-induced upregulation of pS2 gene expression, indicating its role as a partial agonist or antagonist in these conditions (Figure 4). Compounds 3e, 3f and 31 increased pS2 expression levels as well (Figure 11).

**EXAMPLE 4**

**Effect of the compounds on TRAP positive multinucleated osteoclasts.**

**Materials and Methods:**

Bone marrow cells isolated from rat femur were cultured in Alpha MEM without phenol red + 10% sterile FBS without phenol red in the presence or absence of 30 ng/mL RANKL and 10 ng/ml GMCSF. The cells were treated for 12 days were stained for tartarate resistant acid phosphatase activity (TRAP) positive multinucleated osteoclasts and were counted.

**Results:**

The administration of GMCSF and RANKL to pluripotent bone marrow progenitors favors their differentiation to osteoclasts. The presence of estrogen strongly suppressed osteoclast differentiation, while the administration of 4a, 4h, and toremifene under these conditions, minimally but dose-dependently suppressed the osteoclast differentiation (Figure 7).

Compound 3e was highly suppressive of osteoclast activity and stimulated osteoblast activity, 3a suppressed osteoclasts, 3d stimulated osteoblasts and suppressed osteoclasts (Figure 14) and 4h was highly suppressive of osteoclast activity under the tested conditions.

**EXAMPLE 5**

**The compounds inhibit androgen independent prostate cancer cell growth.**

**Materials and Methods:**

The prostate cancer cell line PC-3 was plated in RPMI + 10% csFBS at 6000 cells per well of a 96 well plate. Medium was changed to RPMI + 1% csFBS without phenol red and cells were treated for 72 hrs with increasing concentrations of SERMs.

**Results:**

Compounds 4r, 4u and toremifene all inhibited PC-3 cell growth by 100% at 10 μM concentrations. Compound 4h, however, under the same conditions inhibited PC-3 cell growth by 75% even at 1 μM concentration. Compound 4a partially inhibited growth by about 50% (Figure 8). PC-3 growth inhibition was determined qualitatively, *in vitro,* and represented as a grading system based on the ability of the SERMs to inhibit growth. The number -4 is for compounds that induced 100% growth inhibition at 1 uM, - 3 for compounds that inhibit growth by about 75-90%, -2 for about 50-70% and -1 for inhibition less than 50% growth.

Toremifene and compounds 4a, 31, 4e, 4u, 4b, 4r and 4h each inhibited growth (data not shown). Toremifene inhibited growth to a level of -2, as did compounds 31, and 4h. Compounds 4a, 4e and 4b showed moderate inhibition (-1); 4u and 4r demonstrated appreciably greater inhibition represented qualitatively as -3 and -4, respectively.

**EXAMPLE 6**

***In vivo* estrogenic activity** of **some embodiments of the compounds**

**Materials and Methods:**

Female rats were administered increasing doses of toremifene, estrogen and the respective SERMs, and/or ICI-182,780 and uterine weights were determined. Rats that were administered the vehicle alone served as controls.

**Results:**

Rats given compounds 4a, 3d and 4g exhibited estrogenic activity, in terms of increased uterine weight, under the conditions tested (Figures 12 & 13). When 4a, 4g were coadministered with estrogen, an increase in uterine weight was observed. ICI-182,780 reversed 4a and 4g effects on uterine weight increase under these conditions.

Compounds 3e, 31, 4h and 4e exhibited no estrogenic, or anti-estrogenic activity in uterus, and compounds 3a, 3f, 4g, and 4a exhibited estrogenic activity, *in vivo* (data not shown).

**EXAMPLE 7**

**Metabolic stability of some embodiments of the compounds in human liver**

**microsomes**

**Materials and Methods:**

Human liver microsomes were utilized as a representative system in order to assess the potential of the compounds to form pharmacologically inactive or undesired potentially toxic metabolites due to phase I metabolism.

Each substrate or reference control was dissolved at a concentration of 10 mM in DMSO, from which a 5 μM spiking solution was prepared by dilution in water. Substrates (1 μM) were incubated in the presence of human liver microsomes (Xenotech LLC, Kansas City MO) at 0.5 mg/mL fortified with an NADPH regenerating system at 37°C and pH 7.4. The NADPH regenerating system consisted of glucose-6-phosphate dehydrogenase (1 units/mL) in 0.05M $K_2HPO_4$. Duplicate incubations were performed in 96-well polypropylene cluster tubes in a final volume of 250 μL per reaction. At 0, 2, 4, 6, 10, 30, and 60 minutes a stop solution (300 μL acetonitrile) was added to aliquots of the reaction mixture. Precipitated protein was removed by centrifugation (3000 rpm for 15 minutes) and the supernatants were transferred to clean 96-Well plates for analysis.

LC-MS/MS analysis:

The samples were injected onto a Phenomenex Luna hexylphenyl 50X2 mm i.d. 5 uM, column fitted with a guard column. An isocratic mobile phase consisting of 50% acetonitrile and 0.1% formic acid in water was used at a flow rate of 0.3 mL/min. The protonated molecular ion $(M + H)^+$ of the analyte was monitored by MDS/Sciex API 4000QTrap triple quadrupole mass spectrometer using electrospray positive mode ionization with a temperature of 500°C and a spray voltage of 4000V. Total analysis time was 1.5 min per sample. Data Evaluation:

Metabolic stability was defined as the amount of substrate metabolized by the incubation with hepatic microsomes and expressed as a percentage of the initial amount of substrate (% remaining) based on peak area. Initial substrate concentration for each analyte was 1 μM. The initial peak area of each substrate was determined at time zero and metabolic stability was assessed based on the change in analyte peak area from time 0 min to a single fixed timepoint for each sample (2-60 min, representative timepoints are shown in Table 2 below).

**Results:**

Table 2. shows the percent of substrate remaining after designated incubation intervals (0-60 minutes)

**Table 2:**

| | % Remaining | | | |
|---|---|---|---|---|
| Substrate | 0 min | 10 min | 30 min | 60 min |
| [a]Propranolol | 100 | 76 | 69 | 56 |
| [b]Verapamil | 100 | 101 | 36 | 11 |
| 3a | 100 | 140 | 118 | 103 |
| 4a | 100 | 105 | 95 | 74 |
| 3k | 100 | 119 | 120 | 94 |
| 4h | 100 | 99 | 86 | 70 |
| [a]Human Liver Microsomes Slow Reference Control<br>[b]Human Liver Microsomes Intermediate Reference Control | | | | |

Four embodiments of the compounds tested showed reasonable stability in the Phase I metabolic system compared to verapamil, a known substrate for cytochrome P450-mediated inactivation in human liver microsomes (See Table 2). Compounds 3a and 3k were resistant to oxidative and reductive reactions, with 103 and 94% of the initial substrate remaining, respectively, after a 60 minute reaction tune. 4a and 4h showed moderate reactivity in the assay, with 74 and 70% remaining after the incubation period. These data suggest that the piperidine ring substitution on the 4a and 4h compounds render them partially susceptible to Phase I metabolic transformation. Generally the compounds evaluated are not likely to have significant Phase I-mediated first pass hepatic extraction. As the determination of metabolic stability is but an *in vitro* measure to describe the rate and extent of the potential *in vivo* metabolic fate of the compounds, additional studies are ongoing to identify other metabolic pathways which likely contribute to the biologic inactivation of the leads, elucidate the structure of relevant metabolites, and confirm whether the *in vivo* pharmacokinetic profile is consistent with these preliminary *in vitro* data.

**EXAMPLE 8**

**General Synthesis of *N*,*N*-bis Aryl Benzamide Derivatives.**

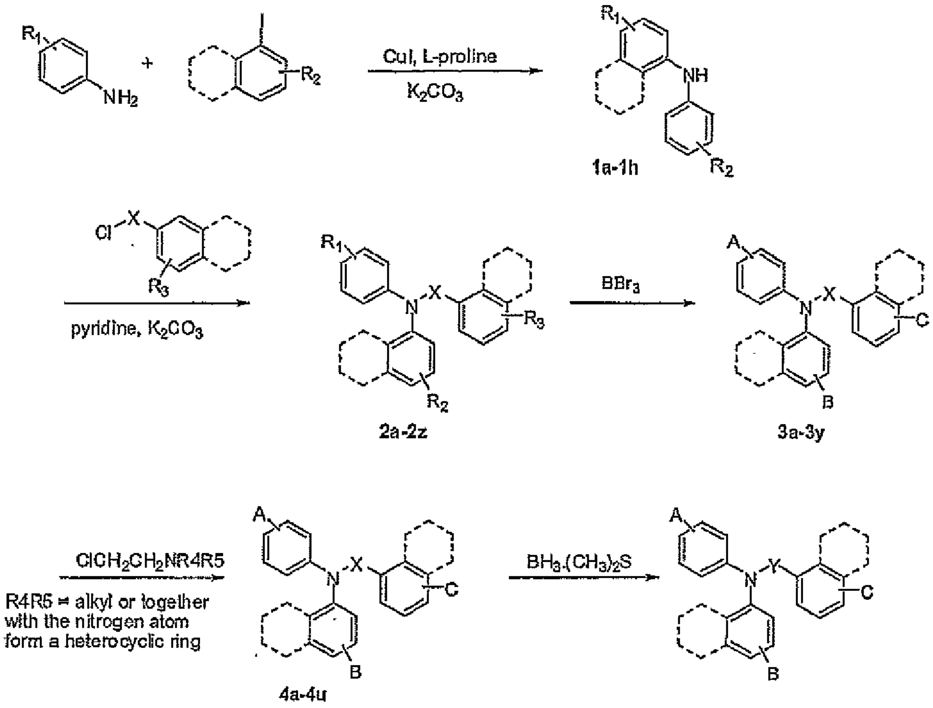


A or B or C = $OCH_2CH_2NR4R5$

***General synthesis of diarylanilines.*** A mixture of arylamine (1.5 equivalent), aryl iodide (1 equivalent), $K_2CO_3$ (2 equivalents), CuI (0.1 equivalent) and *L*-proline (0.2 equivalent) were mixed together and dissolved in anhydrous DMSO at room temperature. Then, the reaction mixture was stirred and heated to 90 °C for 28 hours. The mixture was cooled to room temperature and hydrolyzed with water. EtOAc was added to partition the solution. The EtOAc layer was separated, washed with brine, dried over anhydrous $MgSO_4$. The solvent was removed under reduced pressure. The solid residue was purified by flash column chromatography (silica gel) using 5% EtOAc/hexanes as eluent to afford the corresponding diarylaniline.

**Bis-(4-methoxyphenyl)amine (1a):** pale-yellow solid, 73% yield. M.p. 98.6-99.0 °C. $^1H$ NMR ($CDCl_3$, 300 MHz) δ 6.93-6.81 (m,8H), 5.37(s, br, 1H), 3.78(s, 6H). MS *m/z* 228.4$(M-H)^+$.

***N*-(4- Methoxyphenyl)-phenylamine (1b):** pale-yellow solid, 70% yield. M.p. 106.3 -106.5 °C. $^1H$ NMR. ($CDCl_3$, 300 MHz) δ 7.24-7.18 (m,3R), 7.08-7.06 (m, 2H), 6.92-6.84 (m, 4H), 5.61(s, br, 1H), 3.79(s, 3H). MS *m/z* 200.1$(M+H)^+$.

***N*-(4-Methoxyphenyl)-*N*-3-methoxyphenylamine (1c):** pale-yellow solid, 54% yield. M.p. 69.7-70.0 °C. $^1H$ NMR ($CDCl_3$, 300 MHz) δ 6.93-6.81 (m,8H), 5.37(s, br, 1H), 3.78(s, 6H). MS *m/z* 228.4$(M-H)^+$.

***N*-(4-Fluorophenyl)-*N*-4-methoxyphenylamine (1d):** pale-yellow solid, 54% yield. M.p. 60.6-61.0 °C. $^1H$ NMR ($CDCl_3$, 300 MHz) δ 7.01-6.83 (m,8H), 3.78(s, 3H). MS *m/z* 217 $(M)^+$.

***N*-(4-Methoxyphenyl)-*N*-1-naphthylamine (1e):** pale-yellow solid, 54% yield. M.p. 105.8-106.0 °C. $^1H$ NMR ($CDCl_3$, 500 MHz) δ 8.00 (d, 1H, J = 8.0Hz), 7.92 (d, 1H, J = 8.0Hz), 7.50-7.43 (m, 3H), 7.33-7.30 (m, 1H), 7.10 (d, 1H, J = 7.5Hz), 7.05 (d, 2H, J= 8.5Hz), 6.88 (d, 2H, J = 8.5HZ), 3.80 (s, 3H). MS *m/z* 249 $(M)^+$.

***N*-(4-Benzyloxyphenyl)-*N*-4-methoxyphenylamine (1f):** pale-yellow solid, 54% yield. M.p. 108.0-108.4 °C. $^1H$ NMR ($CDCl_3$, 300 MHz) δ 7.34-7.08 (m, 5H), 6.90-6.81(s, 3H), 3.78 (s, 3H). MS *m/z* 306$(M+H)^+$.

***N*-[4-(Benzyloxy) phenyl) biphenyl- 4- amine (1g):** tan solid, 40.2% yield. M.p. 136-138 °C. $^1H$ NMR ($CDCl_3$, 300 MHz) δ 5.04 (s, 2H, $CH_2$), 6.93-6.99 (m, 4H, ArH), 7.02-7.11 (m, 2H, ArH), 7.22-7.48 (m, 9H, ArH), 7.53-7.56 (m, 3H, ArH). MS *m/z* 352.2 $(M+H)^+$.

***N*-[4-(Benzyloxy) phenyl] biphenyl- 4- amine (1h):** tan solid. 40.2% yield. M.p. 136-138 °C. $^1H$ NMR ($CDCl_3$, 300 MHz) δ 7.56-7.53 (m, 3H, ArH), 7.48-7.22 (m, 9H, ArH), 7.11-7.02 (m, 2H, ArH), 6.99-6.93 (m, 4H, ArH), 5.04 (s, 2H, $CH_2$); MS *m/z* 352.2 $(M+H)^+$.

## EXAMPLE 9

### General Synthesis of Benzamides.

A mixture of arylaniline (1 equivalent), benzoyl chlorides (1.3 equivalents), and pyridine (6 equivalents) was

mixed together and dissolved in anhydrous THF at room temperature. The mixture was stirred and refluxed for 24 hours. The reaction solution was cooled to room temperature, and hydrolyzed by addition of 2N HCl solution. The solution was extracted with ethyl acetate. The organic layer was washed with a saturated aqueous $NaHCO_3$ solution to remove excess acid, dried over anhydrous $MgSO_4$, filtered and concentrated under reduced pressure. The residue was purified by flash column chromatography using EtOAc/hexanes (3/7 v/v) to afford the corresponding benzamide compounds.

**4-Methoxy-*N,N*-bis-(4- methoxyphenyl)-benzamide (2a):** white solid, 98% yield. M.p. 119.5-120 °C. $^1$H NMR ($CDCl_3$, 300 MHz) δ 7.42(d, 2H, J = 8.9Hz), 7.05(d, 4H, J = 8.8 Hz), 6.81 (d, 4H, J = 8.9 Hz), 6.71 (d, 2H, J = 8.9Hz), 3.77 (s, 9H). MS *m/z* 364(M+H).

**3-Methoxy-*N,N*-bis-(4- methoxyphenyl)-benzamide (2b):** white solid, 99% yield. M.p. 113.5-113.6 °C. $^1$H NMR (DMSO-$d_6$, 300 MHz) δ 7.17-7.14 (m, 5H), 6.97-6.95 (m, 3H), 6.87-6.84 (m, 4H). MS *m/z* 364(M+H)$^+$.

**4-Methoxy-*N*-(4-methoxyphenyl)-*N*-(3- methoxyphenyl)-benzamide (2c):** white solid, 79% yield. M.p. 154.5-154.9 °C. $^1$H NMR ($CDCl_3$, 300 MHz) δ 7.47-7.43 (m, 2H), 7.31-7.13 (m, 7H), 6.75-6.68 (m, 4H), 3.77 (s, 3H), 3.71 (s, 3H). MS *m/z 356* (M+Na)$^+$.

***N,N*-Bis-(4- methoxyphenyl)-benzamide (2d):** white solid, 98% yield. M.p. 77-77.5 °C. $^1$H NMR ($CDCl_3$, 300 MHz) δ 7.46-7.42(m, 2H), 7.29-7.17(m, 3H), 7.09-7.06(m, 4H), 6.81-6.78 (m, 4H), 3.76 (s, 6H). MS *m/z* 356 (M+Na)$^+$.

**4-Methoxy-*N,N*-diphenyl- benzamide (2e):** white solid, 99% yield. M. p. 133.5-133.9°C. $^1$H NMR ($CDCl_3$, 300 MHz) δ 7.45-7.42(m, 2H), 7.29-7.24(m, 4H), 7.18-7.12 (m, 6H), 6.71-6.68 (m, 2H), 3.74 (s, 3H). MS *m/z* 326 (M+Na)$^+$.

**3-Methoxy-*N,N*-diphenyl- benzamide (2f):** white solid, 98% yield. M. p. 122-122.2°C. $^1$H NMR ($CDCl_3$, 300 MHz) δ 7.45-7.42(m, 2H), 7.29-7.24(m, 4H), 7.18-7.12 (m, 6H), 6.71-6.68 (m, 2H), 3.74 (s, 3H). MS *m/z* 326 (M+Na)$^+$.

***N,N*-Diphenyl- benzamide (2g):** white solid, 89% yield. M.p. 178.4-179.3 °C. $^1$H NMR ($CDCl_3$, 300 MHz) δ 7.46-7.44(m, 2H), 7.28-7.23(m, 5H), 7.21-7.14(m, 8H). MS *m/z* 296 (M+Na.)$^+$.

***N*-(4-Methoxyphenyl)-*N*-phenyl- benzamide (2h):** white solid, 95% yield. M. p. 153-154.2°C. $^1$H NMR ($CDCl_3$, 300 MHz) δ 7.47-7.43 (m, 2H), 7.30-7.02 (m, 8H), 6.83-6.78 (m, 2H), 3.76 (s, 3H). MS *m/z* 326 (M+Na)$^+$.

***N*-(3-Methoxyphenyl)-*N*-phenyl- benzamide (2i):** white solid, 93% yield. M. p. 103-105.9°C, $^1$H NMR ($CDCl_3$, 300 MHz) δ 7.49-7.45 (m, 2H), 7.31-7.15 (m, 9H), 6.75-6.70 (m, 3H), 3.76 (s, 3H). MS *m/z* 326 (M+Na)$^+$.

**4-Methoxy-*N*-(4-methoxyphenyl)-*N*-phenyl- benzamide (2j):** white solid, 78% yield. $^1$H NMR ($CDCl_3$, 300 MHz) δ 7.44-7.41 (m, 2H), 7.28-7.26 (m, 2H), 7.15-7.05 (m, 5H), 6.83-6.80 (m, 2H), 6.72-6.70 (m, 2H), 3.77 (s, 6H). MS *m/z* 356 (M+Na)$^+$.

**4-Methoxy-*N*-(3-methoxyphenyl)-*N*-phenyl- benzamide (2k):** white solid, 84% yield. M.p. 119.0-119.5 °C. $^1$H NMR ($CDCl_3$, 300 MHz) δ 7.47-7.43 (m, 2H), 7.31-7.13 (m, 7H), 6.75-6.68 (m, 4H), 3.77 (s, 3H), 3.71 (s, 3H). MS *m/z* 356 (M+Na)$^+$,

***N,N*-Bis (4- methoxyphenyl)- 4- fluorobenzamide (2l):** white solid, 98% yield. M.p. 122.2-122.4 °C. $^1$H NMR ($CDCl_3$, 300 MHz) δ 7.46-7.42(m, 2H), 7.29-7.17(m, 3H), 7.09-7.06(m, 4H), 6.81-6.78 (m, 4H), 3.76 (s, 6H). MS *m/z* 356 (M+Na)$^+$.

**4-Methoxy-*N,N*-diphenyl- sulfonamide (2m):** white solid, 89% yield. M.p. 153.0-153.5 °C. $^1$H NMR ($CDCl_3$, 300 MHz) δ 7.64-7.61 (m, 2H), 7.34-7.22 (m, 10H), 6.94-6.91 (m, 2H), 3.86 (s, 3H). MS *m/z* 362 (M+Na)$^+$.

**4-Methoxy-*N*-(4-methoxyphenyl)-*N*-(4- fluorophenyl)-benzamide (2n):** white solid, 97% yield. M.p. 133.5.0-134.5 °C. $^1$H NMR ($CDCl_3$, 300 MHz) δ 8.11-6.66 (m, 15H), 3.74 (s, 3H), 3.73 (s, 3H). MS *m/z* 384 (M+H)$^+$.

**4-Methoxy-*N*-(4-methoxyphenyl)-*N*-(1- naphthyl)-benzamide (2o):** white solid, 65% yield. M.p. 144.0-144.5 °C. $^1$H NMR ($CDCl_3$, 300 MHz) δ 8.11-6.66 (m, 15H), 3.74 (s, 3H), 3.73 (s, 3H). MS *m/z* 384 (M+H)$^+$.

***N*-(4-Methoxyphenyl)-*N*-(4- benzyloxyphenyl)- 1- naphthylamide (2p).** white solid, 95% yield. M.p. 143.5 -144.0 °C. $^1$H NMR ($CDCl_3$, 300 MHz) δ 8.25-8.22 (m, 1H), 7.79-7.69 (m, 2H), 7.57-7.22 (m, 9H), 6.96-6.63 (m, 8H), 4.99 (s, 2H), 3.71 (s, 3H). MS *m/z* 460 (M+H)$^+$.

**4-Chloro-*N*-(4-methoxyphenyl)-*N*-(4- benzyloxyphenyl)-benzamide (2q):** white solid, 96% yield. M.p. 130.0-131.4 °C. $^1$H NMR (DMSO-$d_6$, 300 MHz) δ. MS *m/z* 444 (M+H)$^+$.

**4-Cyano-*N*-(4-methoxyphenyl)-*N*-(4- benzyloxyphenyl)-benzamide (2r):** white solid, 85% yield. M.p. 147.6-148.0 °C. $^1$H NMR (DMSO-$d_6$, 300 MHz) δ. MS *m/z* 435 (M+H)$^+$.

***N*-(4-Methoxyphenyl)-*N*-(4- benzyloxyphenyl)- 2- naphthylamide (2s):** white solid, 58% yield. M.p. 174.9-175.5 °C. $^1$H NMR ($CDCl_3$, 300 MHz) δ 8.04 (s, 1H), 7.77-7.74 (m, 2H), 7.64-7.61 (m, 1H), 7.51-7.43 (m, 4H), 7.40-7.31 (m, 4H), 7.13-7.10 m, 4H), 6.88-6.78 (m, 4H), 4.99 (s, 2H), 3.74 (s, 3H). MS *m/z* 460 (M+H)$^+$.

**4-(Benzyloxy)-*N*-[4-(benzyloxy)phenyl]-*N*-(4- methoxyphenyl) benzamide (2t):** tan solid. 72.4% yield. M.p. 175-178 °C. $^1$H NMR (DMSO-$d_6$, 300 MHz) δ 7.37-7.34 (m, 12H, ArH), 7.13-7.10 (m, 4H, ArH), 6.96-6.89 (m, 2H, ArH), 6.88-6.86 (m, 4H, ArH), 5.06 (s, 4H, 2 X $CH_2$), 3.72 (s, 3H, $OCH_3$). MS *m/z* 516.3 (M+H)$^+$.

***N*-[4-(Benzyloxy)phenyl]-4-methoxy-*N*-(4- methoxyphenyl) benzamide (2u):** yellow oil. 71.3% yield. $^1$H NMR ($CDCl_3$, 300MHz) δ 7.44-7.34 (m, 7H, ArH), 7.07-7.04 (m, 4H, ArH), 6.89-6.86 (m, 2H, ArH), 6.82-6.79 (m, 2H, ArH), 6.72-6.69 (m, 2H, ArH), 5.01 (s, 2H, $CH_2$), 3.77 (s, 6H, 2 X $OCH_3$). MS *m/z* 462.1 (M+Na)$^+$.

***N*-[4-(Benzyloxy)phenyl]-*N*-biphenyl- 4- yl- 4- methoxybenzamide (2v):** light-yellow foam. 78.6% yield. M.p. 70-72 °C. $^1$H NMR (DMSO-$d_6$, 300 MHz) δ 7.66-7.57 (m, 4H, ArH). 7.47-7.32 (m, 10H, ArH), 7.30-7.23 (m, 2H, ArH),

7.18-7.07 (m, 2H, ArH), 7.00-6.92 (m, 2H, ArH), 6.89-6.80 (m, 2H, ArH), 5.06 (s, 2H, $CH_2$), 3.72 (s, 3H, $OCH_3$). MS *m/z* 508.3 (M+Na)$^+$.

**4-Cyano-*N*-(4-methoxyphenyl)-*N*-phenylbenzamide (2w):** pale-yellow solid. 96.3% yield. M.p. 125-128 °C. $^1$H NMR (DMSO-$d_6$, 300 MHz) δ 7.77-7.74 (m, 2H, ArH), 7.61-7.58 (m, 2H, ArH), 7.34-7.21 (m, 7H, ArH), 6.88(d, *J*=7.92 Hz, 2H, ArH), 3.71 (s, 3H, $OCH_3$). MS *m/z* 351.1 (M+Na)$^+$.

**3-Methoxy-*N*-(4-methoxyphenyl)-*N*-phenylbenzamide (2x):** pale-yellow oil. 98.8% yield. $^1$H NMR ($CDCl_3$, 300 MHz) δ 7.30-7.25 (m, 2H, ArH), 7.18-7.07 (m, 6H, ArH), 7.01-6.98 (m, 2H, ArH), 6.83-6.80 (m, 3H, ArH), 3.77 (s, 3H, $OCH_3$), 3.68 (s, 3H, $OCH_3$). MS *m/z* 356.1 (M+Na)$^+$.

**4-Cyano-*N*-(3-methoxyphenyl)-*N*-phenylbenzamide (2y):** brown oil. 84.8% yield. $^1$H NMR (DMSO-$d_6$, 300 MHz) δ 7.77-7.75 (m, 2H, ArH). 7.63-7.61 (m, 2H, ArH), 7.35-7.30 (m, 4H, ArH), 7.25-7.22 (m, 2H, ArH), 6.91 (s, 1H, ArH), 6.83-6.80 (m, 2H, ArH), 3.67 (s, 3H, $OCH_3$). MS *m/z* 351.1 (M+Na)$^+$.

**4-Cyano-*N,N*-diphenylbenzamide (2z):** tan solid. 85.2% yield. M.p. 145-147 °C. $^1$H NMR (DMSO-$d_6$, 300 MHz) δ' 7.76-7.74 (m, 2H, ArH), 7.61-7.59 (m, 2H, ArH), 7.34-7.22 (m, 10H, ArH). MS *m/z* 321.0 (M+Na)$^+$.

## EXAMPLE 10

### General Procedure for Demethylation of Benzamide Derivatives Using $BBr_3$.

A methoxybenzamide compound was dissolved in dry $CH_2Cl_2$. $BBr_3$ (1.0 M $CH_2Cl_2$ solution) was added dropwise at 0 °C. The reaction solution was slowly warmed to room temperature and allowed to stir overnight at room temperature. The mixture was cooled to 0 °C in an ice bath and hydrolyzed by adding water. EtOAc was added to partition the solution. The organic layer was separated; the aqueous layer was extracted with EtOAc. The organic layer was washed with brine and dried over anhydrous $MgSO_4$. The solvent was removed under reduced pressure. The residue was purified by flash column chromatography using $CH_3OH/CH_2Cl_2$ (1/9 v/v) to afford the phenolic compounds.

The following compounds where synthesized as described herein above and characterized and summarized in Table 1: 4-Hydroxy-*N,N*-bis-(4-hydroxyphenyl)-benzamide (3a); 3-Hydroxy-*N*-bis-(4-hydroxyphenyl)-benzamide (3b); 4-Hydroxy-*N*-(4-hydroxyphenyl)-*N*-(3-hydroxyphenyl)-benzamide (3c); *N,N*-Bis-(4-hydroxyphenyl)-benzamide (3d); 4-Hydroxy-*N,N*-diphenyl-benzamide (3e); 3-Hydroxy-*N,N*-diphenylbenzamide (3f); *N*-(4-Hydroxyphenyl)-*N*-phenyl-benzamide (3g); *N*-(3-Hydroxyphenyl)-*N*-phenyl-benzamide (3h); 4-Hydroxy-*N*-(4-hydroxyphenyl)-*N*-phenyl-benzamide (3i); 4-Hydroxy-*N*-(3-hydroxyphenyl)-*N*-phenyl-benzamide (3j); *N,N*-Bis(4-hydroxyphenyl)-4-fluorobenzamide (3k); 4-Hydroxy-*N,N*-diphenylphenylsulfonamide (3l); 4-Hydroxy-*N*-(4-hydroxyphenyl)-*N*-(fluorophenyl)-benzamide (3m); *N,N*-Bis (4-hydroxyphenyl)-1-naphthylamide (3n); 4-Hydroxy-*N*-(1-Naphthyl)-*N*-(4-hydroxyphenyl)-benzamide (3o); 4-Cyano-*N,N*-Bis(4-hydroxyphenyl)-benzamide (3p); 3-Cyano-*N,N*-Bis(4-hydroxyphenyl)-benzamide (3q); *N,N*-Bis(4-hydroxyphenyl)-2-naphthylamide (3r); 4-Cyano-*N*-[4-(2-piperidin-1-ylethoxy)-phenyl]-*N*-(4-hydroxyphenyl)-benzamide (3s); 3-chloro-*N*-[4-(2-piperidin-1-ylethoxy)-phenyl]-*N*-(4-hydroxyphenyl)-benzamide (3t); *N*-Biphenyl-4-yl-*N*-(4-hydroxyphenyl)-4-methoxybenzamide (3u) ; *N*-Biphenyl-4-yl-4-hydroxy-*N*-(4-hydroxyphenyl)-benzamide (3v); 4-Hydroxy-*N*-(4-hydroxyphenyl)-*N*-[4-(2-piperidin-1-ylethoxy)phenyl]-benzamide (3w); 3-Hydroxy-*N*-(4-hydroxyphenyl)-*N*-phenylbenzamide (3x); *N*-Biphenyl-4-yl-4-hydroxy-*N*-[4-(2-piperidin-1-ylethoxy)phenyl]-benzamide (3y); 4-Cyano-N-(4-hydroxyphenyl)-*N*-phenylbenzamide (10a); *N,N*-bis(4-hydroxyphenyl)biphenyl-4-carboxamide (10c), *N,N*-bis(4-hydroxyphenyl)-3,4-dimethylbenzamide (10d); *N*-(biphenyl-4-yl)-4-cyano-N-(4-hydroxyphenyl)-benzamide (10e); 3-fluoro-4-hydroxy-*N*-(4-hydroxyphenyl)-*N*-phenylbenzamide (10f); 4-hydroxy-*N,N*-bis(4-hydroxyphenyl)-3,5-dimethylbenzamide (10i); *N,N*-bis (4-hydroxyphenyl)-2,3-dimethylbenzamide (10j); 3-fluoro-4-hydroxy-*N,N*-bis(4-hydroxyphenyl)-benzamide (10k); *N,N*-bis(4-hydroxyphenyl)-4-propylbenzamide (101); 3,4-dihydroxy-*N,N*-bis(4-hydroxyphenyl)-benzamide (10m); 4-hydroxy-*N,N*-bis(4-hydroxyphenyl)-3-methylbenzamide (10n); *N,N*-bis(4-hydroxyphenyl)-2,4-dimethylbenzamide (10q); *N,N*-bis (4-hydroxyphenyl)-4-methylbenzamide (10s); 4,4'-(2,3-dimethylbenzylazanediyl)diphenol (10t); 4-formyl-*N,N*-bis(4-hydroxyphenyl)-benzamide (10u); *N,N*-bis(4-hydroxyphenyl)-4-(trifluoromethyl)benzamide (11b); *N,N*-bis(4-hydroxyphenyl)-4-nitrobenzamide (11d); 3-fluoro-*N,N*-bis(4-hydroxyphenyl)benzamide (11e); *N,N*-bis(4-hydroxyphenyl)-4-methoxy-1-naphthamide (11i); 4-((hydroxyimino)methyl)-*N,N*-bis(4-hydroxyphenyl)benzamide (111); *N,N*-bis(4-hydroxyphenyl)-4-pentylbenzamide (11p); 4-*tert*-butyl-*N,N*-bis(4-hydroxyphenyl)benzamide (11r); 3-{4-[Bis-(4-hydroxy-phenyl)-carbamoyl]-phenyl}acrylic acid (11t); 3-{4-[Bis-(4-hydroxy-phenyl)-carbamoyl]-phenyl}-propionic acid (11u); *N,N*-Bis-(4-hydroxy-phenyl)-4-(3-hydroxy-propyl)-benzamide (11v); *N*-(4-hydroxyphenyl)-4-(3-hydroxypropyl)-*N*-(4-methoxyphenyl)-benzamide (11w); 4-fluoro-*N,N*-bis(4-hydroxyphenyl)-2-(trifluoromethyl)-benzamide (11x); 3-fluoro-*N*-(4-fluorophenyl)-4-hydroxy-*N*-(4-hydroxyphenyl)benzamide (11y); and *N,N*-bis(4-hydroxyphenyl)-isonicotin-amide (11aa).

**General Procedures for Debenzylation of Benzyloxyphenyl-benzamides.**

Compound was dissolved in EtOH in a 250 mL hydrogenation bottle. Pd/C powder (5% mol) was added to the solution. The reaction vessel was mounted to a hydrogenation apparatus under 20 psi pressure hydrogen gas. The reaction was monitored by TLC until the disappearance of starting material. Then, the solvent was removed under reduced pressure. The residue was purified by flash column chromatography with hexanes/EtOAc = 3/2 v/v to afford the desired product.

The following compounds where synthesized as described herein above and characterized and summarized in Table 1: 4-Chloro-*N*-[4-hydroxyphenyl]-*N*-(4-methoxyphenyl)-benzamide (5a); 4-Cyano-*N*-[4-hydroxyphenyl]-*N*-(4-methoxyphenyl)-benzamide (5b); 3-Chloro-*N*-[4-hydroxyphenyl]-*N*-(4-methoxyphenyl)-benzamide (5c); 4-Hydroxy-*N*-(4-hydroxyphenyl)-*N*-(4-methoxyphenyl)-benzamide (5d); *N*-(4-Hydroxyphenyl)-4-methoxy-*N*-(4-methoxyphenyl)-benzamide (5e).

## EXAMPLE 11

**General synthesis of *O*- (2-piperidin-1-ylethoxy)-benzamides and analogues.**

To a solution of hydroxyphenyl containing benzamide analogue (1 equivalent) in acetone, $K_2CO_3$ (3 equivalents) and *N*-chloroethyl-piperidine hydrochloride salt (1.2 equivalents) were added. The solution was heated to reflux for 6 hours. The solution was evaporated to dryness. The residue was hydrolyzed by adding water, and then extracted with ethyl acetate. The organic layers were separated and dried over anhydrous $MgSO_4$. The solvent was removed under reduced pressure. The residue was purified by flash chromatography with methylene chloride/methanol = 9/1 v/v to give the desired compound. The hydrochloride salts were prepared by adding HCl in $Et_2O$ to the methanol solution of the compounds followed by evaporation of solvents.

The following compounds where synthesized as described herein above and characterized and summarized in Table 1: *N*-(4-Hydroxyphenyl)-*N*-[4-(2-piporidin-1-ylethoxy)-phenyl]-benzamide (4a); *N*-(phenyl)-*N*-[4-(2-piperidin-1-ylethoxy)-phenyl]-benzamide (4b); *N,N*-diphenyl-[3-(2-piperidinylethoxy)]-benzamide hydrochloride (4c); *N,N*-diphenyl-[3-(2-piperidinylethoxy)]-benzamide hydrochloride (4d); *N-(4-*Hydroxyphenyl)-*N*-phenyl-[4-(2-piperidin-1-ylethoxy)]-benzamide hydrochloride (4e); *N,N*-diphenyl-bis[4-(2-piperidin-1-ylethoxy)-phenyl]-sulfonamide hydrochloride (4f); *N*-(4-Fluorophenyl)-*N*-[4-hydroxyphenyl]-[4-(2-piperidin-1-ylethoxy)]-benzamide (4g); *N*-(4-Hydroxyphenyl)-*N*-[4-(2-Piperidin-1-ylethoxy)-phenyl]-4-fluoro-benzamide hydrochloride (4h); 3-(2-piperidin-1-ylethoxy)-*N,N*-bis(4-hydroxyphenyl)-benzamide (4i); 4-Cyano-*N*-[4-(2-piperidin-1-ylethoxy)-phenyl]-*N*-(4-methoxyphenyl)-benzamide (4j); 4-Chloro-*N*-[4-(2-piperidin-1-ylethoxy)-phenyl]-*N*-(4-methoxyphenyl)-benzamide (4k); 4-Cyano-*N*-[4-(2-piperidin-1-ylethoxy)-phenyl]-*N*-(4-methoxyphenyl)-benzamide (4l); 3-Chloro-*N*-[4-(2-piperidin-1-ylethoxy)-phenyl]-*N*-(4-methoxyphenyl)-benzamide (4m); 4-Methoxy-*N*-(4-methoxyphenyl)-*N*-[4-(2-piperidin-1-ylethoxy)phenyl]-benzamide (4n); *N*-Biphenyl-4-yl-*N*-(4-hydroxyphenyl)-4-(2-piperidin-1-ylethoxy)-benzamide (4o); 4-Methoxy-*N*-phenyl-*N*-[4-(2-piperidin-1-ylethoxy)phenyl]-benzamide (4p); *N*-(4-Hydroxyphenyl)-*N*-phenyl-3-(2-piperidin-1-ylethoxy)-benzamide (4q); *N*-(4-hydroxyphenyl)-*N*-(4-(2-(piperidin-1-yl)ethoxy)phenyl)-4-propylbenzamide (10o); *N*-(4-hydroxyphenyl)-2,3-dimethyl-*N*-(4-(2-(piperidin-1-yl)ethoxy)phenyl)-benzamide (10p); *N*-(4-hydroxyphenyl)-*N*-(4-(2-(pyrrolidin-1-yl)ethoxy)phenyl)benzamide (11a); *N*-(4-hydroxyphenyl)-*N*-(4-(2-(piperidin-1-yl)ethoxy)phenyl)-4-(trifluoromethyl)benzamide (11c); *N*-(4-Hydroxyphenyl)-*N*-(4-(2-(piperidin-1-yl)ethoxy)phenyl)-1-naphthamide (11f); 3-fluoro-*N*-(4-hydroxyphenyl)-*N*-(4-(2-(piperidin-1-yl)ethoxy) phenyl)benzamide (11g); *N*-(4-hydroxyphenyl)-4-nitro-*N*-(4-(2-piperidin-1-yl)ethoxy)phenyl)benzamide (11h); *N*-(4-hydroxyphenyl)-*N*-(4-(2-piperidin-1-yl)ethoxy)phenyl)-2-naphthamide (11j); *N*-(4-hydroxyphenyl)-2,4-dimethyl-*N*-(4-(2-(piperidin-1-yl)ethoxy)phenyl)benzamide (11m); *N*-(4-hydroxyphenyl)-3,5-dimethyl-*N*-(4-(2-(piperidin-1-yl)ethoxy)phenyl)benzamide (11n); 4-((2,3-dimethylbenzyl)(4-(2-(piperidin-1-yl)ethoxy)phenyl)amino)phenol (11o); *N*-(4-hydroxyphenyl)-4-pentyl-*N*-(4-(2-(piperidin-1-yl)ethoxy)phenyl)benzamide (11q); 4-*tert*-butyl-*N*-(4-hydroxyphenyl)-*N*-(4-(2-(piperidin-1-yl)ethoxy)phenyl)benzamide (11s); *N*-(4-hydroxyphenyl)-4-methyl-*N*-(4-(2-(piperidin-1-yl)ethoxy)phenyl)benzamide (11z); and *N*-(4-hydroxyphenyl)-*N*-(4-(2-(piperidin-1-yl)ethoxy)phenyl)-isonicotinamide (11ab).

**Synthesis of Two-Tailed SERMs**

*N*-(4-Fluorophenyl)-*N*-[4-(2-piperidin-1-ylethoxy)-phenyl]-[4-(2-piperidin-1-yl-ethoxy)]-benzamide dihydrochloride (4r); *N,N*-Bis[4-(2-piperidin-1-ylethoxy)-phenyl]-4-fluoro-benzamide dihydrochloride (4s); *N,N*-Bis[4-(2-piperidin-1-ylethoxy)-phenyl]-benzamide dihydrochloride (4t); and *N*-[4-(2-piperidin-1-ylethoxy)-phenyl]-*N*-phenyl-[4-(2-piperidin-1-ylethoxy)]-benzamide dihydrochloride (4u).

## EXAMPLE 12

### General procedures for synthesis of cyclohexanecarboxylic acid bis-arylamides.

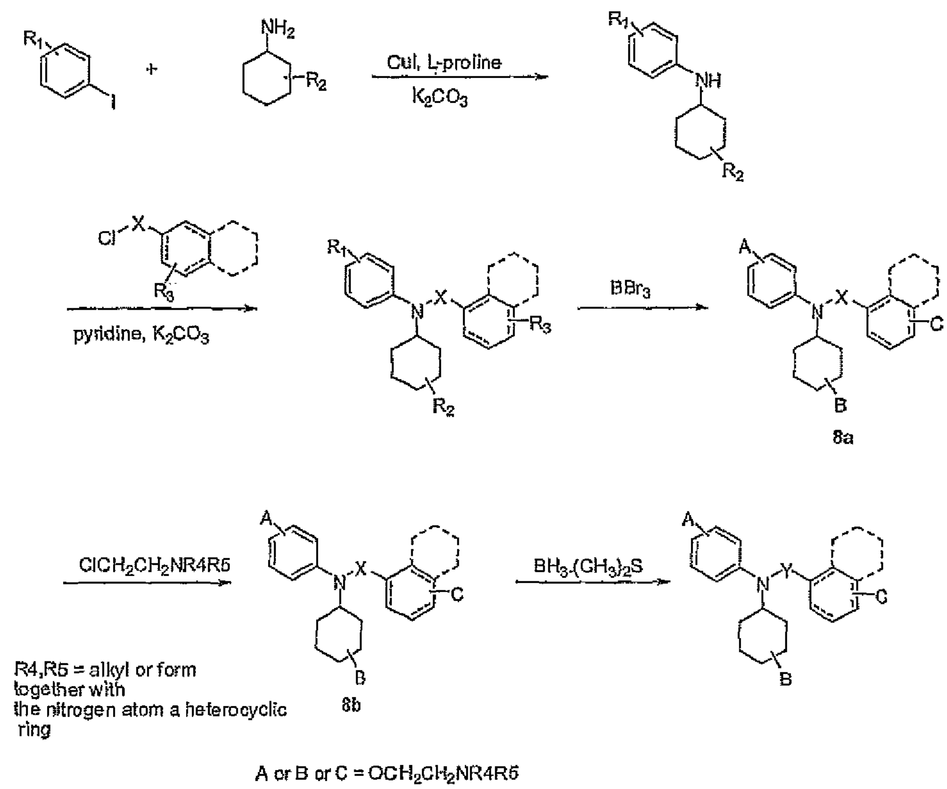


***N*-Cyclohexyl-4-methoxyphenylamine.** This compound was synthesized according the literature. The NMR, MS data and melting point are consistent with those reported in literature.[ref] (D. Ma, Q. Cai, H. Zhang, Org. Lett. 2003, 5, 2453.)

### General Procedures for Synthesis of Cyclohexanecarboxylic acid bis-aryl amides.

Arylaniline (1 equivalent), cyclohexylcarbonyl chloride (1.3 equivalents), and pyridine (6 equivalents). The reaction mixture was stirred and heated to 90 °C for 24 hours. The reaction solution was cooled to room temperature, and hydrolyzed by addition of 2N HCl solution. The solution was extracted with ethyl acetate twice. The combined organic layers were washed with saturated aqueous $NaHCO_3$ solution to remove excess acid, dried over anhydrous $MgSO_4$, filtered and concentrated under reduced pressure. The residue was purified by flash column chromatography using EtOAc/hexanes (3/7 v/v) to afford the corresponding cyclohexylamide compound.

**Cyclohexanecarboxylic acid *N*-(4-methoxyphenyl)-*N*-(4- benzyloxyphenyl) amide (8a):** white solid, 92% yield. M.p. 102.7-128.0 °C. $^1H$ NMR ($CDCl_3$, 300 MHz) δ 7.40-6.90 (m, 13H), 5.03 (s, 2H), 3.80 (s, 3H), 2.41-2.04 (m, 1H), 1.78-1.53 (m, 7H), 1.28-1.06 (m, 3H). MS *m/z* 438 $(M+Na)^+$.

### General Procedure for Synthesis of bis *N*-Hydroxyphenyl Cyclohexanecarboxylic acids,

A methoxyphenylcyclohexylamide compound was dissolved in dry $CH_2Cl_2$. $BBr_3$ (1.0 M $CH_2Cl_2$ solution) was added dropwise with stirring via a syringe at 0 °C. The reaction solution was slowly warmed to room temperature and allowed to stir overnight at room temperature. The mixture was cooled to 0 °C in an ice bath and hydrolyzed by adding water. EtOAc was added to partition the solution. The organic layer was separated; the aqueous layer was extracted with EtOAc twice. The organic layers were combined, washed with brine and dried over anhydrous $MgSO_4$. The solvent

was removed under vacuum. The residue was purified by flash column chromatography using silica-gel with $CH_3OH/CH_2Cl_2$ (1/9 v/v) to afford the pure desired phenolic compound.

**Cyclohexanecarboxylic acid bis(4-hydroxyphenyl)-amide (8b):** white solid, 86% yield. M.p. 265.1-266.2 °c (decomposed). $^1H$ NMR (DMSO-$d_6$, 500 MHz) δ 9.65 (s, 1H), 9.37 (s, 1H), 7.17-6.70 (m, 4H), 6.78-6.67 (m, 4H), 2.29-2.23 (m, 1H), 1.71-1.62 (m, 4H), 1,54-1,51 (m, 1H), 1.41-1.32 (m, 2H), 1.21-1.07 (m, 1H), 0,97-0.90 (m, 2H). MS *m*/*z* 334 $(M+Na)^+$.

## EXAMPLE 13

### General synthesis of 5-[4-methoxyphenyl]-5*H*-phenanthridin-6-ones.

A mixture of 6-(5*H*)-phenathridinone (1.5 equivalent), 4-iodoanisole (1 equivalent), $K_2CO_3$ (2 equivalents), CuI (0.1 equivalent) and *L*-proline (0.2 equivalent) were mixed together and dissolved in anhydrous DMSO at room temperature. Then, the reaction mixture was stirred and heated to 150 °C for 28 hours. The mixture was cooled to room temperature and hydrolyzed with water. EtOAc was added to partition the solution. The EtOAc layer was separated, washed with brine, dried over anhydrous $MgSO_4$. The solvent was removed under reduced pressure. The residue was purified by flash column chromatography (silica gel) using EtOAc/hexanes (2/3 v/v) to afford the desired product.

**5-[4-methoxyphenyl]-5*H*-phenanthridin- 6- one (7a):** yellow solid. 65% yield. M.p. 217.0-218.5 °C (decomposed). $^1H$ NMR (DMSO-$d_6$, 500 MHz) δ 8.61-8.59 (m, 1H), 8.54-8.51 (m, 1H), 8.36-8.34 (m, 1H), 7.94-7.89 (m, 1H), 7.71-7.66 (m , 1H), 7.43-7.28 (m, 4H), 7.19-7.16 (m, 2H), 6.63-6.60 (m, 1H). MS m/z 302 $(M+H)^+$.

**General Synthesis of 5-[4-hydroxyphenyl]-5*H*-phenanthridin-6-ones.**

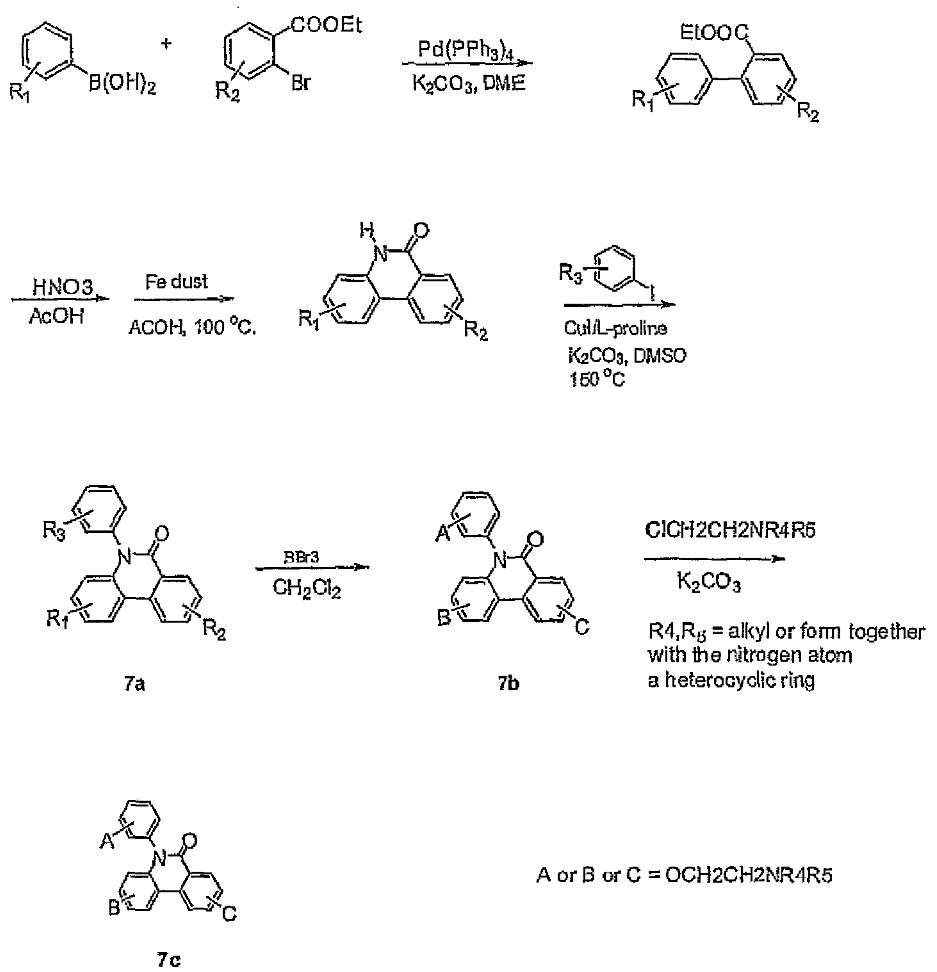


7a

7b

7c

A 5-[4-methoxyphenyl]-5*H*-phenanthridin-6-one was dissolved in dry $CH_2Cl_2$. $BBr_3$ (1.0 M $CH_2Cl_2$ solution) was added dropwise with stirring via a syringe at 0 °C. The reaction solution was slowly warmed to room temperature and allowed to stir overnight at room temperature. The mixture was cooled to 0 °C in an ice bath and hydrolyzed by adding water. EtOAc was added to partition the solution. The organic layer was separated; the aqueous layer was extracted with EtOAc twice. The organic layers were combined, washed with brine and dried over anhydrous $MgSO_4$. The solvent was removed under vacuum. The residue was purified by flash column chromatography using silica-gel with $CH_3OH/CH_2Cl_2$ (1/9 v/v) to afford the pure desired phenolic compound.

**5-[4-hydroxyphenyl]-5*H*-phenanthridin- 6- one (7b):** yellow solid. 78% yield. M.p. 325.7- 327.0 °C (decomposed). $^1H$ NMR (DMSO-$d_6$, 500 MHz) δ 9.82 (s, 1H), 8.60-8.58 (m, 1H), 8.52-8.51 (m, 1H), 8.35-8.33 (m, 1H), 7.92-7.89 (m, 1H), 7.69-7.66 (m, 1H), 7,41-7.38 (m, 1H), 7.32-7.29 (m, 1H), 7.15-7.13 (m, 2H), 6.99-6.97 (m, 2H), 6.65-6.63 (m, 1H). MS m/z 310 $(M+Na)^+$.

**General Synthesis of 5-[4-(2-piperidin-1-ylethoxy)-phenyl]-phenanthridin-6-one Derivatives.**

To a solution of 5-[4-hydroxyphenyl]-phenanthridin-6-one (1 equivalent) in acetone, $K_2CO_3$ (3 equivalents) and *N*-chloroethylpiperidine hydrochloride salt (1.2 equivalents) were added. The solution was heated to reflux for 6 hours. The solution was evaporated to dryness. The residue was hydrolyzed by adding water, and then extracted with ethyl acetate. The organic layers were separated and dried over anhydrous $MgSO_4$. The solvent was removed under reduced pressure. The residue was purified by flash chromatography (silica-gel; methylene chloride/methanol = 9/1 v/v) to give the desired compound. The hydrochloride salts were prepared by adding HCl in $Et_2O$ to the methanol solution of the compounds followed by evaporation of solvents.

**5-[4-(2-piperidin-1-ylethoxy)-phenyl]-5*H*-phenanthridin- 6- one (7c):** yellow solid. 79% yield. M.p. 220.0-221.5 °C (decomposed). $^1H$ NMR (DMSO-$d_6$, 300 MHz) δ 8.56-8.53 (m, 1H), 8.35-8.29 (m, 2H), 7.84-7.79 (m, 1H), 7.64-7.59 (m, 1H), 7.36-7.24 (m , 4H), 7.23-7.10 (m, 2H), 6.76-6.73 (m, 1H), 4.45 (tr, 2H, J = 5.1Hz), 3.16 (tr, 2H, J = 5.1Hz), 2.94 (br, 4H), 1.90-1.85 (m, 4H), 1.61-1.59 (m, 2H). MS m/z 399 $(M+H)^+$.

**EXAMPLE 14**

**General synthesis of 6b, 6c, 6d, 6e, 6f, and 6g**

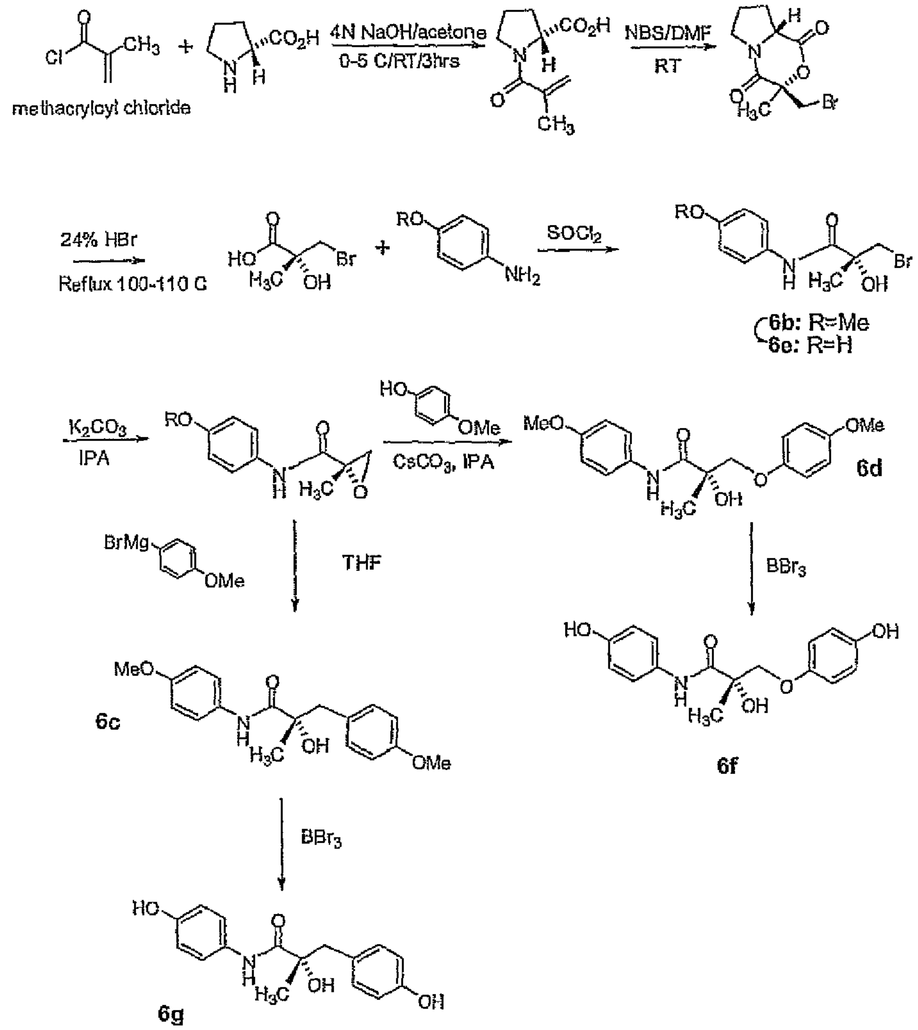


6b: R=Me
6e: R=H

6d

6c

6f

6g

**(2*R*)-1-Methacryloylpyrrolidin-2-carboxylic Acid.** *D*-Proline, 14.93 g, 0.13 mol) was dissolved in 71 mL of 2 N NaOH and cooled in an ice bath; the resulting alkaline solution was diluted with acetone (71 mL). An acetone solution (71 mL) of metacryloyl chloride (13.56 g, 0.13 mol) and 2N NaOH solution (71 mL) were simultaneously added over 40 min to the aqueous solution of *D*-proline in an ice bath. The pH of the mixture was kept at 10-11°C during the addition of the metacryloyl chloride. After stirring (3 h, room temperature), the mixture was evaporated *in vacuo* at a temperature at 35-45 °C to remove acetone. The resulting solution was washed with ethyl ether and was acidified to pH 2 with concentrated HCl. The acidic mixture was saturated with NaCl and was extracted with EtOAc (100 mL x 3). The combined extracts were dried over $Na_2SO_4$, filtered through Celite, and evaporated *in vacuo* to give the crude product as a colorless oil. Recrystallization of the oil from ethyl ether and hexanes afforded 16.2 (68%) of the desired compound as colorless crystals: mp 102-103 °C (lit. [214] mp 102.5-103.5 °C); the NMR spectrum of this compound demonstrated the existence of two rotamers of the title compound. $^1$H NMR (300 MHz, DMSO-$d_6$) δ 5.28 (s) and 5.15 (s) for the first rotamer, 5.15 (s) and 5.03 (s) for the second rotamer (totally 2H for both rotamers, vinyl $CH_2$), 4.48-4.44 for the first rotamer, 4.24-4.20 (m) for the second rotamer (totally 1H for both rotamers, CH at the chiral canter), 3.57-3.38 (m, 2H, $CH_2$), 2.27-2.12 (1H, CH), 1.97-1.72 (m, 6H, $CH_2$, CH, Me); $^{13}$C NMR (75 MHz, DMSO-$d_6$) δ for major rotamer 173.3, 169.1, 140.9, 116.4, 58.3, 48.7, 28.9, 24.7, 19.5: for minor rotamer 174.0, 170.0, 141.6, 115.2, 60.3, 45.9, 31.0, 22.3, 19.7; IR (KBr) 3437 (OH), 1737 (C=O), 1647 (CO, COOH), 1584, 1508, 1459, 1369, 1348, 1178 $cm^{-1}$; $[\alpha]_D^{26}$ +80.8° (c = 1, MeOH); Anal. Calcd. for $C_9H_{13}NO_3$: C 59.00, H 7.15, N 7.65. Found: C 59.13, H 7.19, N 7.61.

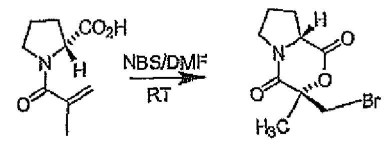

**(3*R*,8a*R*)-3-Bromomethyl-3-methyl-tetrahydro-pyrrolo[2,1-*c*][1,4]oxazine-1,4-dione.** A solution of *N*-bromosuccimide (NBS) (23.5g, 0.132 mol) in 100 mL of DMF was added dropwise to a stirred solution of the (methyl-acryloyl)-pyrrolidine (16.1g, 88 mmol) in 70 mL of DMF under argon at room temperature, and the resulting mixture was stirred 3 days. The solvent was removed *in vacuo,* and a yellow solid was precipitated. The solid was suspended in water, stirred overnight at room temperature, filtered, and dried to give 18.6 (81%) (smaller weight when dried ~ 34%) of the title compound as a yellow solid: mp 152-154 °C (lit. [214] mp 107-109 °C for the *S*-isomer); $^1H$ NMR (300 MHz, DMSO-$d_6$) δ 4.69 (dd, *J* = 9.6 Hz, *J* = 6.7 Hz, 1H, CH at the chiral center), 4.02 (d, *J* = 11.4 Hz, 1H, CHHa), 3.86 (d, *J* = 11.4 Hz, 1H, $CHH_b$), 3.53-3.24 (m, 4H, $CH_2$), 2.30-2.20 (m, 1H, CH), 2.04-1.72 (m, 3H, $CH_2$ and CH), 1.56 (s, 2H, Me); $^{13}C$ NMR (75 MHz, DMSO-$d_6$) δ 167.3, 163.1, 83.9, 57.2, 45.4, 37.8, 29.0, 22.9, 21.6; IR (KBr) 3474, 1745 (C=O), 1687 (C=O), 1448, 1377, 1360, 1308, 1227, 1159, 1062cm$^{-1}$; $[\alpha]_D^{26}$ +124.5 ° (c = 1.3, chloroform); Anal. Calcd. for $C_9H_{12}BrNO_3$: C 41.24, H 4.61, N 5.34. Found: C 41.46, H 4.64, N 5.32.

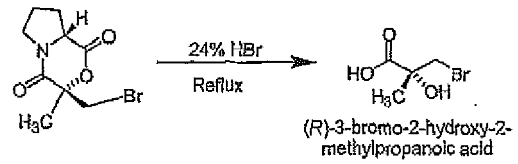


(*R*)-3-bromo-2-hydroxy-2-methylpropanoic acid

**(2*R*)-3-Bromo-2-hydroxy-2-methylpropanoic Acid.** A mixture of bromolactone (18.5g, 71 mmol) in 300 mL of 24% HBr was heated at reflux for 1 h. The resulting solution was diluted with brine (200 mL), and was extracted with ethyl acetate (100 mL x 4). The combined extracts were washed with saturated $NaHCO_3$ (100 mL x 4). The aqueous solution was acidified with concentrated HCl to pH = 1, which, in turn, was extracted with ethyl acetate (100 mL x 4). The combined organic solution was dried over $Na_2SO_4$, filtered through Celite, and evaporated *in vacuo* to dryness. Recrystallization from toluene afforded 10.2 g (86%) of the desired compound as colorless crystals: mp 107-109 °C (lit. [214] mp 109-113 °C for the *S*-isomer); $^1H$ NMR (300 MHz, DMSO-$d_6$) δ 3.63 (d, *J* = 10.1 Hz, 1H, $CHH_a$), 3.52 (d, *J* = 10.1 Hz, 1H, $CHH_b$), 1.35 (s, 3H, Me); IR (KBr) 3434 (OH), 3300-2500 (COOH), 1730 (C=0), 1449, 1421, 1380, 1292, 1193, 1085 cm$^{-1}$; $[\alpha]_D^{26}$+10.5° (c = 2.6, MeOH); Anal. Calcd. for $C_4H_7BrO_3$: C 26.25, H 3.86. Found: C 26.28, H 3.75.

**Synthesis of (*R*)-3-bromo-2-hydroxy-N-(4-methoxyphenyl)-2-methylpropanamide (6b).** (*R*)-3-Bromo-2-hydroxy-2-methylpropanic acid (8.54 g, 46.7 mmol) was placed in a 250 mL three-necked round-bottomed flask fitted with a stirring bar and an addition funnel, and dissolved in 100 mL anhydrous THF at room temperature. The solution was cooled to 0 °C. Then, $SOCl_2$ (7.78 g, 65.4 mmol) was added dropwise with stirring in 3 hours. *p*-Anisidine (5.00 g, 40.6 mmol) and triethylamine (6.62 g, 65.4 mmol) were added to the mixture at 0 °C. The reaction mixture was stirred at room temperature overnight. The solvent was removed under reduced pressure to give a yellow residue which was dissolved in ethyl acetate and water. The organic layer was separated, washed with saturated $NaHCO_3$ solution and dried over anhydrous $MgSO_4$. The solvent was removed and residue was subjected to flash column chromatography (silica gel, EtOAc/hexanes = 1/1 v/v) to give a white solid product, 8.50 g, 63.2% yield.

**Synthesis of (*S*)-2-hydroxy-*N*,3-bis(4-methoxyphenyl)-2-methylpropanamide (6c).** (*R*)-3-bromo-2-hydroxy-N-(4-methoxyphenyl)-2-methylpropanamide (6b) (5.80 g, 20.13 mmol) and $K_2CO_3$ (5.56 g, 40.26 mmol) were placed in a 500 mL round-bottomed flask fitted with a stirring bar. 150 mL of acetone was added at room temperature. The reaction solution was heated to reflux for 3 hours. The solvent was removed under reduced pressure. The residue was purified by flash column chromatography (silica gel, EtOAc/hexanes = 1/1 v/v) to give a white solid product, (*S*)-*N*-(4-methoxyphenyl)-2-methyloxirane-2-carboxamide, 4.00 g, 96.0% yield. To a 500 mL single-necked round-bottomed flask fitted with a stirring bar, rubber stopper and a nitrogen inlet was added (*S*)-*N*-(4-methoxyphenyl)-2-methyloxirane-2-carboxamide (1.00 g, 4.83 mmol) and anhydrous THF (50 mL). The solution was cooled to -78 °C in dry ice-acetone bath. 4-Methoxyphenylmagnesium bromide solution (14.50 mL of 0.5 M THF solution, 7.25 mmol) was added dropwise with stirring at -78 °C. The resulted solution was stirred at -78 °C for 30 minutes and then at room temperature for 3 hours. The reaction was quenched by adding 20 mL of saturated $NH_4Cl$ solution at 0 °C. EtOAc (3x 30 mL) was added to extract the solution. The organic layers were separated, washed with brine (20 mL) and dried over anhydrous $MgSO_4$. The solvent was removed under reduced pressure and the residue was purified by flash column chromatography (silica gel, EtOAc/hexanes = 1/1 v/v) to give a white solid product, (*S*)-2-hydroxy-*N*,3-bis(4-methoxyphenyl)-2-methylpropanamide (**6c**), 0.60 g, 39.5% yield.

**Synthesis of (*S*)-2-hydroxy-3-(4-methoxyphenoxy)-*N*-(4-methoxyphenyl)-2-methylpropanamide (6d).** (*S*)-*N*-(4-Methoxyphenyl)-2-methyloxirane-2-carboxamide (0.50 g, 2.41 mmol), 4-methylphenol (0.39 g, 3.14 mmol) and $K_2CO_3$ (0.67 g, 4.82 mmol) were placed in a 250 mL round-bottomed flask fitted with a stirring bar. 100 mL of isopropanol was added at room temperature. The reaction solution was heated to reflux for 3 hours. The solvent was removed under reduced pressure. The residue was purified by flash column chromatography (silica gel, EtOAc/hexanes = 2/3 v/v) to give a white solid product, (*S*)-2-hydroxy-3-(4-methoxyphenoxy)-*N*-(4-methoxyphenyl)-2-methylpropanamide (**6d**), 0.79

g, 98.8% yield.

**Synthesis of (*R*)-3-bromo-2-hydroxy-*N*-(4-hydroxyphenyl)-2-methylpropanamide** (**6e**). (*R*)-3-bromo-2-hydroxy-*N*-(4-methoxyphenyl)-2-methylpropanamide (**6b**) (0.55 g, 1.91 mmol) was dissolved in 25 mL of anhydrous methylene chloride in a dry 250 mL round-bottomed flask fitted with a stirring bar, anitrogen inlet and rubber stopper. $BBr_3$ solution (16.0 mL of 0.5 M $CH_2Cl_2$ solution, 8.0 mmol) was added dropwise with stirring at 0 °C. The reaction solution was stirred at room temperature overnight. The reaction was quenched by adding 20 mL of water and extracted with EtOAc (3x30 mL). The EtOAc layers were separated and dried over anhydrous $MgSO_4$. The solvent was removed under reduced pressure. The residue was purified by flash column chromatography (silica gel, EtOAc/hexanes = 1/1 v/v) to give a white solid product, (R)-3-bromo-2-hydroxy-*N*-(4-hydroxyphenyl)-2-methylpropanamide (**6e**), 0.51 g, 97.9% yield.

**Synthesis of (*S*)-2-hydroxy-3-(4-hydroxyphenoxy)-*N*-(4-hydroxyphenyl)-2-methylpropanamide (6f).** (*S*)-2-hydroxy-3-(4-methoxyphenoxy)-*N*-(4-methoxyphenyl)-2-methylpropanamide (6d) (0.20 g, 0.60 mmol) was dissolved in dry $CH_2Cl_2$ (30 mL). $BBr_3$ (4 mL of 1.0 M $CH_aCl_2$ solution) was added dropwise with stirring via a syringe at room temperature. The reaction solution was allowed to stir overnight at room temperature. The mixture was cooled to 0 °C in a ice bath and hydrolyzed by adding water (25 mL). EtOAc (50 mL) was added to partition the solution. The organic layer was separated; the aqueous layer was extracted with EtOAc (2 x 10 mL). The organic layers were combined, washed with brine and dried over anhydrous $MgSO_4$. The solvent was removed under vacuum. The residue was purified by flash column chromatography using silica-gel with hexanes/EtOAc (3/7 v/v) to afford a white solid product, (*S*)-2-hydroxy-3-(4-hydroxyphenoxy)-*N*-(4-hydroxyphenyl)-2-methylpropanamid (**6f**), 0.13 g, 67.2% yield.

**Synthesis of (*S*)-2-hydroxy-*N*,3-bis(4-hydroxyphenyl)-2-methylpropanamide** (6g). (*S*)-2-hydroxy-*N*,3-bis (4-methoxyphenyl)-2-methylpropanamide (6c) (0.20 g, 0.63 mmol) was dissolved in dry $CH_2Cl_2$ (20 mL). $BBr_3$ (6 mL of 1.0 M $CH_2Cl_2$ solution) was added dropwise with stirring via a syringe at room temperature. The reaction solution was allowed to stir overnight at room temperature. The mixture was cooled to 0 °C in a ice bath and hydrolyzed by adding water (25 mL). EtOAc (50 mL) was added to partition the solution. The organic layer was separated; the aqueous layer was extracted with EtOAc (2 x 10 mL). The organic layers were combined, washed with brine and dried over anhydrous $MgSO_4$. The solvent was removed under vacuum. The residue was purified by flash column chromatography using silica-gel with hexanes/EtOAc (3/7 v/v) to afford a white solid product, (*S*)-2-hydroxy-*N*,3-bis(4-hydroxyphenyl)-2-methylpropanamide (6g), 0.12 g, 65.6% yield.

## EXAMPLE 15

### Synthesis of 11k

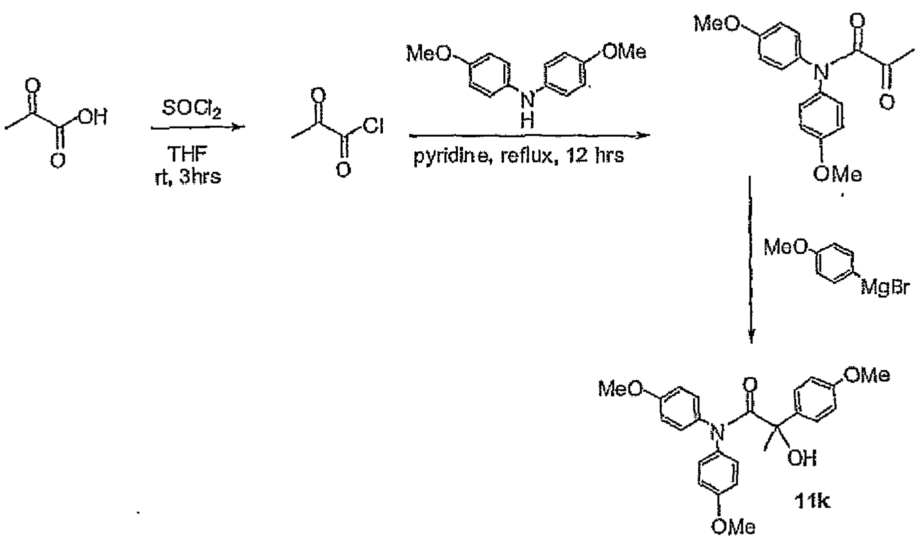


11k

**Synthesis of 2-hydroxy-*N*,*N*,2-tri(4-hydroxyphenyl)propanamide (11k).** Pyruvic acid (1.00 g, 11.34 mmol) was placed in a 250 mL three-necked round-bottomed flask fitted with a stirring bar, reflux condenser and a nitrogen inlet, and dissolved in 30 mL anhydrous THF at room temperature. Then, $SOCl_2$ (2.03 g, 17.01 mmol) was added dropwise with stirring in 3 hours at room temperature. Bis(4-methoxyphenyl)amine (2.00 g, 8.72 mmol) was added under

nitrogen protection. Pyridine (4.14 g, 52.3 mmol) were added to the mixture at 0 °C. The reaction mixture was heated to reflux for 12 hours. The reaction was quenched by adding 30 mL of 2N HCl solution. The mixture was extracted with EtOAc (3x20 mL). The organic layers were separated, washed with brine (20 mL) and dried over anhydrous $MgSO_4$. The solvent was removed under reduced pressure to give a yellow residue. The solvent was removed and residue was subjected to flash column chromatography (silica gel, EtOAc/hexanes= 3/7 v/v) to give a white solid product, *N,N*-bis(4-methoxyphonyl)-2-oxopropanamide, 2.15 g, 82.4% yield. MS: m/z 322 $[M+Na]^+$.

*N,N*-bis(4-methoxyphenyl)-2-oxopropanamide (0.53 g, 1.77 mmol) was placed in a 250 mL three-necked round-bottomed flask fitted with a stirring bar, a rubber stopper and a nitrogen inlet, and dissolved in 30 mL anhydrous THF. The solution was cooled to -78 °C in dry ice-acetone bath. 4-Methoxyphenylmagnesium bromide solution (3.89 mL of 0.5 M THF solution, 7.25 mmol) was added dropwise with stirring at -78 °C under nitrogen atmosphere. The resulted solution was stirred at -78 °C for one hour and then at room temperature for one hour. The reaction was quenched by adding 20 mL of saturated $NH_4Cl$ solution. EtOAc (3x 30 mL) was added to extract the solution. The organic layers were separated, washed with brine (20 mL) and dried over anhydrous $MgSO_4$. The solvent was removed under reduced pressure and the residue was purified by flash column chromatography (silica gel, EtOAc/hexanes= 1/1 v/v) to give a white solid product, 2-hydroxy-*N,N*,2-tris(4-methoxyphenyl)propanamide, 0.61 g, 84.7% yield.

2-hydroxy-*N,N*,2-tris(4-methoxyphenyl)propanamide (0.60 g, 1.47 mmol) was dissolved in 30 mL of anhydrous methylene chloride in a dry 250 mL round-bottomed flask fitted with a stirring bar, anitrogen inlet and rubber stopper. $BBr_3$ solution (6.00 mL of 1 M $CH_2Cl_2$ solution, 6.00 mmol) was added dropwise with stirring at 0 °C. The reaction solution was stirred at room temperature overnight. The reaction was quenched by adding 20 mL of water and extracted with EtOAc (3x30 mL). The EtOAc layers were separated and dried over anhydrous $MgSO_4$. The solvent was removed under reduced pressure. The residue was purified by flash column chromatography (silica gel, $CH_2Cl_2$/MeOH = 9/1 v/v) to give a white solid product, 2-hydroxy-NN,2-tri(4-hydroxyphenyl)propanamide (11k), 0.42 g, 77.8% yield.

**EXAMPLE 16:**

**Toremifene lowers total LDL cholesterol and triglycerides and raises HDL on prostate cancer patients on Androgen Deprivation Therapy (ADT)**

**Methods:**

1392 men 50 years old or more, with histologically documented prostate cancer and receiving ADT were randomized to toremifene (80 mg/day) or placebo treated groups in a human clinical trial. An interim analysis evaluated changes in circulating lipid levels from baseline to month 12 in the first 197 subjects to complete their first year to determine changes in total cholesterol, low density lipoprotein (LDL) cholesterol, high density lipoprotein (HDL) cholesterol, triglycerides and the ratio of total circulating cholesterol to HDL levels in the respective subjects.

**Results:**

Prostate cancer patients having undergone Androgen Deprivation Therapy (ADT) who received toremifene were compared to placebo groups. Toremifene treatment resulted in lower total circulating cholesterol (-7.1%; p=0.001), LDL (-9.0%; p=0.003), and triglyceride (-20.1%; p=0.009) levels, a reduction in the total cholesterol/HDL ratio (-11.7%; p<0.001), and higher HDL levels (+5.4%; p=0.018) (Fig 1).

Subjects concurrently administered Statins demonstrated further reduction of total cholesterol, yet the magnitude of lipid changes elicited by toremifene treatment was greater in patients who were not concomitantly taking statins. Accordingly, patients treated with toremifene had a statistically significant improvement in all serum lipid parameters measured.

**EXAMPLE 17:**

**Exemplified SERM compounds Lowering LDL Cholesterol** Levels.

**Methods:**

In addition to Toremifene, other SERM compounds may be similarly evaluated in clinical trial settings. The following compounds may be similarly administered as described in Example 1, and their effect in altering lipid profiles in subjects with prostate cancer, undergoing ADT may be similarly evaluated. Some of the compounds thus evaluated may comprise:

*Compound (1): N,N*-bis(4-hydroxyphenyl)-3,4-dimethylbenzamide;
*Compound (2): N,N-bis*(4-hydroxyphenyl)-4-propylbenzamide;
*Compound (3):* 3-fluoro-4-hydroxy-*N*-(4-hydroxyphenyl)-*N*-phenylbenzamide;
*Compound (4): N,N-bis*(4-hydroxyphenyl)-4-pentylbenzamide; and/or Ospemifene.

While certain features of the invention have been illustrated and described herein, many modifications, substitutions, changes, and equivalents will now occur to those of ordinary skill in the art. It is, therefore, to be understood that the appended claims are intended to cover all such modifications and changes as fall within the true spirit of the invention.

While certain features of the invention have been illustrated and described herein, many modifications, substitutions, changes, and equivalents will now occur to those of ordinary skill in the art. It is, therefore, to be understood that the appended claims are intended to cover all such modifications and changes as fall within the true spirit of the invention.

## Claims

1. A nuclear receptor binding agent (NRBA) compound represented by the structure of formula I:

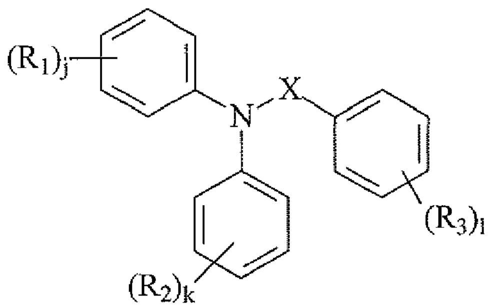

(I)

wherein
X is CO;
$R_1$, $R_2$ and $R_3$ are independently, halogen, aldehyde group, COOH, CHNOH, $CH=CHCO_2H$, hydroxyalkyl, hydroxyl, alkoxy group of 1-4 carbon atoms, cyano, nitro, $CF_3$, $NH_2$, NHR, NHCOR, $N(R)_2$, sulfonamide, $SO_2R$, alkyl, aryl, $OCH_2CH_2$-heterocycle in which the heterocycle is a 3-7 membered substituted or unsubstituted heterocyclic ring, optionally aromatic;
R is alkyl, hydrogen, haloalkyl, dihaloalkyl, trihaloalkyl, $CH_2F$, $CHF_2$, $CF_3$, $CF_2CF_3$, aryl, phenyl, halogen, alkenyl of 2-12 carbon atoms, CN, $NO_2$, or OH;
j, k, l are independently 1-5;
Alk is linear alkyl of 1-7 carbons, branched alkyl of 1-7 carbons, or cyclic alkyl of 3-8 carbons; and
if X is CO and $R_2$ is $OCH_2CH_2$-heterocycle when k is 1, then $R_1$ or $R_3$ is not hydrogen, alkyl of 1 -4 carbons, alkoxy of 1-4 carbons, halogen, nitro or amino;
if X is CO, and $R_3$ is $OCH_2CH_2$-heterocycle when 1 is one, then $R_1$ or $R_2$ is not alkyl of 1 -4 carbons, alkoxy of 1-4 carbons, halogen, nitro or amino.

2. A nuclear receptor binding agent (NRBA) compound according to claim 1, wherein the 3-7 membered substituted or unsubstituted heterocyclic ring, optionally aromatic, represented by the structure of formula B:

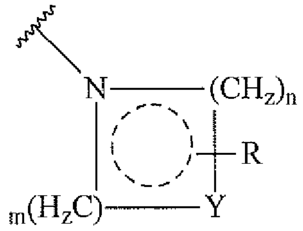

B

wherein Y is $CH_2$, CH, bond, O, S, NH, N or NR;
R is alkyl, hydrogen, haloalkyl, dihaloalkyl, trihaloalkyl, $CH_2F$, $CHF_2$, $CF_3$, $CF_2CF_3$, aryl, phenyl, halogen, alkenyl, CN, $NO_2$, or OH;
if B is aryl then z is 1; and if B is cycloalkyl z is 2;
m is 0-4;
n is 0-4;
wherein m and n cannot both be zero.

**3.** A nuclear receptor binding agent (NRBA) compound according to claim 2, wherein formula B is substituted or unsubstituted piperidine, pyrrolidine, morpholine or piperazine.

**4.** A nuclear receptor binding agent (NRBA) compound according to claim 1, wherein said compound is N-(4-hydroxyphenyl)-2,4-dimethyl-N-(4-(2-(piperidin-1-yl)ethoxy)phenyl)-benzamide.

**5.** A nuclear receptor binding agent (NRBA) compound according to any one of claims 1 to 4, wherein said compound is its isomer, pharmaceutically acceptable salt, pharmaceutical product, *N*-oxide, hydrate or any combination thereof.

**6.** A nuclear receptor binding agent (NRBA) compound according to claim 5, wherein said pharmaceutically acceptable salt is an HCL salt.

**7.** A composition comprising a nuclear receptor binding agent (NRBA) compound according to any preceding claim and a suitable carrier or diluent.

**8.** A compound according to any one of claims 1-6, wherein said compound is a selective estrogen receptor modulator (SERM).

**9.** A compound according to claims 1-6 and 8, for use in: treating, preventing, inhibiting or reducing the severity of osteoporosis in a subject; reducing the incidence of, inhibiting, suppressing, or treating androgen-deprivation induced osteoporosis, bone fractures and/or loss of bone mineral density (BMD) in men having prostate cancer; ameliorating symptoms and/or clinical complications associated with menopause in a female subject; treating, preventing, inhibiting or reducing the risk of breast cancer in a subject; or treating, preventing, inhibiting or reducing the risk of endometrial cancer in a subject.

**10.** A compound for use according to claim 9, wherein said nuclear receptor binding agent (NRBA) compound is an ER-$\alpha$ ligand.

**11.** A compound for use according to any of claims 1-6 and 8, for treating, preventing, inhibiting or reducing the risk of mortality from cardiovascular disease in a subject; for treating, preventing, inhibiting or reducing the incidence of inflammation in a subject; for treating, preventing, inhibiting or reducing the risk of colon cancer in a subject; or for treating, preventing, inhibiting or reducing the risk of leukemia in a subject.

**12.** A compound for use according to claim 11, wherein said nuclear receptor binding agent (NRBA) compound is an ER-$\beta$ ligand.

**13.** A compound for use according to any of claims 1-6 and 8 , for improving a lipid profile in a subject, or for treating, preventing, inhibiting or reducing the risk of bladder cancer in a subject.

**14.** A compound for use according to any of claims 1-6 and 8, for treating, preventing, suppressing, inhibiting or reducing the severity of Alzheimer's disease in a subject; for treating, preventing, suppressing, inhibiting, or reducing the incidence of hot flashes, gynecomastia, and/or hair loss in a male subject having prostate cancer; for treating, suppressing, inhibiting or reducing the risk of developing prostate cancer in a subject; or for treating, suppressing, inhibiting or reducing the amount of precancerous precursors of prostate adenocarcinoma lesions in a subject.

**15.** A compound for use according to claim 14, wherein said nuclear receptor binding agent (NRBA) compound is an ER-$\alpha$ or ER-$\beta$ ligand.

**16.** A compound for use according to claim 14, wherein said precancerous precursors of prostate adenocarcinoma is prostate intraepithelial neoplasia (PIN).

**17.** A compound for use according to any of claims 1-6 and 8, for reducing circulating lipid levels in a male subject with prostate cancer having undergone Androgen Deprivation Therapy (ADT).

**18.** A compound for use according to claim 17, wherein said lipid levels, which are reduced comprise a triglyceride, low density lipoprotein (LDL) cholesterol, or a combination thereof.

**19.** A compound for use according to claim 17, wherein said use comprises increasing circulating levels of high density lipoprotein (HDL) cholesterol in said subject.

**20.** A compound for use according to claim 17, wherein said use further comprises reducing the ratio of total circulating cholesterol levels to high density lipoprotein (HDL) levels in a subject.

**21.** A compound for use according to claim 17, wherein said subject further suffers from atherosclerosis and its associated diseases, premature aging, Alzheimer's disease, stroke, toxic hepatitis, viral hepatitis, peripheral vascular insufficiency, renal disease, hyperglycemia, or any combination thereof.

**22.** A compound for use according to any of claims 1-6 and 8, for treating atherosclerosis and its associated diseases including cardiovascular disorders, cerebrovascular disorders, peripheral vascular disorders, and intestinal vascular disorders in a subject with prostate cancer having undergone Androgen Deprivation Therapy (ADT).

**23.** A compound for use according to any of claims 1-6 and 8, for treating ischemia in a tissue of a subject with prostate cancer having undergone Androgen Deprivation Therapy (ADT).

Europäisches Patentamt
European Patent Office
Office européen des brevets

# EUROPEAN SEARCH REPORT

Application Number
EP 12 15 0818

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HEINE H.W. ET AL: "The Reactions of an o-Quinone Monoimide with Some Phenols", JOURNAL OF ORGANIC CHEMISTRY, vol. 55, no. 13, 1990, pages 4039-4043, XP002627484, * page 4040, Scheme I, compound 3a-c; compound 7; page 4040, Scheme II, compound 9 * | 1-23 | INV. C07C233/65 A61K31/165 C07D221/12 A61K31/55 A61K31/445 A61K31/40 A61K31/138 |
| X | EP 1 593 665 A1 (IDEMITSU KOSAN CO [JP]) 9 November 2005 (2005-11-09) * [0009, compound of formula (1) in combination with the definition for Ar, Ar1 and Ar2) * | 1-23 | |
| Y | US 3 960 886 A (SCHULENBERG JOHN W) 1 June 1976 (1976-06-01) * examples, e.g. Example 51a, 51b, 52b, 59b, 60, 62, 64, 66c; claims * | 1-23 | |
| Y | US 2002/156301 A1 (KANEKO HIDEO [JP] ET AL) 24 October 2002 (2002-10-24) * [0002-0003]; claims * | 1-23 | TECHNICAL FIELDS SEARCHED (IPC): C07C A61K |

The present search report has been drawn up for all claims

1

| Place of search | Date of completion of the search | Examiner |
|---|---|---|
| Munich | 5 April 2012 | Sen, Alina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 12 15 0818

This annex lists the patent family members relating to the patent documents cited in the above-mentioned European search report.
The members are as contained in the European Patent Office EDP file on
The European Patent Office is in no way liable for these particulars which are merely given for the purpose of information.

05-04-2012

| Patent document cited in search report | Publication date | Patent family member(s) | Publication date |
|---|---|---|---|
| EP 1593665 A1 | 09-11-2005 | CN 1753860 A | 29-03-2006 |
| | | EP 1593665 A1 | 09-11-2005 |
| | | JP 3768196 B2 | 19-04-2006 |
| | | JP 2004262768 A | 24-09-2004 |
| | | KR 20050099995 A | 17-10-2005 |
| | | US 2006063952 A1 | 23-03-2006 |
| | | WO 2004069785 A1 | 19-08-2004 |
| US 3960886 A | 01-06-1976 | NONE | |
| US 2002156301 A1 | 24-10-2002 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5543158 A
- US 5641515 A
- US 5399363 A


### Non-patent literature cited in the description


- **LANGER.** *Science,* 1990, vol. 249, 1527-1533
- **TREAT et al.** Liposomes in the Therapy of Infectious Disease and Cancer. Liss, 1989, 353-365
- **LOPEZ-BERESTEIN.** LIPOSOMES IN THE THERAPY OF INFECTIOUS DISEASE AND CANCER. 317-327
- *Invest Ophthal Vis Sci,* 1997, vol. 38, 1193-1202
- *Invest Ophthal Vis Sci,* 2003, vol. 44 (7), 3155-3162
- *Br J Ophthalmol,* 2001, vol. 85, 877-882
- **WAHNER H W ; DUNN W L ; THORSEN H C et al.** Nuclear Medicine: ''Quantitative Procedures. Toronto Little, Brown & Co, 1983, 107-132
- Assessment of Bone Mineral Part 1. *Journal of Nuclear Medicine,* 1984, 1134-1141
- Bone Mineral Density of The Radius. *Nov. Journal of Nuclear Medicine,* 1985, vol. 26 (11), 13-39
- **S. HOORY et al.** *Radiology,* 1985, vol. 157 (P), 87
- **C. R. WILSON et al.** *Radiology,* 1985, vol. 157 (P), 88
- **D. MA ; Q. CAI ; H. ZHANG.** *Org. Lett.,* 2003, vol. 5, 2453